# EUROPEAN PATENT SPECIFICATION

(11) **EP 1 117 435 B1**
(45) Date of publication and mention of the grant of the patent: **14.11.2007**
(21) Application number: 99951639.6
(22) Date of filing: 30.09.1999
(51) Int. Cl.: A61K 39/39, A61K 39/095, A61K 39/102, A61K 39/106, A61K 39/245, A61K 39/155, A61K 39/15, C12N 15/63, C12N 5/10

(54) **MUTANT CHOLERA HOLOTOXIN AS AN ADJUVANT**
MUTIERTES CHOLERA HOLOTOXIN ALS HILFSMITTEL
HOLOTOXINE MUTANTE DU CHOLERA UTILISEE COMME ADJUVANT

(30) Priority: 30.09.1998 US 102430 P
(43) Date of publication of application: 25.07.2001
(73) Proprietor: Wyeth Holdings Corporation, Madison, NJ 07940-0874 (US); The Government of the United States of America as represented by the Uniformed Services University of the Health Sciences, Bethesda, MD 20814-4799 (US)
(72) Inventor: HOLMES, Randall, K., Golden, CO 80401 (US); JOBLING, Michael, G., Denver, CO 80222 (US); ELDRIDGE, John, H., Fairport, NY 14450 (US); GREEN, Bruce, A., Pittsford, NY 14534 (US); HANCOCK, Gerald, E., Honeoye Falls, NY 14472 (US); PEEK, Joel, A., Pittsford, NY 14534 (US)
(74) Representative: Silveston, Judith
(86) International application number: PCT/US1999/022520
(87) International publication number: WO 2000/018434

(56) References cited:
- WO-A-97/02348
- WO-A-97/05267
- WO-A-97/29771
- WO-A-98/45324
- GLINEUR C ET AL: "Importance of ADP-ribosylation in the morphological changes of PC12 cells induced by cholera toxin." INFECTION AND IMMUNITY, (1994 OCT) 62 (10) 4176-85. , XP002129167
- YAMAMOTO S ET AL: "Mutants in the ADP-ribosyltransferase cleft of cholera toxin lack diarrheagenicity but retain adjuvanticity." JOURNAL OF EXPERIMENTAL MEDICINE, (1997 APR 7) 185 (7) 1203-10. , XP002129168
- RICHARDS C M ET AL: "Enhancement of the immune response to non-replicating herpes simplex virus type-1 preparations by mucosal administration in the presence of cholera toxin" VACCINE,GB,BUTTERWORTH SCIENTIFIC. GUILDFORD, vol. 15, no. 10, 1 July 1997 (1997-07-01), pages 1065-1069, XP004085964 ISSN: 0264-410X
- NEDRUD J. ET AL: "Oral immunization against respiratory viruses in mice." ADVANCES IN EXPERIMENTAL MEDICINE AND BIOLOGY, (1995) 371/B (1595-1598). , XP000867733
- ZHU, D. ET AL: "Intragastric immunization with recombinant H. pylori urease formulated with attenuated cholera toxin elicits systemic, mucosal and protective immune responses in C57BL/6 mice." FASEB JOURNAL, (MARCH 12, 1999) VOL. 13, NO. 4 PART 1, PP. A291. MEETING INFO.: ANNUAL MEETING OF THE PROFESSIONAL RESEARCH SCIENTISTS FOR EXPERIMENTAL BIOLOGY 99 WASHINGTON, D.C., USA APRIL 17-21, 1999 , XP000867558

### SEQUENCE LISTING

<110> American Cyanamid Company
<120> Mutant Cholera Holotoxin As An Adjuvant
<130> 33383-00 PCT
<140> 60/102,430
   <141> 1998-09-30
<160> 7
<170> PatentIn Ver. 2.0
<210> 1
   <211> 20
   <212> DNA
   <213> Vibrio cholerae
<400> 1
   aagttatata aggcagattc 20
<210> 2
   <211> 18
   <212> DNA
   <213> Vibrio cholerae
<400> 2
   cagattctaa acctcctg 18
<210> 3
   <211> 22
   <212> DNA
   <213> Vibrio cholerae
<400> 3
   gacagagtna gtactttgac cg 22
<210> 4
   <211> 22
   <212> DNA
   <213> Vibrio cholerae
<400> 4
   cagatgakca agakgtttct gc 22
<210> 5
   <211> 22
   <212> DNA
   <213> Vibrio cholerae
<400> 5
   cagatgakca agakgtttct gc 22
<210> 6
   <211> 32
   <212> DNA
   <213> Vibrio cholerae
<400> 6
   ttttttgggc tagcatggag gaaaagatga gc 32
<210> 7
   <211> 26
   <212> DNA
   <213> Vibrio cholerae
<400> 7
   gcaggtcgaa gcttgcatgt ttgggc 26

## Description

### Field of the Invention

This invention relates to the use of an immunogenic mutant cholera holotoxin having reduced toxicity compared to a wild-type cholera toxin and a substitution other than aspartic acid for the glutamic acid at position 29 of the A subunit of the cholera holotoxin as an adjuvant to enhance the immune response in a vertebrate host to a selected antigen.

### Background of the Invention

The immune system uses a variety of mechanisms for attacking pathogens. However, not all of these mechanisms are necessarily activated after immunization. Protective immunity induced by immunization is dependent on the capacity of the vaccine to elicit the appropriate immune response to resist or eliminate the pathogen. Depending on the pathogen, this may require a cell-mediated and/or humoral immune response.

A substance that enhances the immune response when administered together with an immunogen or antigen is known as an adjuvant.

The Gram-negative bacterium *Vibrio cholerae* (*V. cholerae*) is the causative agent of the gastrointestinal disease cholera. The diarrhea caused by *V. cholerae* is due to the secretion of cholera toxin (CT).

CT comprises a single A subunit (CT-A), which is responsible for the enzymatic activity of the toxin, and five identical B subunits (CT-B), which are involved in the binding of the toxin to intestinal epithelial cells, as well as other cells which contain ganglioside GM₁ on their surface. Together, the CT-A and CT-B subunits comprise a holotoxin. The sequence of CT has been described (Bibliography entry 1).

CT a is hexaheteromeric complex consisting of one A polypeptide and five identical B polypeptides (2). The B pentamer is required for binding to the cell surface receptor ganglioside GM₁ (3). The A subunit can be proteolytically cleaved within the single disulfide-linked loop between C187 and C199 to produce the enzymatically active A1 polypeptide (4) and the smaller polypeptide A2, which links fragment A1 to the B pentamer (5). Upon entry into enterocytes, CT-A1 ADP-ribosylates a regulatory G-protein (Gsα), which leads to constitutive activation of adenylate cyclase, increased intracellular concentration of cAMP, and secretion of fluid and electrolytes into the lumen of the small intestine (6). *In vitro*, ADP-ribosyl transferase activity of CT is stimulated by the presence of accessory proteins called ARFs (7), small GTP-binding proteins known to be involved in vesicle trafficking within the eukaryotic cell.

The need for effective immunization procedures is particularly acute with respect to infectious organisms which cause acute infections at, or gain entrance to the body through, the gastrointestinal, pulmonary, nasopharyngeal or genitourinary surfaces. These areas are bathed in mucus, which contains immunoglobulins consisting largely of secretory IgA (8,9,10). This antibody is derived from large numbers of IgA-producing plasma cells which infiltrate the lamina propria regions underlying these mucosal membranes (11,12). IgA is specifically transported to the lumenal surface through the action of the secretory component (13).

However, parenteral immunization regimes are usually ineffective in inducing secretory IgA responses. Secretory immunity is most often achieved through the direct immunization of mucosally-associated lymphoid tissues. Following their induction at one mucosal site, the precursors of IgA-producing plasma cells extravasate and disseminate to diverse mucosal tissues where final differentiation to high-rate IgA synthesis occurs (14,15,16). Extensive studies have demonstrated the feasibility of mucosal immunization to induce this common mucosal immune system (17), but with rare exceptions the large doses of antigen required to achieve effective immunization have made this approach impractical for purified vaccine antigens. Among the strategies investigated to overcome this problem is the use of mucosal adjuvants. It is known that CT is one of the most potent adjuvants, and that the coadministration of CT with an unrelated antigen results in the induction of concurrent circulating and mucosal antibody responses to that antigen (18). Thus, CT can act as an adjuvant.

International Patent Publication No. WO 97/29771 refers generally to an immunogenic detoxified protein comprising the amino acid sequence of subunit A of cholera toxin wherein at least one amino acid is substituted with another amino acid, and summarizes prior publications which disclose cholera toxin mutants at positions 7, 9, 11, 44, 53, 54, 61, 63, 70, 97, 104, 106, 107, 110, 112, 114, 127, 146 or 192, a double mutant at positions 63 and 192 and truncation at 180. The mutants are characterized in that in purified form, the immunogenic detoxified protein exhibits a residual toxicity greater than 10,000 fold lower than its naturally occurring counterpart. This publication exemplifies a mutant at position 106 of CT-A, preferably a proline to serine (P to S) mutation.

International Patent Publication No. WO 97/02348 refers to detoxified mutants of cholera toxin which demonstrate double amino acid replacements at serine at position 63 (S63) and arginine at position 192 (R192), preferably lysine at position 63 (K63) with either asparagine at position 192 (N192) or glycine at position 192 (G192). This publication also summarizes the prior art references as disclosing other cholera toxin mutants having amino acid replacements at positions 7, 9, 11, 44, 53, 54, 61, 63, 70, 97, 104, 106, 107, 110, 112, 114, 122, 127, 146 or 192 and a truncation at position 180.

Yamamoto S. et al, 1997 J. Exp. Med, 185(7):1203-1209 refers to two cholera toxin subunit A mutants: one having an amino acid replacement at position 61 and the other at position 112.

Glineur et al., Infection and Immunity, 1994 62(10):4176-85 refers to two cholera toxin subunit A mutants: one having an amino acid deletion at position 29 (the naturally-occurring glutamic acid is deleted) and the other mutant replacing the naturally-occurring glutamic acid at position 29 with aspartic acid (E29D). Glineur demonstrates that the deletion mutant showed strongly reduced ADP-ribosyltransferase activity and no morphological changes of PC12 cells, while the E29D mutant was as active as wild-type CT-A.

It would be preferable to use as an adjuvant a form of the CT holotoxin that has reduced toxicity so as to reduce the undesirable symptoms of diarrhea caused by wild-type CT. Thus, there is a need to identify a mutant CT holotoxin which is able to enhance the immune response while reducing the toxicity of the CT holotoxin.

### Summary of the Invention

Accordingly, it is an object of this invention to utilize a mutant form of the CT holotoxin that has reduced toxicity compared to a wild-type CT as an adjuvant in an antigenic composition to enhance the immune response in a vertebrate host to a selected antigen from a pathogenic bacterium, virus, fungus or parasite.

These objects of the invention are achieved with a mutant cholera holotoxin featuring a point mutation at amino acid 29 of the A subunit, wherein the glutamic acid residue is replaced by histidine. The mutated CT (also referred to as CT-CRM) is useful as an adjuvant in an antigenic composition to enhance the immune response in a vertebrate host to a selected antigen from a pathogenic bacterium, virus, fungus or parasite. The mutant CT is produced by site-directed mutagenesis of the DNA encoding the wild-type CT using conventional techniques. The antigenic composition may further comprise a diluent or carrier.

The invention is also directed to methods for increasing the ability of an antigenic composition containing a selected antigen from a pathogenic bacterium, virus, fungus or parasite to elicit the immune response of a vertebrate host by including an effective adjuvanting amount of a mutant cholera holotoxin, wherein the holotoxin has reduced toxicity compared to a wild-type CT and the glutamic acid at amino acid position 29 of the A subunit of the cholera holotoxin is replaced by a histidine.

The invention further relates to plasmids containing isolated and purified DNA sequences comprising DNA sequences which encode an immunogenic mutant cholera holotoxin having a histadine substitution at position 29 of the A subunit of the cholera holotoxin, and wherein such a DNA sequence is operatively linked to an arabinose inducible promoter, as well as to suitable host cells transformed, transduced or transfected with such plasmids. The immunogenic mutant cholera holotoxin is produced by transforming, transducing or transfecting a host cell with a plasmid described above and culturing the host cell under conditions which permit the expression of said recombinant immunogenic detoxified protein by the host cell.

### Brief Description of the Figures

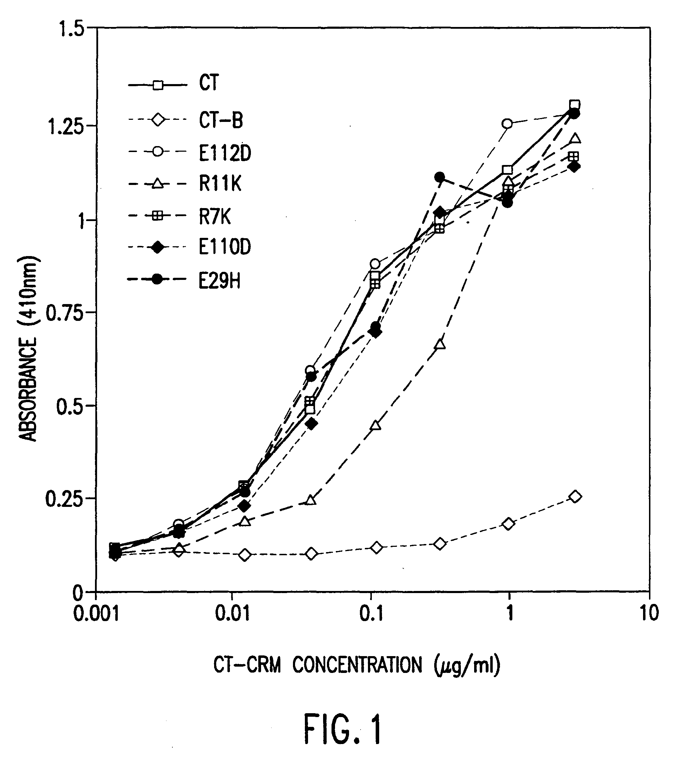

Figure 1 depicts the capacity of CT-CRMs to bind ganglioside GM₁. Each CT-CRM was diluted three-fold at an initial concentration of 3 µg/ml and tested in duplicate. The binding capacity was expressed as the mean absorbance at 410 nm for each dilution.

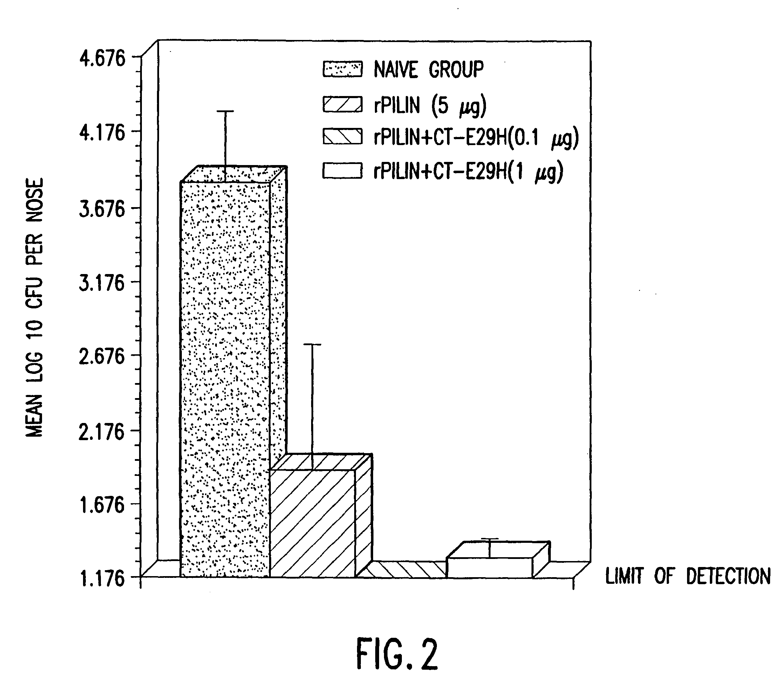

Figure 2 depicts the reduction in nasal colonization in mean Log₁₀ cfu per nose in mice immunized intranasally (n = 10 per group) with meningococcal recombinant pilin (rpilin) with or without CT-CRM_{E29H} adjuvant, or non-immunized mice, where each group was then challenged with the homologous meningococcal bacterial strain.

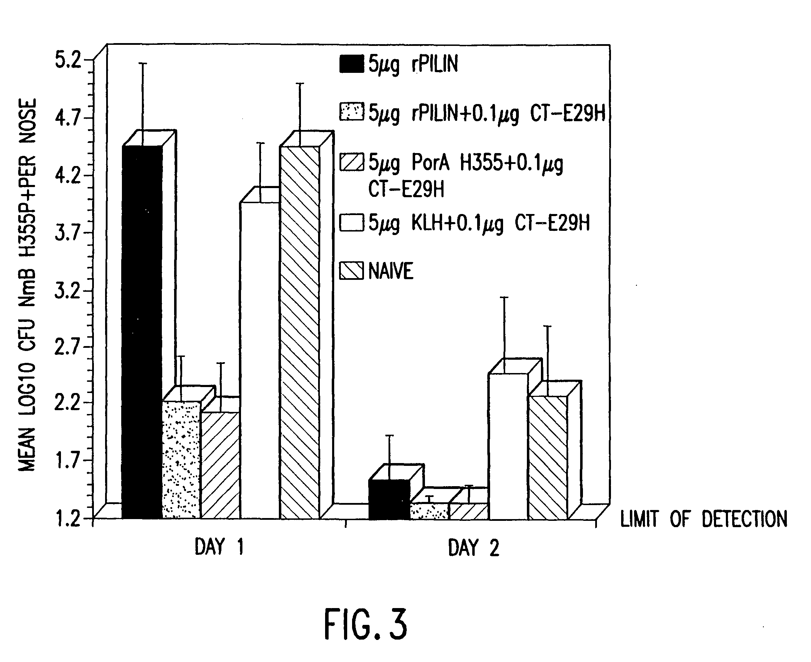

Figure 3 depicts the reduction in nasal colonization in mean Log₁₀ cfu per nose in mice immunized intranasally (n = 5 per group) with meningococcal rpilin with or without CT-CRM_{E29H} adjuvant, meningococcal class 1 outer membrane protein (Por A) with CT-CRM_{E29H} adjuvant, KLH with CT-CRM_{E29H} adjuvant, or non-immunized mice, where each group was then challenged with the homologous meningococcal bacterial strain.

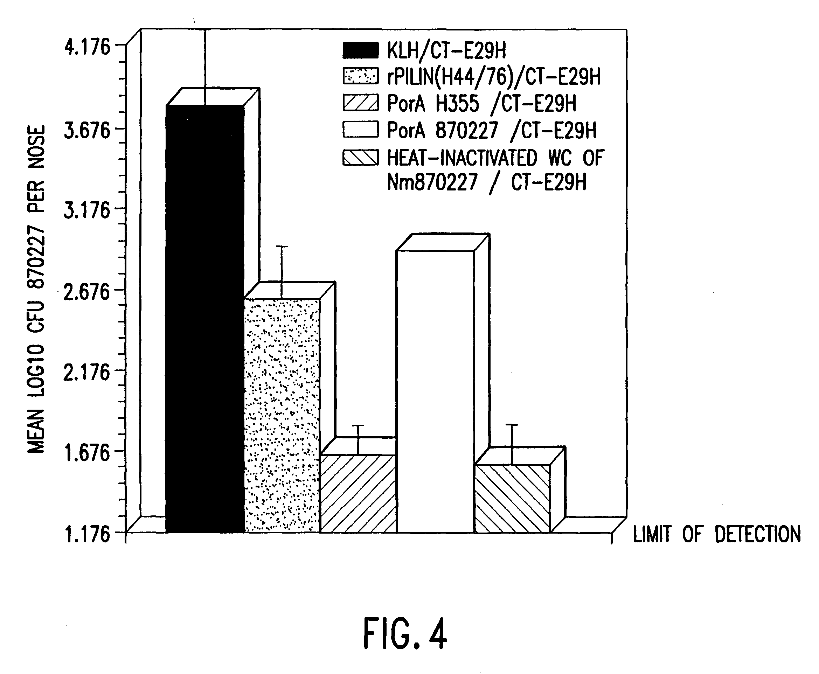

Figure 4 depicts the reduction in nasal colonization in mean Log₁₀ cfu of meningococcal strain 870227 per nose in mice immunized intranasally (n = 10 per group) with meningococcal rpilin, PorA from meningococcal strain H355, PorA from meningococcal strain 870227, heat-inactivated meningococcal strain 870227 whole cells or KLH, each adjuvanted with CT-CRM_{E29H}, where each group was then challenged with a heterologous meningococcal bacterial strain (870227).

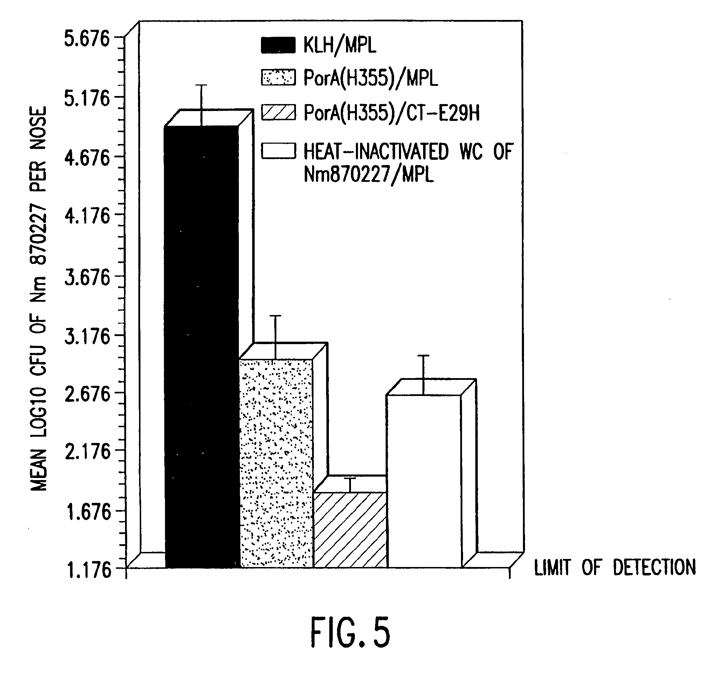

Figure 5 depicts the reduction in nasal colonization in mean Log₁₀ cfu of meningococcal strain 870227 per nose in mice immunized subcutaneously (n = 10 per group) with PorA from meningococcal strain H355 with CT-CRM_{E29H} or MPL^{™} adjuvant, KLH with MPL^{™} adjuvant, or heat-inactivated meningococcal strain. 870227 whole cells with MPL^{™} adjuvant, where each group was then challenged with a heterologous meningococcal bacterial strain (870227).

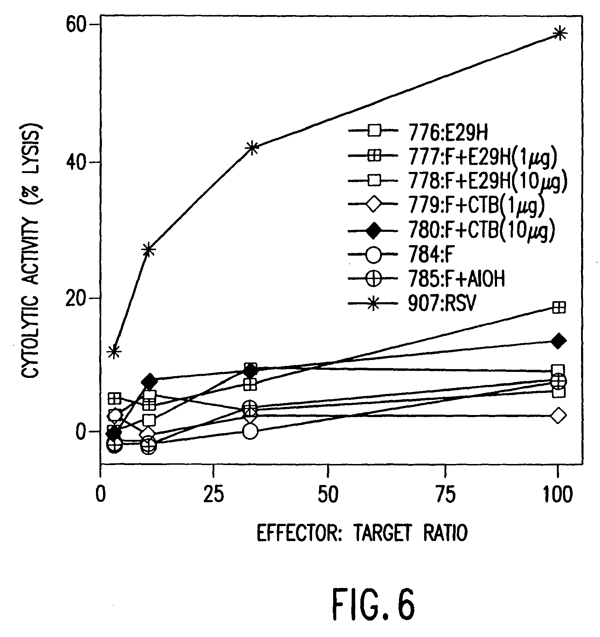

Figure 6 depicts a first assay of the antigen-dependent cytolytic activity to respiratory syncytial virus (RSV)-infected target cells as the percentage of cell lysis versus effector:target ratio.

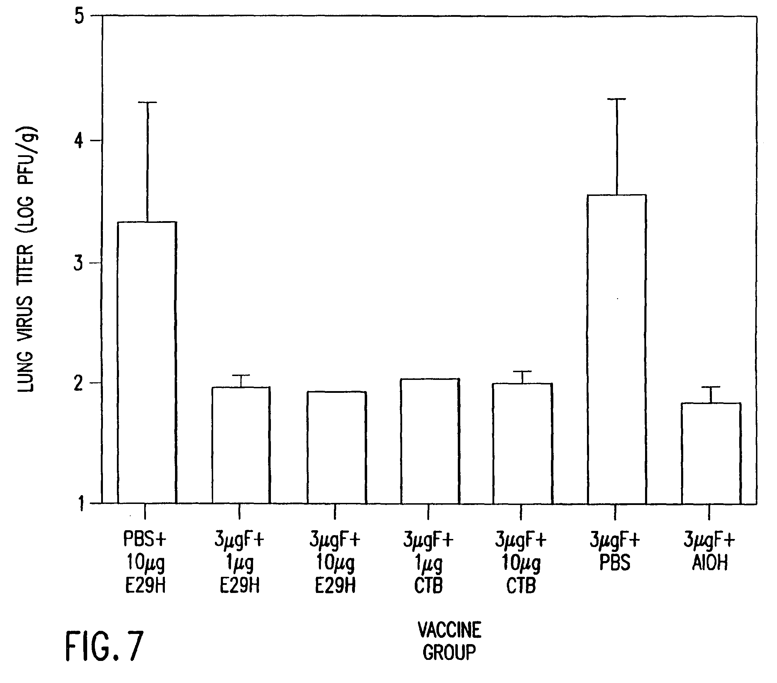

Figure 7 depicts a first assay of the protection of mouse lung to RSV challenge by immunization with F protein plus adjuvant, where the lung virus titer is measured as Log₁₀ PFU per gram.

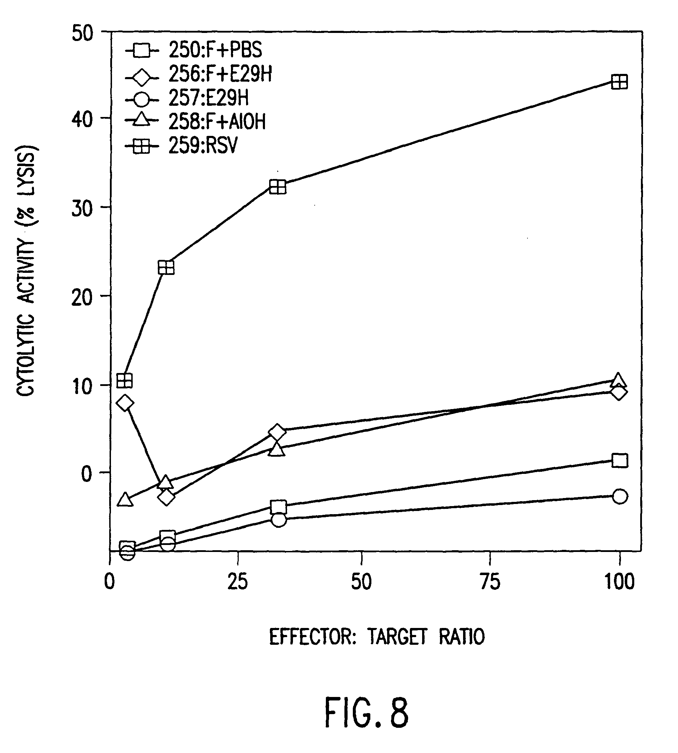

Figure 8 depicts a second assay of the antigen-dependent cytolytic activity to RSV-infected target cells as the percentage of cell lysis versus effector:target ratio.

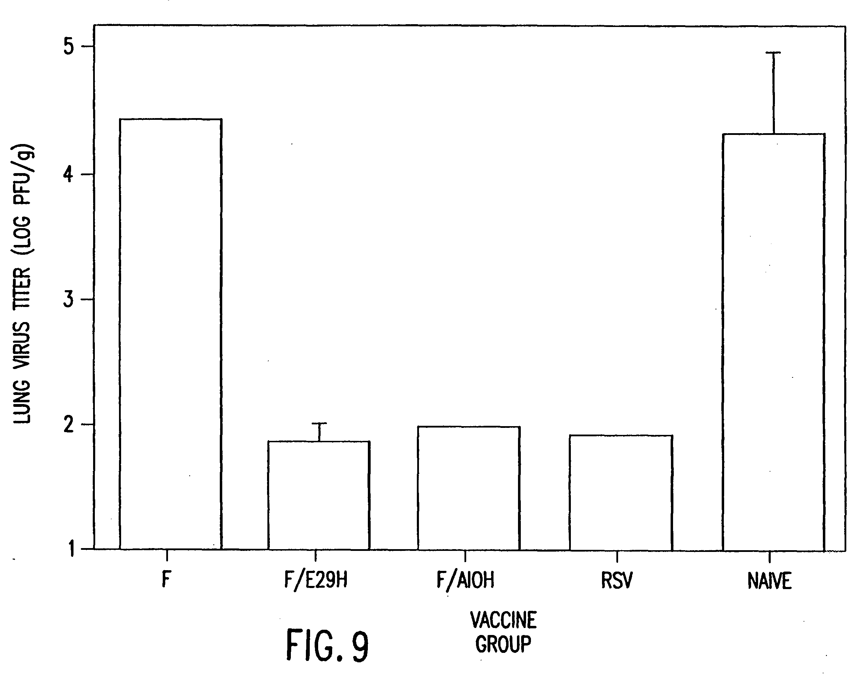

Figure 9 depicts a second assay of the protection of mouse lung to RSV challenge by immunization with F protein plus adjuvant, where the lung virus titer is measured as Log₁₀ PFU per gram.

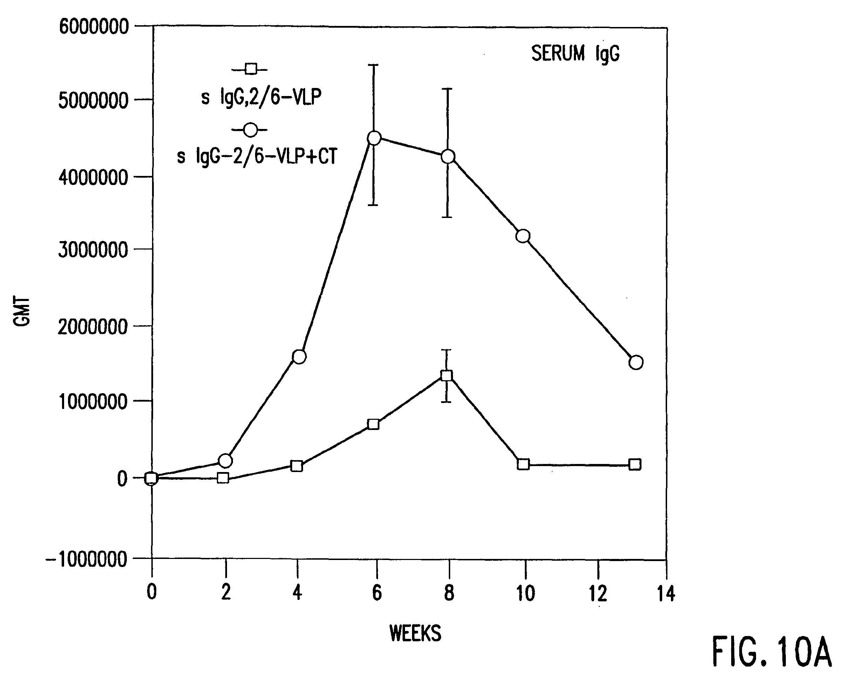

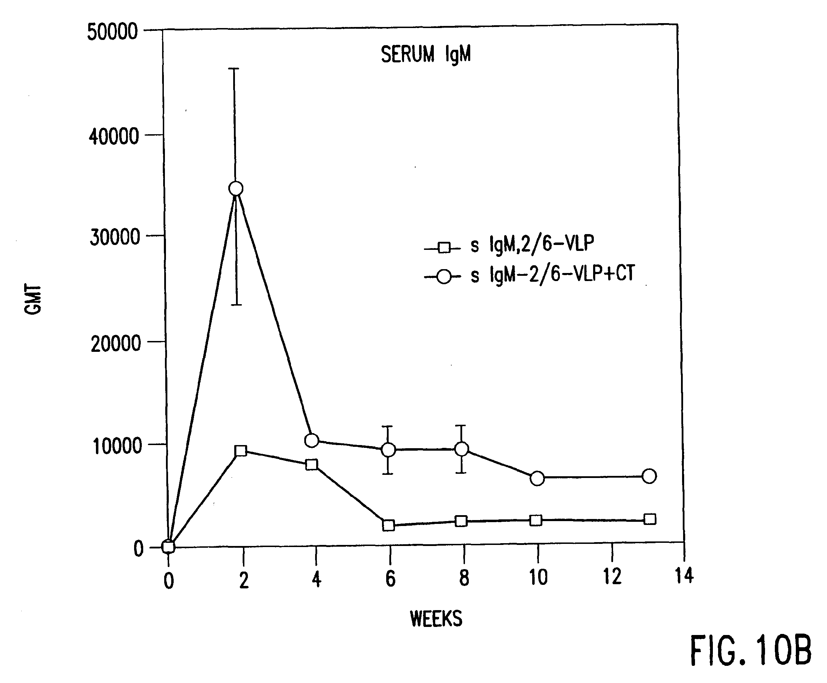

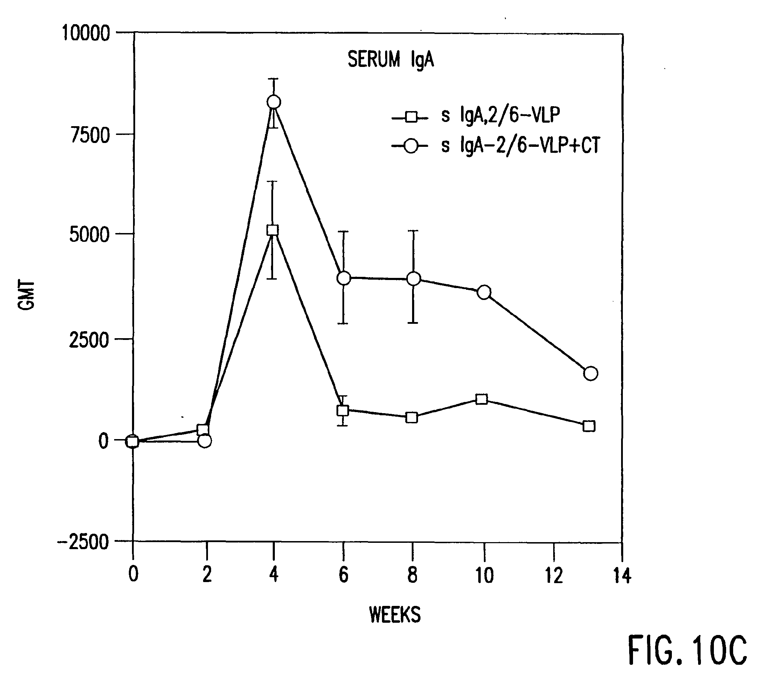

Figure 10 depicts rotavirus-specific serum antibody responses in BALB/c mice immunized intranasally with 2/6-VLPs with or without CT-CRM_{E29H} adjuvant. BALB/c mice were immunized intranasally with 2/6-VLPs with (n=4) or without (n=5) CT-CRM_{E29H} and levels of rotavirus-specific IgG (Figure 10A), IgM (Figure 10B) and IgA (Figure 10C) were measured. Standard deviations are shown.

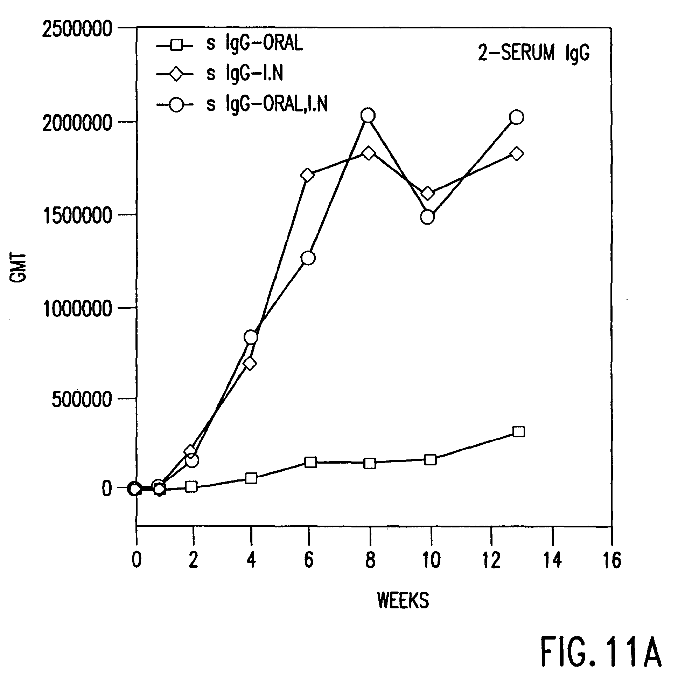

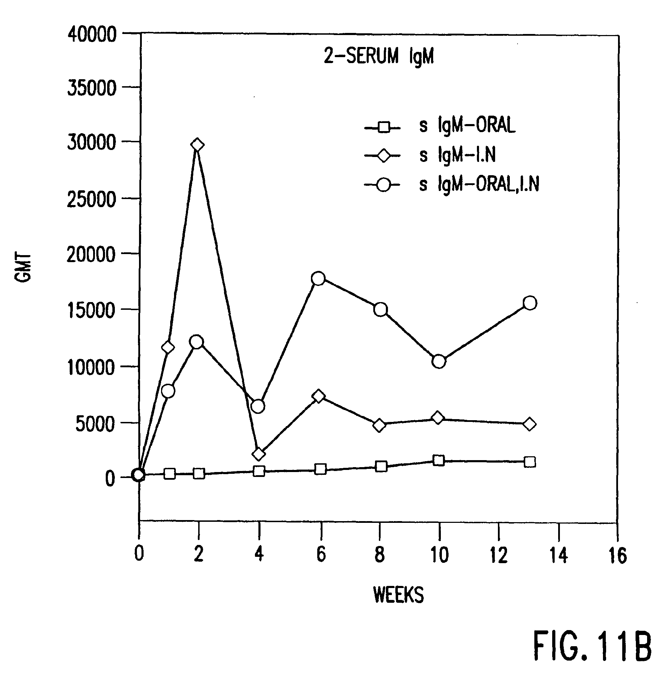

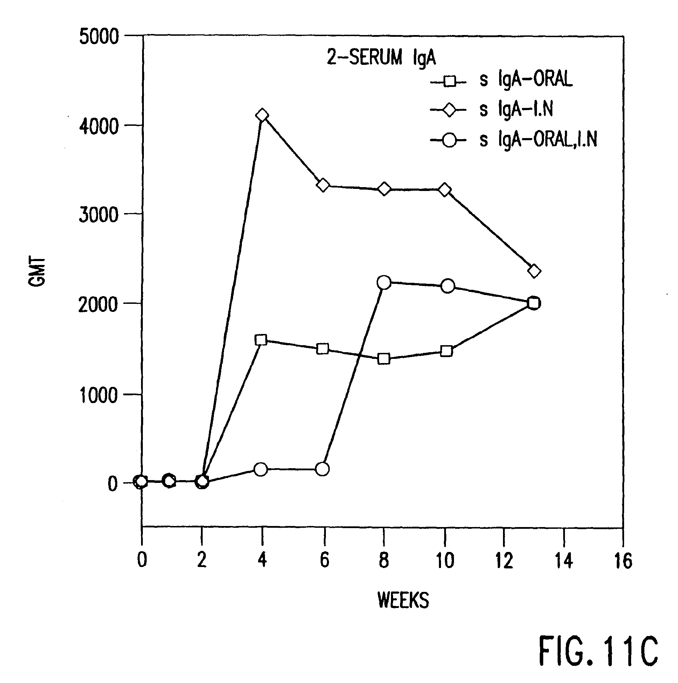

Figure 11 depicts rotavirus-specific serum antibody responses in BALB/c inbred mice immunized with 2/6-VLPs. Groups of BALB/c mice were immunized, orally (square, n=4), intranasally (diamond, n=5) or in combination (intranasal plus oral, circle, n=4), with 2/6-VLPs plus CT-CRM_{E29H} on week 0 and 2. Serum samples were collected from individual mice in each group on weeks shown, and levels of serum IgG (Figure 11A), IgM (Figure 11B) and IgA (Figure 11C) were determined for each mouse by ELISA. Geometric mean titers (GMT) were calculated for each group and plotted against weeks post-immunization.

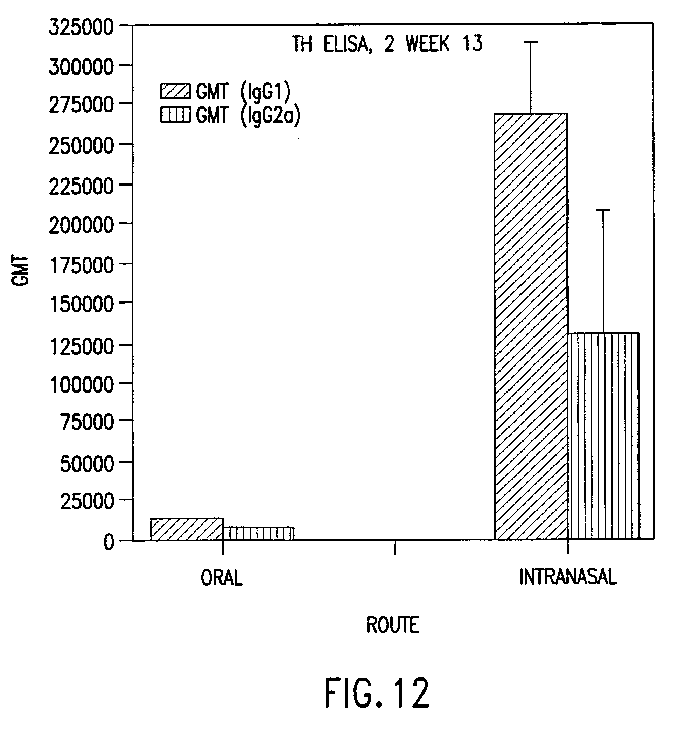

Figure 12 depicts IgG1 and IgG2a antibody subclasses in BALB/c mice. Pre-challenge sera of BALB/c mice immunized orally or IN, with rotavirus 2/6-virus-like particles (VLPs) plus CT-CRM_{E29H}, were used to determine IgG subclasses. Standard deviations are shown.

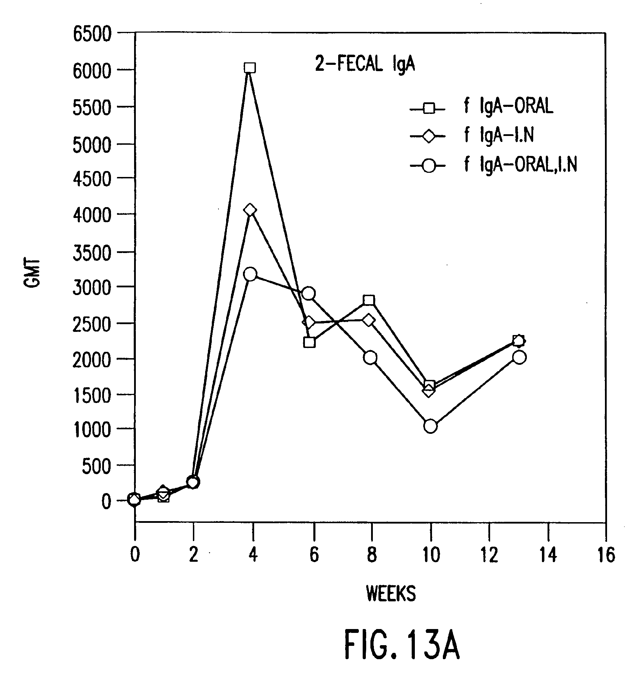

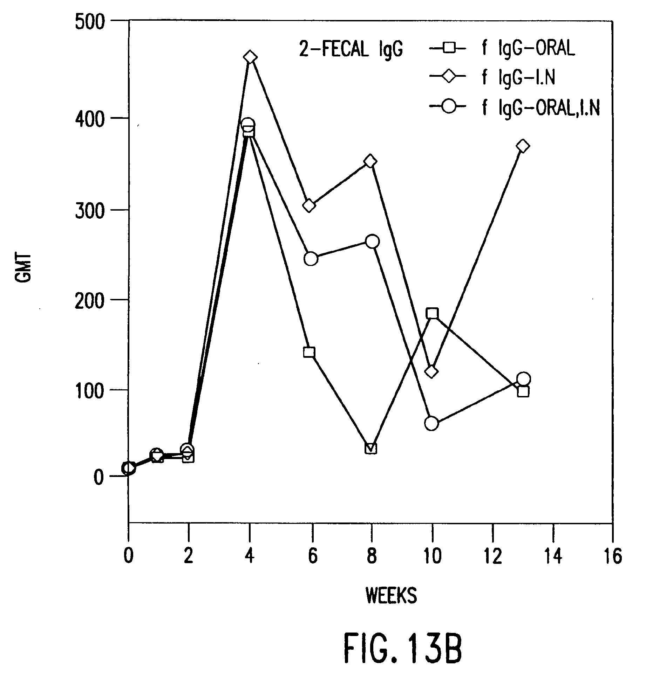

Figure 13 depicts rotavirus-specific intestinal antibody responses in inbred BALB/c mice immunized with 2/6-VLPs. Groups of BALB/c mice were immunized with 2/6-VLPs plus CT-CRM_{E29H} as described for Figure 11, and levels of rotavirus-specific intestinal IgA (Figure 13A) and IgG (Figure 13B) were measured. No rotavirus-specific intestinal IgM was detected in any mice.

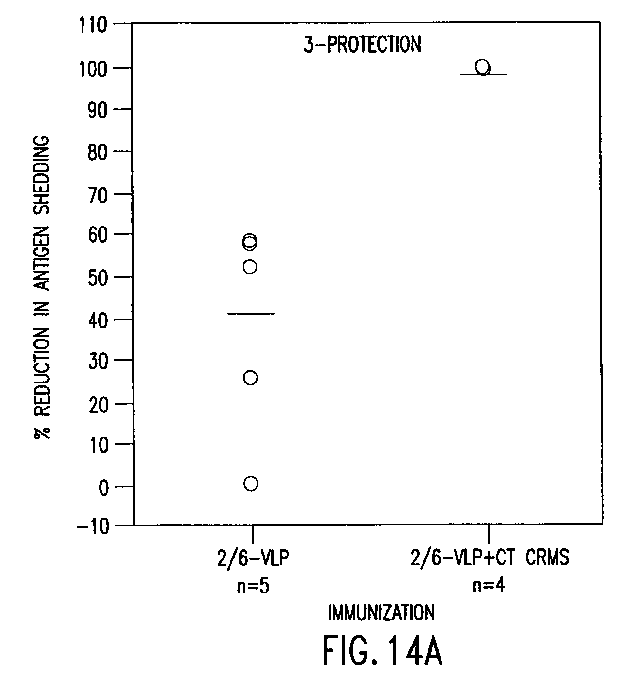

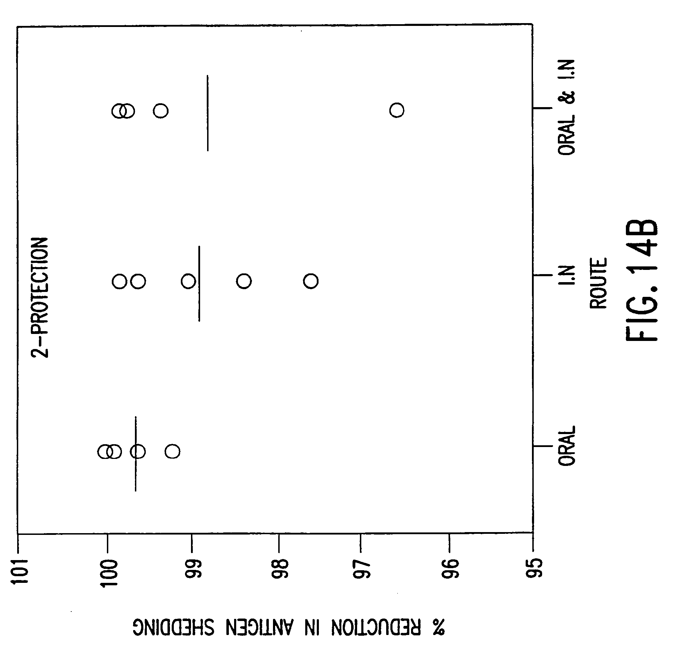

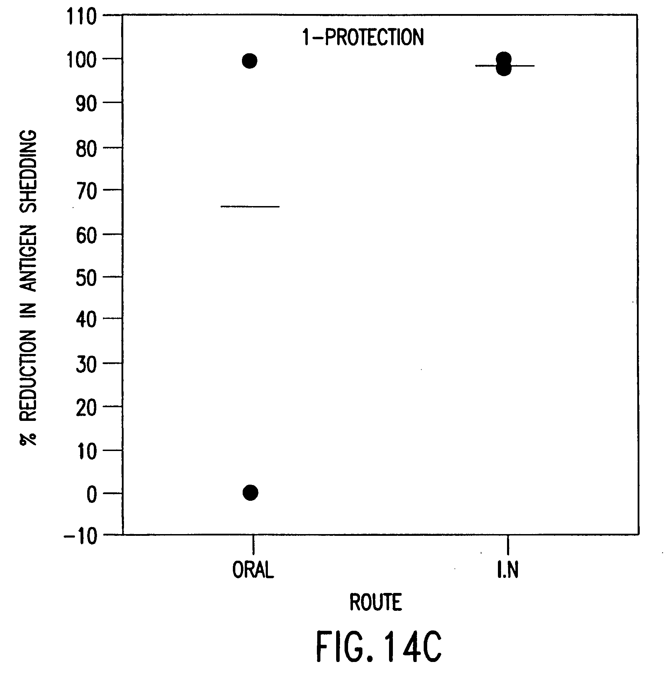

Figure 14 depicts protection of 2/6-VLP immunized BALB/c and CD-1 mice following challenge with murine rotavirus. Figure 14A depicts a comparison of percent reduction in antigen shedding (PRAS) between mice immunized with 2/6-VLPs with (n=5) or without (n=4) CT-CRM_{E29H}. PRAS for each mouse (•) and the mean of each group (-) were calculated. Figure 14B depicts groups of BALB/c mice immunized with 2/6-VLP plus CT-CRM_{E29H} by different routes as shown, and protection levels were determined as described above. Figure 14C depicts groups of outbred CD-1 mice immunized with 2/6-VLPs plus CT-CRM_{E29H} orally and intranasally as described above. On week 26, immunized and control mice were challenged and PRAS calculated as described above. In the oral group (n=4), one mouse died before challenge (n=3 for protection).

### Detailed Description of the Invention

The utility of mutant forms of CT as adjuvants for antigenic compositions is described herein. A set of mutant CT clones (CT-CRMs) in *E. coli* was generated. The data indicate that the CT-CRM with superior adjuvanting properties is the mutant with a nonconservative amino acid substitution (glutamic acid to histidine) at position 29 in the A subunit (CT-CRM_{E29H}). The cumulative data demonstrate that CT-CRM_{E29H} is a holotoxin and is less toxic than wild-type CT. Importantly, CT-CRM_{E29H} is able to augment mucosal and systemic immune responses following either intragastric (IG) or intranasal (IN) administration of disparate vaccine antigens. These vaccine antigens are from either bacterial or viral pathogens. Results in the murine models of *Helicobacter felis,* rotavirus and respiratory syncytial virus (RSV) infection indicate that the immune responses facilitated by intragastric or intranasal immunization with a CT-CRM_{E29H}-Prepared vaccine are protective. The data indicate that CT-CMR_{E29H} is at least as active as an adjuvant as wild-type CT- Even in the presence of pre-existing anti-CT immune responses, CT-CRM_{E29H} is able to serve as a mucosal adjuvant.

The mutant CT-A retained its ability to assemble with CT-B to form a holotoxin that resembled wild-type CT in its adjuvanticity, but exhibited reduced toxicity compared to a wild-type CT. The B subunits may have their native sequence or may themselves be mutated.

The resulting reduced level of toxicity provides an altered CT for use as an adjuvant. The immunogenic mutant CT according to the present invention exhibits a balance of reduced toxicity and retained adjuvanticity, such that the protein functions as an adjuvant while being tolerated safely by the vertebrate host immunized with the antigenic composition.

The antigenic compositions of the present invention modulate the immune response by improving the vertebrate host's antibody response and cell-mediated immunity after administration of an antigenic composition comprising a selected antigen from a pathogenic bacterium, virus, fungus or parasite and an effective adjuvanting amount of a mutant CT, where the CT has reduced toxicity compared to a wild-type CT and the glutamic acid at position 29 of the A subunit of the cholera holotoxin is replaced by histidine.

As used herein, the term "the holotoxin has reduced toxicity" means that the CT-CRM mutant, such as the CT-CRM_{E29H} mutant, exhibits a substantially lower toxicity per unit of purified toxin protein compared to the wild-type CT, which enables the mutant to be used as an adjuvant in an antigenic composition without causing significant side effects.

As used herein, the term "effective adjuvanting amount" means a dose of the CT-CRM mutant, such as the CT-CRM_{E29H} mutant, which is suitable to elicit an increased immune response in a vertebrate host. The particular dosage will depend upon the age, weight and medical condition of the host, as well as on the method of administration. Suitable doses are readily determined by persons skilled in the art.

Five CT-CRMs were generated as described in Example 1 below with the following mutations in the A subunit:

| Amino Acid | Native | Mutant | Abbreviation |
|---|---|---|---|
| 7 | arginine | lysine | CT-CRM_{R7K} |
| 11 | arginine | lysine | CT-CRM_{R11K} |
| 29 | glutamic acid | histidine | CT-CRM_{E29H} |
| 110 | glutamic acid | aspartic acid | CT-CRM_{E110D} |
| 112 | glutamic acid | aspartic acid | CT-CRM_{E112D} |

The phenotypic effects of these mutations on structure and function of CT were then assessed.

The variant CT-A's R7K, E29H, E110D and E112D were able to assemble into immunoreactive holotoxin as determined by a ganglioside GM₁ binding assay (Figure 1). However, a portion of purified R11K did not appear to be a holotoxin when tested with the polyclonal antibodies described in Example 2.

Each holotoxin variant was tested in a Y-1 adrenal tumor cell assay (19) to determine its residual toxicity compared to wild-type CT holotoxin. The results presented in Table 2 demonstrated that CT-CRM_{E29H} and commercial CT-B (Sigma) had 1.2% residual toxicity. The 1.2% residual toxicity associated with commercial CT-B was most likely due to contaminating A subunit (approximately 0.5%). The residual toxicity of the remaining CT-CRMs with mutations at amino acid positions 7, 11, 110, or 112 were less than or equal to 0.4%.

CT-CRM_{E29H} was tested in the patent mouse gut weight assay (20) to estimate intestinal fluid accumulation as an in vivo measure of toxicity. The results presented in Table 3 demonstrated that CT-CRM_{E29H} was significantly less active in stimulating an increase in fluid accumulation into the intestinal tract of mice than was wild-type CT.

Each CT-CRM was also compared to CT in an ADP-ribosyltransferase activity assay. The results were generally in agreement with those generated in the Y-1 adrenal cell assay and suggested that mutation in the A1 subunit resulted in diminished ADP-ribosyltransferase activity by the various CT-CRMs when compared to wild-type CT (Table 4). The mutant with the largest enzyme activity appeared to be CT-CRM_{E29H}. This activity was approximately 10% that of wild-type CT.

Trypsinization at 37°C of CT-CRM_{E29H} caused cleavage of CT-A into fragments A1 and A2 in a manner indistinguishable from treatment of wild-type CT based on Western blot analyses. This provides further evidence that the structure of CT-CRM_{E29H} is similar to that of wild-type CT.

The apparent differences in activity of CT-CRM_{E29H} in the Y-1 adrenal tumor cell and ADP-ribosylation activity assays are due to trypsin activation of the mutant holotoxin in the latter assay. Thus, the lack of CT-A cleavage into A1 and A2 subunits due to the reduced protease activity in *E. coli* contributes to the attenuation of the *E. col*i-expressed CT-CRM_{E29H}. Collectively, the accumulated data show that CT-CRM_{E29H} is a holotoxin that binds to ganglioside GM₁ and is significantly less toxic than wild-type CT.

A series of studies was conducted to evaluate the efficacy of CT-CRM_{E29H} as a mucosal adjuvant for compositions containing bacterial or viral antigens which have been identified as vaccine candidates as follows: (1) Nontypable *Haemophilus influenzae* (NTHi) recombinant P4 protein, also known as protein "e" (rP4) (21), recombinant NTHi P6 protein (rP6) (22), and purified native *Haemophilus influenzae* adherence and penetration (Hapₛ) protein (23); (2) *Helicobacter pylori* recombinant Urease protein (rUrease) (24); (3) *Neisseria meningitidis* Group B recombinant class 1 pilin (25) and *Neisseria meningitidis* Group B class 1 outer membrane protein (26); (4) Respiratory syncytial virus purified native fusion protein (RSV F) (27); and (5) 2/6-virus-like particles of rotavirus (28).

CT-CRM_{E29H} was compared to four other CT mutants and wild-type CT as an adjuvant for the NTHi rP4 and rP6 proteins. The results indicated that the five different CT-CRMs augmented the capacity of rP4 and rP6 proteins to elicit systemic humoral immune responses (Tables 5 and 6). For example, two weeks after tertiary IN immunization the anti-rP4 IgG antibody titers of mice immunized with rP4 and rP6 proteins formulated with either CT-CRM_{E29H} or CT-CRM_{E110D} were 40 times greater that of mice immunized with the recombinant proteins in PBS alone (Table 5). The antibody titers of mice administered the recombinant proteins plus wild-type CT holotoxin were elevated 20-fold. The anti-rP4 antibody titers of mice immunized with the CT-CRM_{R11K} were elevated 10-fold.

Even more dramatic differences were observed when the sera were examined for anti-native P6 antibody titers (Table 6). Two weeks after secondary IN immunization the serum anti-native P6 antibody titers of mice immunized with either the CT-CRM_{E29H} or CT-CRM_{E110D} formulated vaccines were more than 30 times greater than that of mice immunized with rP6 plus PBS. In comparison, the vaccine prepared with wild-type CT elicited anti-native P6 antibody titers that were 90 times greater than that generated by the PBS prepared formulation. The anti-native P6 antibody titers of mice immunized with either the CT-CRM_{E112D}, CT-CRM_{R7K'} or CT-CRM_{R11K} preparations were only two to four times greater than that of recipients immunized with rP4 plus rP6 formulated with PBS alone.

An examination of the protein-specific antibodies in the mucosal secretions two weeks after tertiary immunization further indicated that the CT-CRMs facilitated the generation of local immune responses against the rP4 protein. Moreover, the anti-rP4 antibody titers were comparable to those induced by wild-type CT (Table 7). Local antibody titers were not detected against native P6 protein (data not shown). Thus, the data when taken together suggested that the most propitious mutant CTs for generating both systemic and local antibody responses against rP4 and rP6 proteins were the CT-CRMs which contained a mutation at either position 29 or 110.

An additional study was performed to confirm the potential of CT-CRM_{E29H} as an adjuvant and determine the appropriate dose for IN immunization (Table 8).
The results indicated that 1 µg of CT-CRM_{E29H} facilitated the greatest systemic and local humoral immune responses against rP4 protein. When the dose of CT-CRM_{E29H} was increased from 1 to 10 or 30 µg per dose, the data suggested that both systemic and mucosal immune responses were diminished. For example, the serum anti-P4 IgG antibody titers of mice immunized with 10 µg CT-CRM_{E29H} was one-seventh that of mice immunized with the 1 µg CT-CRM_{E29H} on day 48 of the study (Table 8). Moreover, the local anti-P4 IgA antibody titers from the bronchoalveolar and vaginal wash fluids of the former group were one-thirty-fourth and one-sixteenth that of the latter group of mice on day 49. The data indicated that the local and systemic humoral immune responses of mice immunized with rP4 plus rP6/CT-CRM_{E29H} were essentially identical to those attained after immunization with the wild-type CT adjuvanted vaccine (Table 8).

The effect of the addition of CT-CRM_{E29H} on the serum antibody responses elicited by immunization with the Hapₛ protein was examined. Addition of CT-CRM_{E29H} helped induce a serum antibody response to the Hapₛ protein (Table 9). The immune response was seen in week 7 sera; no antibody titers were detected in earlier sera. The anti-Hapₛ ELISA titers of the sera obtained from immunized mice are shown in Table 9. The responses increased in a dose dependent manner and were augmented approximately three-fold by adddition of 0.1 µg of CT-CRM_{E29H}. This augmentation occurred at both dosage levels.

The potential of the five different CT-CRMs to augment systemic and local humoral immune responses after intragastric (IG) immunization with the rUrease protein of *H. pylori* was assessed using a mouse model (29). The results were similar to those obtained with the NTHi proteins after intranasal administration. The data indicated that CT-CRM_{E29H} was the mutant with the most potential for augmenting systemic and local humoral immune responses after IG immunization. The geometric mean serum anti-rUrease IgG (Table 10) and IgA (Table 11) antibody titers elicited by the CT-CRM_{E29H} formulated vaccine were six and three times greater, respectively, than those induced by CT-CRM_{E110D} on day 28 of the study. Furthermore, the serum IgG and IgA antibody titers elicited by the CT-CRM_{E29H} formulated vaccine were equivalent to that generated by the vaccine containing wild-type CT.

Most importantly, IG immunization with the rUrease formulated with CT-CRM_{E29H} appeared to generate the greatest local humoral immune responses (Table 12). This was most evident after the examination of the bronchoalveolar wash fluids. The anti-rUrease IgA antibody titers in the bronchoalveolar wash fluids were five times greater than that elicited by the CT-CRM_{E110D} prepared vaccine. In comparison to the wild-type CT formulation, the anti-rUrease IgA antibody titers were at one-fifth the level. However, the protein-specific IgA antibody titers in the vaginal wash fluids of the group immunized with the CT-CRM_{E29H} formulated vaccine were essentially equivalent to those elicited by the wild-type CT prepared vaccine (Table 12).

It was noteworthy that the data imply that parenteral immunization did not elicit remarkable rUrease-specific IgA antibodies in the bronchoalveolar wash fluids when compared to those elicited in mice immunized IG with the CT-CRM_{E29H} prepared vaccine (Table 12).

Therefore, a second study was conducted to test the efficacy of the immune responses generated by rUrease formulated with CT-CRM_{E29H}. The data suggest that CT-CRM_{E29H} is as potent as wild-type CT in supporting the induction of protective immune responses against *H. felis* (Table 13). The serum anti-rUrease IgA antibody titers of the former group were equivalent to those of the latter group of mice on day 28 of the study. The protein-specific IgA antibody titers in the sera of mice parenterally immunized with rUrease plus Stimulon^{™} QS-21 were 12 times greater than those of mice immunized IG with the CT-CRM_{E29H} prepared vaccine. However, the protein-specific IgA antibody titers in the bronchoalveolar wash fluids of mice immunized with CT-CRM_{E29H} were more than ten times greater than those of parenterally immunized mice (Table 13).

The results suggested a correlation between IG immunization and the ability of mice to clear *H. felis* from the stomach tissue. Ten days after the last challenge, 80% of the of mice immunized IG with vaccines formulated with either CT or CT-CRM_{E29H} were able to clear urease-containing bacteria from the stomach tissues. In contrast, naive control mice (10%), mice immunized IG with rUrease plus PBS alone (20%), or mice immunized subcutaneously with rUrease admixed with Stimulon^{™} QS-21 (30%) appeared to have less ability to eradicate *H. felis* (Table 13). It was noteworthy that the data did not suggest a relationship between efficacy and protein-specific IgA antibody titers in the bronchoalveolar wash fluids. The protein-specific IgA antibody titers in the bronchoalveolar wash fluids of mice immunized IG with rUrease plus wild-type CT were one-tenth those of mice immunized with CT-CRM_{E29H} (Table 13). Yet 80% protection was achieved with either vaccine. Thus, monitoring local humoral immune responses in the pulmonary tissues may have little relevance to protective immune responses that occur in the stomach.

It has been suggested by Dr. Jani O'Rourke (University of New South Wales; personal communication) that C57B1/6 mice, unlike BALB/c mice, experience disease similar to that observed in humans after infection with *H. pylori.* To test the efficacy of the anti-rUrease immune responses facilitated by CT-CRM_{E29H} to clear *H. pylori* from the gastric tissues, a separate series of studies were initiated using C57B1/6 mice. The results suggested that IG immunization with rUrease formulated with CT-CRM_{E29H} generated systemic and local humoral immune responses that were similar to those elicited by rUrease formulated with wild-type CT (Table 14). The serum and bronchoalveolar and vaginal wash fluid anti-rUrease IgA antibody titers of mice immunized with either wild-type CT or CT-CRM_{E29H} prepared vaccines on day 28 of the study were indistinguishable. The only disparities were the IgA antibody titers detected in extracts of the fecal pellets from the mice immunized with the CT-CRM_{E29H} prepared vaccine (Table 14), which were three times greater. It was noteworthy that the protein-specific IgA antibody titers in the feces of mice parenterally immunized with rUrease plus alum were substantially lower than those of mice IG immunized with either wild-type CT or CT-CRM_{E29H} formulations (one thirty-eighth and one-fourteenth, respectively). Thus, the C57Bl/6 mouse model appeared capable of assessing the capacity of CT-CRM_{E29H} to adjuvant immune responses generated after IG immunization. Moreover, the data indicated that the model was capable of defining the roles of local and systemic immune responses in protecting mucosal surfaces from *H. pylori.*

It has been reported that CT is contraindicated as an adjuvant for mucosal immune responses (30,31). The hypothesis was that CT predisposed vaccines to elicit heightened IgE antibody titers, which are undesirable. IgE is associated with hypersensitivity and allergic reactions. It was further implied that the heat-labile toxin of *E. coli* (LT), or LT-CRMs, had less potential to elicit heightened IgE antibody titers. Hence, the conclusion was that LT or LT-CRMs are more appropriate vaccine adjuvants for the generation of mucosal immune responses. To test this hypothesis, vaccines composed of rUrease were formulated with either CT, LT, or CT-CRM_{E29H} and tested in C57B1/6 mice for their ability to elicit IgE antibodies after IG immunization (Table 15). The data suggested that vaccines prepared with either CT-CRM_{E29H} or wild-type CT were less likely than wild-type LT to generate total or urease-specific IgE antibodies in the circulation. Indeed, the implication was that vaccines formulated with CT-CRM_{E29H} were less likely to generate elevated IgE antibody titers. Both the endpoint total and the rUrease-specific IgE antibody titers were one-fourth those of mice immunized with the vaccine prepared with wild-type LT (Table 15). Thus, these data suggest that, at least in a rUrease formulation, CT-CRM_{E29H} is preferred over LT as an adjuvant.

Without being bound by theory, the mechanism of LT activity recently proposed by van den Akker et al. (32) presents a more likely explanation for the reduced toxicity of CT variants altered in or around E29. After cleavage of the disulfide loop and reduction of the disulfide bond linking domains A1 and A2, the loop consisting of residues 30-33 in the A1 domain is proposed to change position as a consequence of movement of the long helix of domain A2. Substitutions for E29 may alter the behavior of loop 30-33, resulting in decreased activation of CT-A1. A potential explanation for the reduced toxicity of CT-Y30WAH and CT-G34GGP may also be explained by effects on the 30-33 loop that are detrimental to activation of CT-A1. The next step in the proposed activation pathway is movement of the entire 25-36 loop, which disrupts interactions between R25 and Y55. CT-R25G showed a reduction in toxicity to a greater extent than CT-R25W, possibly because the side chains of R25 and Y55 participate in hydrophobic interactions, which may be retained by CT-R25W but not CT-R25G. The phenotypes of our variants are consistent with the model for activation of heat-labile enterotoxins proposed by van den Akker et al. (32).

A series of experiments was conducted to evaluate the efficacy of CT-CRM_{E29H} as a mucosal and parenteral adjuvant for two vaccine candidates from *Neisseria meningitidis* Group B. The first candidate was a recombinant class 1 pilin (rpilin) (25). The second candidate was a class 1 outer membrane protein (PorA) expressed by a mutant meningococcal strain that did not express the class 2/3 protein (26).

A mucosal adjuvant effect was shown in a first experiment, in that rpilin-specific serum IgG antibodies were enhanced in CT-CRM_{E29H} added groups, ranging from 3 to 19-fold increases in comparison to the titers obtained in mice receiving rpilin in saline (Table 16). Specific serum IgA also increased 2 to 5-fold in the mice immunized with rpilin delivered in CT-CRM_{E29H} (at both 0.1 and 1.0 µg). It is noteworthy that CT-CRM_{E29H} (1 µg) increased rpilin-specific IgA 3 to 10-fold in the nasal, vaginal, and bronchoalveolar washes. Furthermore, IN immunization with rpilin plus CT-CRM_{E29H} significantly reduced nasal colonization by the Group B *N. meningitidis* homologous strain to the level of detection in Swiss-Webster mice (Figure 2).

A second experiment was conducted to demonstrate that CT-CRM_{E29H} enhanced protection of rpilin against a homologous meningococcal strain. As shown in Table 17, sera IgG titers of meningococcal B whole cell ELISA were increased in the rpilin plus CT-CRM_{E29H} group at least four-fold compared to the homologous strain, as well as to heterologous strains FAM18 and M982 in comparison to the titers from mice receiving rpilin alone. As a control, mice immunized with KLH plus CT-CRM_{E29H} did not induce sera IgG in whole cell ELISA to any of the strains tested. In Table 18, rpilin-specific IgG and IgA antibodies were substantially increased in the rpilin plus CT-CRM_{E29H} group, as compared to the unadjuvanted group.
Moreover, CT-CRM_{E29H} as a mucosal adjuvant for rpilin protected mice against nasal colonization by the homologous Group B meningococcal strain (Figure 3).

Next, the immunogenicity of PorA plus CT-CRM_{E29H} in IN immunization was demonstrated. The group receiving PorA adjuvanted with CT-CRM_{E29H} generated increased serum IgG antibodies to *N. meningitidis* H44/76 whole cells and generated 7 and 14-fold higher PorA-specific antibodies compared to the unadjuvanted PorA group (Table 19). However, serum IgA antibodies to PorA H44/76 were not detectable. There were also no PorA-specific antibodies detected in any of the mucosal secretion samples collected (Table 19).

A fourth experiment was conducted to demonstrate that CT-CRM_{E29H} enhanced protection by either rpilin or PorA against a heterologous meningococcal strain. The data, particularly from Table 20, indicate that IN administration of class 1 rpilin or PorA delivered with CT-CRM_{E29H} not only elicited high serum antibody titers to antigens and meningococcal whole cells, but also protected Swiss-Webster mice against nasal colonization by a heterologous strain of group B *N. meningitidis.* Specifically, CT-CRM_{E29H} enhanced serum antibody response to class 1 rpilin as compared to that of the unadjuvanted group. Similarly, the adjuvanted class 1 rpilin group provided enhanced nasal clearance of the bacteria.

Next, the ability of CT-CRM_{E29H} to serve as an adjuvant for meningococcal parenteral immunization was examined. As shown in Figure 5, PorA H355 adjuvanted with either MPL^{™} or CT-CRM_{E29H} significantly reduced nasal colonization of Group B meningococcal heterologous strain 870227. In particular, mice immunized subcutaneously with PorA H355 plus CT-CRM_{E29H} had even significantly fewer colonies than the PorA H355 plus MPL^{™} group in the nose 24 hours post-challenge. However, bactericidal activities were detected in the sera only from the PorA and the heat killed whole cell adjuvanted with MPL^{™} immunized groups respectively (Table 22), but not from the PorA plus CT-CRM_{E29H} group. Even though PorA adjuvanted with CT-CRM_{E29H} did not elicit homologous bactericidal activities similar to that of MPL^{™} adjuvant, it was highly efficacious in reducing the colonization by a heterologous Group B meningococcal strain.

The capacity of CT-CRM_{E29H} to augment systemic and mucosal immune responses against respiratory syncytial virus (RSV) glycoproteins was examined using the purified native fusion (F) protein. In addition, the impact of pre-existing anti-CT antibodies on the potency of CT-CRM_{E29H} as an adjuvant was investigated. The results demonstrated that BALB/c mice immunized IN with F protein adjuvanted with either CT or CT-CRM_{E29H} generated systemic and local anti-CT IgG and IgA antibody titers (Table 23). Moreover, the data indicated that the antibody titers generated by the formulation containing CT-CRM_{E29H} were equivalent to those elicited by the formulation containing wild-type CT. For example, 10 days after secondary immunization with F protein/CT-CRM_{E29H} (1 µg per dose), the serum anti-CT IgA and IgG antibody titers were only slightly lower than those of mice immunized with F protein/CT (1 µg per dose). Similar results were also obtained after examination of the vaginal wash fluids from mice immunized with F protein prepared with either 1 or 10 µg CT-CRM_{E29H} (Table 23). The data therefore suggested that CT-CRM_{E29H} was as immunogenic as wild-type CT.

The question of whether anti-CT immune responses could adversely affect the immunogenicity of the F antigen was addressed in a second experiment where BALB/c mice were primed first by two IN administrations with either wild-type CT or CT-CRM_{E29H} in PBS alone (Table 24). Thereafter, the appropriate mice were immunized twice with F protein admixed with either wild-type CT or CT-CRM_{E29H}. An examination of the sera collected two weeks after the last administration (day 56) indicated that pre-existing anti-CT antibodies did not have a negative impact on the level of local or systemic anti-F protein IgA and IgG antibodies. Indeed, the data indicated that pre-existing anti-CT antibodies were beneficial for the generation of an augmented anti-F protein antibody response. This was most evident when the anti-F protein antibody titers elicited at mucosal surfaces were compared (Table 24). Two weeks after secondary immunization, the anti-F protein IgA antibody titers in the bronchoalveolar and vaginal wash fluids of mice primed first with CT-CRM_{E29H} and then immunized with F protein/CT-CRM_{E29H} were 7 and 17 times greater, respectively, than that of naive mice immunized solely with F protein/CT-CRM_{E29H} (Table 24) .

In a third experiment, systemic and mucosal immune responses of BALB/c mice immunized IN with RSV F protein and CT-CRM_{E29H}, CT-B or alum were assessed. Table 25 sets forth the humoral immune responses of sera collected nine days post-tertiary immunization. Mice that had received immunizations containing F protein and either 1 or 10 µg of CT-CRM_{E29H} (groups 777 and 778, respectively) displayed significantly elevated titers for IgG, IgG1 and IgG2a when compared to mice immunized with F/PBS, F/AlOH or RSV (groups 784, 785 and 907, respectively). In addition, the titers generated by vaccines containing F/CT-CRM_{E29H} (777 and 778) were at least equivalent to those stimulated by F protein and CTB (779 and 780).

Bronchoalveolar lavage fluids, vaginal and nasal washes were collected from the immunized animals one week post-final immunization in order to perform IgG and IgA antibody ELISAs. The data, set forth in Table 26, show titers from pools of five mice. Mice immunized with CT-CRM_{E29H} elicited detectable IgA in both vaginal and nasal washes (groups 777 and 778). IgA was not seen in BALs derived from mice immunized with CT-CRM_{E29H} and this represented a contrast to that seen in the BAL of RSV immunized mice (group 907) and F/CTB immunized mice (780). IgG was seen in all mucosal washes including that from the BAL. The levels of IgG seen in the washes from CT-CRM_{E29H} immunized mice were comparable to those obtained by immunizing with CTB (groups 779 and 780) and live RSV.

In a fourth experiment, the cytolytic (CTL) activity elicited by in vitro stimulated spleen cells derived from immunized mice was assessed. The data are presented in Figure 6. Whereas RSV-immunized mice showed antigen-specific cell lysis of approximately 60%, the CTL activity of each of the remaining mice remained less than 20%. Thus, whereas CT-CRM_{E29H} was able to induce both systemic and mucosal humoral immune responses to RSV F protein (Tables 25 and 26), cell-mediated immune responses to RSV-infected target cells were not observed.

In a fifth experiment, viral protection assays were performed in order to investigate whether the intranasal delivery of F/CT-CRM_{E29H} facilitates protection against live RSV challenge. The data are presented in Figure 7.
Statistical analysis by ANOVA of the results depicted in Figure 7 is as follows:
P < 0.05: F/PBS versus F/CT-CRM_{E29H} (1 µg and 10 µg CT-CRM_{E29H}), F/CTB (1 µg and 10 µg), F/AlOH.
p > 0.05: PBS/CT-CRM_{E29H} versus F/PBS
p > 0.05: F/CT-CRM_{E29H} (1 µg and 10 µg CT-CRM_{E29H}) versus F/CTB (1 µg and 10 µg) versus F/AlOH.

Mice that received IN vaccines containing F/CT-CRM_{E29H} or F/CTB had lung viral titers comparable to those achieved in mice immunized intramuscularly with F/AlOH (p>0.05). Furthermore, intranasal immunization with F/CT-CRM_{E29H} was seen to reduce lung virus titers by Log₁₀ 1.6 and Log₁₀ 1.4 compared to IN immunization with F/PBS or PBS/ CT-CRM_{E29H}, respectively. The differences between F/CT-CRM_{E29H} and F/PBS or PBS/CT-CRM_{E29H} were found to be statistically significant (p<0.05).

In a sixth experiment, systemic and mucosal immune responses of BALB/c mice immunized IN with RSV F protein and CT-CRM_{E29H} or alum were assessed. Table 27 sets forth the humoral immune responses of sera collected two weeks post-tertiary immunization. Mice that received immunizations containing F protein and 1 µg of CT-CRM_{E29H} (group 256) displayed significantly elevated titers for IgG, IgG1 and IgG2a when compared to mice immunized with F/PBS or PBS/CT-CRM_{E29H} (groups 250 and 257, respectively). No significant differences were observed in the IgG1 titers between mice immunized with F/CT-CRM_{E29H} (256) and F/AlOH (258). However, IN immunization with F/CT-CRM_{E29H} (256) elicited significantly elevated IgG2a titers compared to those seen by immunization with F/AlOH (258). Collectively, these results are in agreement with those presented in Table 25. Whereas serum IgA was detected in groups of mice receiving F/CT-CRM_{E29H}, the titers were much lower than previously observed (16,202±2,031 for group 777 and 444±1,458 for group 256). The reasons for the apparent difference are not clear. Nevertheless, the ability of F/CT-CRM_{E29H} delivered IN to induce serum IgA is consistent in both studies and contrasts favorably with the capacity of F/AlOH in this regard.

Bronchoalveolar lavage fluids, vaginal and nasal washes were collected from the immunized animals two weeks post-final immunization in order to perform IgG and IgA antibody ELISAs. The data, set forth in Table 28, show titers from pools of five mice. Similar to the results shown in Table 26, mice immunized with CT-CRM_{E29H} elicited detectable IgA in both vaginal and nasal washes (group 256). Again similar to the data presented in Table 26, IgA was not seen in BALs derived from mice immunized with CT-CRM_{E29H}. However, IgG was seen in all of the mucosal washes including that from the BAL. The levels of IgG observed in the washes from F/CT-CRM_{E29H}-immunized mice were at least comparable to those obtained by immunizing with live RSV (Table 28, groups 256 versus 259).

In a seventh experiment, the cytolytic (CTL) activity elicited by in vitro stimulated spleen cells derived from immunized mice was assessed. The data are presented in Figure 8. Whereas RSV-immunized mice showed antigen-specific cell lysis of approximately 45%, the CTL activity of each of the remaining mice remained less than 10%. The data confirm the inability of IN immunization with F/CT-CRM_{E29H} to induce a cell-mediated immune defense mechanism against RSV-infected target cells in the splenic lymphocyte population. This confirms the previous observation (Figure 8).

In an eighth experiment, additional viral protection assays were performed in order to investigate whether the IN delivery of F/CT-CRM_{E29H} facilitates protection against live RSV challenge. Statistical analysis by ANOVA of the results depicted in Figure 9 is as follows:
p < 0.05: F/PBS versus F/CT-CRM_{E29H}, F/AlOH, RSV.
p < 0.05: Naive versus F/CT-CRM_{E29H}, F/AlOH, RSV.
p > 0.05: F/CT-CRM_{E29H} versus F/AlOH, RSV.

Similar to the results depicted in Figure 7, mice that received IN vaccines containing F/CT-CRM_{E29H} controlled virus replication in the lungs to an extent which was statistically comparable (p > 0.05) to that achieved in mice immunized intramuscularly with F/AlOH or IN with live RSV (Log₁₀ 1.87 versus Log₁₀ 1.99 and Log₁₀ 1.94, respectively). IN immunization with F/PBS (first bar), or unimmunized mice (naive) displayed lung viral titers of Log₁₀ 4.5 and Log₁₀ 4.3, respectively. Furthermore, these groups had lung virus titers that were found to be statistically elevated (p < 0.05) when compared to the virus titers obtained from mice immunized with F/CT-CRM_{E29H}, F/AlOH or live RSV. Therefore, the data support the conclusion that IN instillation of F/CT-CRM_{E29H} protects against infectious RSV challenge.

In a ninth experiment, the anti-F serum antibody response was assessed. The results showed that anti-F protein IgG was significantly increased in mice immunized with F/CT-CRM_{E29H} (0.1 or 1.0 µg) compared to those given F protein delivered in PBS alone (Table 29). In addition, F protein adjuvanted with either 0.1 or 1.0 µg CT-CRM_{E29H} was at least as effective as either F/AlOH (intramuscular) or experimental infection with RSV in stimulating anti-F protein IgG responses. The magnitude of the anti-F protein antibody titers was dependent on the dose of CT-CRM_{E29H} in the formulation, such that titers were significantly greater in mice that received 1.0 µg CT-CRM_{E29H} versus 0.01 µg. In comparison to F/PBS, both anti-F protein IgG1 and IgG2a titers were augmented with either 0.1 or 1.0 µg CT-CRM_{E29H}. CT-CRM_{E29H} stimulated both type 1 and type 2 immune compartments. Significantly higher serum anti-F protein IgA responses were stimulated by IN immunization with F/CT-CRM_{E29H} (0.1 or 1.0 µg) compared to experimental infection with RSV. In contrast, serum IgA anti-F protein antibodies were not observed in response to F/PBS (IN) or the parenteral administration of F/AlOH (Table 29).

Anti-CT titers also followed a dose-dependent pattern consistent with the anti-F protein titers (Table 29). Statistically equivalent anti-CT titers were observed in sera obtained from mice immunized with either CT-CRM_{E29H} (1.0 µg) or F/CT-CRM_{E29H} (1.0 µg). However, these titers were significantly elevated compared to F/CT-CRM_{E29H} (0.1 or 0.01 µg). In addition, the anti-CT titers in sera of mice immunized with F/CT-CRM_{E29H} (0.1 µg) were statistically heightened compared to titers from mice immunized with F/CT-CRM_{E29H} (0.01 µg). Therefore, the adjuvant effect of CT-CRM_{E29H} for anti-F protein antibody responses is correlated (r = 0.97) with the antibody response to the mutant cholera holotoxin.

In this ninth experiment, mucosal immunity was also assessed. Mucosal IgA was observed only in pooled nasal washes (NW) from mice immunized with either F/CT-CRM_{E29H} (1.0 µg) or F/CT-CRM_{E29H} (0.1 µg) (Table 30). In addition, mice that received IN immunizations containing purified F protein and CT-CRM_{E29H} (0.01 to 1.0 µg) also had anti-F protein IgA in vaginal washes (VW). F protein-specific IgG was observed in the bronchoalveolar lavage (BAL), VW and/or NW of mice that received F/CT-CRM_{E29H} (0.1 and 1.0 µg), or F/AlOH. In contrast, anti-F protein IgA was not detected in mice immunized IM with F/AlOH.

In a tenth experiment, functional immunity in mice immunized with F protein formulated with CT-CRM_{E29H} was assessed. In the presence of complement, statistically heightened anti-RSV neutralizing antibodies were detected in the sera of mice that had received F protein and either 0.1 or 1.0 µg of CT-CRM_{E29H}, F/AlOH or RSV A2, compared to the administration of F/PBS or CT-CRM_{E29H} alone (Table 31). In the absence of complement, no detectable neutralizing titers were observed in any of the groups (log₁₀ < 1.3). Consistent with the serum and mucosal antibody data (Tables 29 and 30), immunization with F/CT-CRM_{E29H} (0.01 µg) was not sufficient to generate anti-RSV neutralizing antibodies.

In an eleventh experiment, immunized mice were challenged two weeks after tertiary immunization, in order to determine the ability of F/CT-CRM_{E29H} to protect against subsequent infection. The results demonstrate that mice immunized with F/CT-CRM_{E29H} (0.1 or 1.0 µg) were protected (Table 32). In comparison to naive mice, or those immunized with F/PBS or CT-CRM_{E29H} alone, the lungs of mice immunized with F protein and either 0.1 or 1.0 µg CT-CRM_{E29H} had significantly reduced virus levels. In addition, significantly reduced virus levels were observed in the nasal tissues of mice immunized with F/CT-CRM_{E29H} (0.1 and 1.0 µg) compared to non-immunized naive mice or those immunized with F/PBS. In contrast, mice immunized parenterally with F/AlOH displayed reduced viral titers in lung tissue compared to F/PBS immunized mice, but no significant reduction in nasal tissue. Overall, the IN administration of F/ CT-CRM_{E29H} (0.1 or 1.0 µg) was sufficient to generate both local and systemic humoral immune responses that may have contributed to the protection of respiratory tissue against subsequent live RSV challenge.

The data presented in Example 10 have illustrated a viable approach to the development of an IN vaccine for RSV F protein. The data indicate that the production of both humoral and mucosal IgG and IgA is stimulated by the IN delivery of F/CT-CRM_{E29H}. That the antibody titers observed were significant is demonstrated in two ways: First, each of the humoral and mucosal antibody titers that were analyzed in mice immunized with F/CT-CRM_{E29H} were qualitatively similar and quantitatively elevated compared to mice immunized with F/PBS. Second, the elevated titers are translated into a biologically relevant immune response, as indicated by the observed level of protection displayed in Figures 7 and 9. Immunization with F/CT-CRM_{E29H} significantly enhanced protection against live RSV challenge compared to immunization with F/PBS or PBS/CT-CRM_{E29H}.

Collectively, the data suggest a mechanism involving the neutralization of infectious virus by either mucosal or humoral immunoglobulins, that are stimulated in response to the IN immunization protocol containing F/CT-CRM_{E29H}.

Mice were immunized with another viral antigen, rotavirus, in the form of recombinantly-expressed VP2 and VP6 proteins. Recombinant baculovirus vectors expressing SA11 rotavirus VP2 and VP6 were constructed as described previously (28); it is known that recombinantly-expressed rotavirus structural proteins self-assemble into particles that are morphogenically indistinguishable from virions. The proteins so expressed co-assembled into 2/6-virus-like particles (VLPs).

Inbred BALB/c mice with a homogeneous genetic background were used, anticipating that they all would be responsive to immunization and thus clarify the profile of systemic and mucosal immunoglobulins to VLPs alone and when combined with CT-CRM_{E29H} adjuvant. Genetically heterogeneous outbred CD-1 mice were also used to determine the effect of genetic diversity on the factors involved in induction of immunity and protection.

Sera of all immunized BALB/c and CD-1 mice, except for two unresponsive orally immunized CD-1 mice, contained antibodies to both VP2 and VP6. Pre-immunization sera as well as sera of unimmunized mice detected no viral antigen in mock and or VP2-6 baculovirus infected cells. 2/6-VLP immunized sera or mAbs specific for VP6 and VP2 exposed to uninfected cells also demonstrated no reactivity (data not shown). Immunogenicity of 2/6-VLP was also confirmed by Western blot analysis using pre-challenged and immunized serum from each mouse against 2/6-VLP as well as SA11 strain of rotavirus (data not shown).

Patterns of rotavirus-specific serum IgG, IgM and IgA in the group immunized IN with 2/6-VLPs alone were similar to those in the group immunized with 2/6-VLPs and CT-CRM_{E29H} (Figure 10). However, week 13 levels of the three serum antibody isotypes were significantly higher in the animals receiving 2/6-VLPs together with CT-CRM_{E29H} (Figure 10) (P=0, P=0.004, and P=0.02 for IgG, IgM and IgA respectively). This indicated that CT-CRM_{E29H} had significantly enhanced humoral responses to 2/6-VLPs. Week 13 was selected as indicative of antibody levels generated following two immunizations but prior to challenge.

As shown in Figure 11A, BALB/c mice given VLPs IN produced a greater rotavirus specific systemic IgG response than those immunized orally. Statistically significant differences in serum IgG titers between the two groups were evident at 13 weeks (P=0). Similarly, serum IgM levels were higher in the IN group as opposed to the oral group when pre-challenge (week 13) values were analyzed (P=0) (Figure 11B). Serum IgM levels peaked by week 2 and decreased by week 4 in the IN and mixed groups, whereas in the oral group, low but relatively constant levels of serum IgM were detected throughout the study. Peak serum IgA in orally and IN immunized animals occurred on week 4. No significant differences in serum IgA levels distinguished the three experimental groups when examined at week 13 (Figure 11C). Overall, IN immunization generated higher levels of systemic IgG and IgM responses than oral immunization. Induction of significantly high levels of IgG and IgM in the IN group support the concept that IN immunization may be the preferred route for administration of future vaccine candidates (33). Thus, IN immunization leading to strong systemic neutralizing responses could be effective against viral pathogens that penetrate mucosal barriers.

Both IgG1 and IgG2a were found in the serum of the IN and the orally immunized BALB/c mice (Figure 12). The IN group had statistically significantly higher levels of both IgG subclasses compared to the oral group (IgG1, P=0.005; IgG2a, P=0.05). However, there were no significant differences between subclasses within each group. These data confirm the utility of IN immunization for effective induction of both T-helper pathways (TH-1 and TH-2).

The peak level of fecal IgA for all three experimental groups occurred four weeks after the first immunization (Figure 13A), coinciding temporally with the time at which serum IgA levels were maximal in orally and IN immunized animals (Figure 11C). No statistically significant differences were found among the three immunization protocols when the pre-challenge fecal IgA levels were examined (Figure 13A). Fecal IgG also was maximal on week 4 (Figure 13B); however, the IN group had significantly more IgG on week 13 compared to oral or mixed groups (P=0 and P=0.002, respectively). In general, all 2/6-VLP immunized mice produced serum IgG, IgM and IgA, as well as fecal IgG and IgA. No fecal IgM was detected in any of the animals.

All CD-1 mice receiving 2/6-VLPs IN (n=4) and two of four mice immunized orally produced rotavirus-specific antibody responses (data not shown). To determine the profile of antibody responses more precisely, serum and fecal samples were analyzed weekly for 26 weeks. The induction pattern of serum and fecal antibodies in CD-1 mice was similar to that in the BALB/c mice (Figures 11 and 13).

In BALB/c mice, two IN immunizations with 2/6-VLPs and CT-CRM_{E29H} proved protective (PRAS=98.7%), in contrast to IN immunization with 2/6-VLPs alone (PRAS=39%) (P=0.007) (Figure 14A). The mixed immunization, IN followed by oral immunization, protected mice to an extent similar to oral and IN groups, indicating that in BALB/c mice mixed immunization was also effective. This demonstrated the significant augmentation in protective immune responses due to CT-CRM_{E29H}. BALB/c mice in all three immunization groups showed nearly complete protection from the challenge. PRAS was 99.6%, 98.8% and 98.8% for the oral, IN and the mixed groups, respectively (Figure 14B). The unimmunized control group shed significantly more viral antigen than the three immunized groups (P=0). There were no significant differences between PRAS values for the three immunized groups.

IN immunization induced both systemic and mucosal responses in all immunized CD-1 mice and protected these animals (PRAS=97.9%) (Figure 14C). Only two of four orally immunized CD-1 mice showed systemic and mucosal antibody responses and protection (2 of 3, PRAS 65.8%) (Figure 14C); in contrast, all orally immunized BALB/c mice showed mucosal and systemic responses and were protected. Notably, the CD-1 mouse that did not produce an immune response was not protected from infection, whereas immune-responsive mice were protected (Figure 14C). One mouse in the oral group, with no antibody response to immunization, died prior to the challenge. Two CD-1 mice were used as controls to reduce the number of samples in the analysis of antibody responses. However, statistical analysis clearly showed that the protection results were significant (P=0, at 95% confidence level). The oral immunization experiment was repeated with CD-1 mice under the same conditions, except that animals were challenged on week 13 rather than 26. Using four mice in the immunized groups and five mice as controls, a similar protection level was observed (PRAS=71.2%) (data not shown). Taken together, these results support those recently published by O Neal et al. (34,35), suggesting that in CD-1 mice intranasal immunization is more effective than oral immunization.

In view of this demonstrated utility of CT-CRM_{E29H} as a vaccine adjuvant, production of suitable quantities of this material is desirable. Using pIIB29H (described in Example 1), several attempts were made to express CT-CRM_{E29H} in *E. coli.* The resulting yield of purified CT-CRM_{E29H} holotoxin was approximately 50µg per liter of culture medium. Initial attempts to increase CT-CRM_{E29H} yield via modifications to the original plasmid, pIIB29H, to create plasmid pPX2492 (see Example 1), showed little or no effect. A moderate increase in yield was achieved through co-expression of pIIB29H, and derivatives, with *Vibrio cholerae* DsbA and *E. coli* RpoH. Co-expression and purification modifications increased the yield of CT-CRM_{E29H} to approximately 2 mg per liter.

In order to increase the expression of CT-CRM_{E29H}, the lactose inducible promoter was replaced with an arabinose inducible promoter (Invitrogen Corporation, Carlsbad, CA), which was operatively linked to the DNA sequence encoding CT-CRM_{E29H}. During cloning it was determined that plasmid pIIB29H contained a *ctxA* gene from *Vibrio cholerae* strain 569B, linked to a *ctxB* gene from V.c. strain 2125. Cross alignment of these genes indicated seven base substitutions between the two *ctxB* genes and a single base change between the *ctxA* genes. Several of these base substitutions led to amino acid changes in the mature subunits. Of special note is the substitution between the *ctxA* genes which leads to an amino acid change within the A-2 portion, or the holotoxin assembly domain of the A subunit. It was not known whether the heterogeneity between these genes had a negative impact on toxin expression or holotoxin assembly; however, it was thought preferable from an evolutionary standpoint that both toxin subunit genes originate from the same source. As such, both the *ctxA* and *ctxB* genes used in the construction of the arabinose inducible system originated from *Vibrio cholerae* strain 569B. The construction of plasmid pPX7490 is described in Example 12. Production of CT-CRM_{E29H} from pPX7490 is approximately 30 mg of purified material per liter of culture.

The invention further relates to plasmids containing isolated and purified DNA sequences comprising DNA sequences which encode an immunogenic mutant cholera holotoxin having a substitution other than aspartic acid for the glutamic acid at position 29 of the A subunit of the cholera holotoxin, and wherein such a DNA sequence is operatively linked to an arabinose inducible promoter, as well as to suitable host cells transformed, transduced or transfected with such plasmids by conventional techniques.

A variety of host cell-plasmid vector systems are used to express the immunogenic mutant cholera holotoxin. The vector system, which preferably includes the arabinose inducible promoter, is compatible with the host cell used. Suitable host cells include bacteria transformed with plasmid DNA, cosmid DNA or bacteriophage DNA; viruses such as vaccinia virus and adenovirus; yeast such as Pichia cells; insect cells such as Sf9 or Sf21 cells; or mammalian cell lines such as Chinese hamster ovary cells; as well as other conventional organisms.

A variety of conventional transcriptional and translational elements can be used for the host cell-vector system. The DNA encoding the CT-CRM is inserted into an expression system, and the promoter (preferably the arabinose inducible promoter) and other control elements are ligated into specific sites within the vector, so that when the plasmid vector is inserted into a host cell (by transformation, transduction or transfection, depending on the host cell-vector system used), the DNA encoding the CT-CRM is expressed by the host cell.

The immunogenic mutant cholera holotoxin is produced by transforming, transducing or transfecting a host cell with a plasmid described above and culturing the host cell under conditions which permit the expression of said recombinant immunogenic detoxified protein by the host cell.

Although this invention is exemplified by a CT-CRM mutant having a histidine at amino acid 29, other nonconservative mutations of the wild-type glutamic acid residue are also within the scope of this invention. Glutamic acid is an acidic (negatively charged) molecule. Therefore, a nonconservative mutation will be one in which a substitution is made to an amino acid other than aspartic acid, which is also an acidic molecule. Suitable alternative amino acids include the amino acids lysine and arginine which, like histidine, are basic (positively charged) molecules. Suitable alternative amino acids further include the amino acids with nonpolar functional groups such as alanine, isoleucine, leucine, methionine, phenylalanine, proline, tryptophan and valine, and the amino acids with uncharged polar functional groups such as asparagine, cysteine, glutamine, glycine, serine, threonine and tyrosine.

An effective amount of the mutant cholera holotoxin, wherein the holotoxin has reduced toxicity compared to a wild-type cholera holotoxin and has a substitution other than aspartic acid for the glutamic acid at position 29 of the A subunit of the cholera holotoxin, in combination with a selected antigen from a pathogenic bacterium, virus, fungus or parasite, is used to prepare an antigenic composition, wherein said holotoxin enhances the immune response in a vertebrate host to said antigen.

The antigenic compositions of this invention also comprise CT-CRM containing at least one additional mutation at a position other than at amino acid residue 29. International application WO 93/13202 (36) describes a series of mutations in the A subunit which serve to reduce the toxicity of the cholera holotoxin. These mutations include making substitutions for the arginine at amino acid 7, the aspartic acid at position 9, the arginine at position 11, the histidine at position 44, the valine at position 53, the arginine at position 54, the serine at position 61, the serine at position 63, the histidine at position 70, the valine at position 97, the tyrosine at position 104, the proline at position 106, the histidine at position 107, the glutamic acid at position 110, the glutamic acid at position 112, the serine at position 114, the tryptophan at position 127, the arginine at position 146 and the arginine at position 192. The nucleotide sequence encoding the A subunit of the cholera holotoxin is set forth in International application WO 93/13202. International application WO 98/42375 (37) describes making a substitution for the serine at amino acid 109 in the A subunit, which serves to reduce the toxicity of the cholera holotoxin. Therefore, using conventional techniques, mutations at one or more of these additional positions are generated.

The antigenic compositions of this invention are administered to a human or non-human vertebrate by a variety of routes, including, but not limited to, intranasal, oral, vaginal, rectal, parenteral, intradermal, transdermal (see, e.g., International application WO 98/20734 (38)), intramuscular, intraperitoneal, subcutaneous, intravenous and intraarterial. The amount of the antigen component or components of the antigenic composition will vary depending upon the identity of the antigen, as well as upon the age, weight and medical condition of the host, as well as on the method of administration. Again, suitable doses are readily determined by persons skilled in the art. It is preferable, although not required, that the antigen and the mutant CT be administered at the same time. The number of doses and the dosage regimen for the antigenic composition are also readily determined by persons skilled in the art. Protection may be conferred by a single dose of the antigenic composition, or may require the administration of several doses, in addition to booster doses at later times to maintain protection. In some instances, the adjuvant property of the mutant CT may reduce the number of doses needed or the time course of the dosage regimen.

The antigenic compositions of this invention may comprise further adjuvants in addition to CT-CRM_{E29H}. Examples of such adjuvants include, but are not limited to, Stimulon^{™} QS-21 (Aquila Biopharmaceuticals, Inc., Framingham, MA), MPL^{™} (3-O-deacylated monophosphoryl lipid A; RIBI ImmunoChem Research, Inc., Hamilton, MT), aluminum phosphate, aluminum hydroxide and IL-12 (Genetics Institute, Cambridge, MA). The antigenic compositions may also be mixed with immunologically acceptable diluents or carriers in a conventional manner.

The immunogenic mutant cholera holotoxin of this invention is suitable for use as an adjuvant in antigenic compositions containing a wide variety of antigens from a wide variety of pathogenic microorganisms, including but not limited to those from bacteria, viruses, fungi or parasitic microorganisms which infect humans and non-human vertebrates. The antigen may comprise a whole cell or virus, or one or more saccharides, proteins, protein subunits or fragments, poly- or oligonucleotides, or other macromolecular components. If desired, the antigenic compositions may contain more than one antigen from the same or different pathogenic microorganisms.

Desirable bacterial vaccines including the CT-CRM mutants as an adjuvant include those directed to the prevention and/or treatment of disease caused by, without limitation, *Haemophilus influenzae* (both typable and nontypable), *Haemophilus somnus*, *Moraxella catarrhalis, Streptococcus pneumoniae*, *Streptococcus pyogenes, Streptococcus agalactiae*, *Streptococcus faecalis, Helicobacter pylori, Neisseria meningitidis, Neisseria gonorrhoeae, Chlamydia trachomatis, Chlamydia pneumoniae, Chlamydia* psittaci, *Bordetella pertussis, Salmonella typhi, Salmonella typhimurium, Salmonella choleraesuis, Escherichia coli, Shigella, Vibrio cholerae*, *Corynebacterium diphtheriae, Mycobacterium tuberculosis, Mycobacterium avium- Mycobacterium intracellulare* complex, *Proteus mirabilis, Proteus vulgaris, Staphylococcus aureus*, *Clostridium tetani, Leptospira interrogans, Borrelia burgdorferi, Pasteurella haemolytica, Pasteurella multocida, Actinobacillus pleuropneumoniae* and *Mycoplasma gallisepticum.*

Desirable viral vaccines including the CT-CRM mutants as an adjuvant include those directed to the prevention and/or treatment of disease caused by, without limitation, Respiratory syncytial virus, Parainfluenza virus types 1-3, Influenza virus, Herpes simplex virus, Human cytomegalovirus, Human immunodeficiency virus, Hepatitis A virus, Hepatitis B virus, Hepatitis C virus, Human papillomavirus, poliovirus, rotavirus, caliciviruses, Measles virus, Mumps virus, Rubella virus, adenovirus, rabies virus, canine distemper virus, coronavirus, parvovirus, infectious rhinotracheitis viruses, feline leukemia virus, feline infectious peritonitis virus, avian infectious bursal disease virus, Newcastle disease virus, Marek's disease virus, porcine respiratory and reproductive syndrome virus, equine arteritis virus and various Encephalitis viruses.

Desirable vaccines against fungal pathogens including the CT-CRM mutants as an adjuvant include those directed to the prevention and/or treatment of disease caused by, without limitation, *Aspergillis, Blastomyces, Candida, Coccidiodes, Cryptococcus* and *Histoplasma.*

Desirable vaccines against parasites including the CT-CRM mutants as an adjuvant include those directed to the prevention and/or treatment of disease caused by, without limitation, *Leishmania major*, *Ascaris, Trichuris, Giardia, Schistosoma, Cryptosporidium, Trichomonas, Toxoplasma gondii* and *Pneumocystis carinii.*

The CT-CRM mutants are also suitable for inclusion as an adjuvant in polynucleotide vaccines (also known as DNA vaccines). Such vaccines may further include facilitating agents such as bupivicaine (see U.S. Patent Number 5,593,972 (39)).

CT-CRM_{E29H} was compared to wild-type CT as an adjuvant for the administration of plasmid DNA (pDNA) encoding the full length glycoprotein D of herpes simplex virus (HSV) type 2 (gD2), formulated with bupivicaine (40). The results indicated that BALB/c mice which received CT-CRM_{E29H} along with pDNA vaccine for HSV-2 by the intradermal route generated a higher average cellular response than those that received pDNA HSV gD2 vaccine by itself by the intradermal route (Table 34). In addition, the average antibody response in serum for mice which received the pDNA HSV gD2 vaccine along with CT-CRM_{E29H} was approximately at the same level as that seen for mice which received the pDNA HSV gD2 vaccine without adjuvant (Table 35).

Similarly, the pDNA HSV gD2 vaccine generated a gD2-specific antibody response in vaginal wash samples at levels that were comparable to those seen following the delivery of non-adjuvanted vaccine by intradermal or intramuscular routes (Table 36).

Mice immunized with the pDNA HSV gD2 vaccine adjuvanted with CT-CRM_{E29H} or CT and delivered by the intradermal route generated substantially higher levels of gamma interferon than mice which received the pDNA HSV-gD2 vaccine without adjuvant (Table 37). Mice which received the CT-CRM_{E29H} also generated IL-5.

Thus, CT-CRM_{E29H} enhanced proliferative and gamma interferon responses when administered with a plasmid DNA vaccine against HSV.

In order that this invention may be better understood, the following examples are set forth. The examples are for the purpose of illustration only and are not to be construed as limiting the scope of the invention.

### Examples

### Example 1

### Expression of CT Mutants

### Bacterial strains, plasmids and growth conditions

*E. coli* TG1 (Amersham-Pharmacia Biotech, Piscataway, NJ), and TX1, a nalidixic acid-resistant derivative of TG1, carrying FTc, lacI^{q} from XL1 blue (Stratagene, LaJolla, CA; (41)) and CJ236(FTc, lacI^{q}) (Bio-Rad, Hercules, CA) were used as hosts for cloning recombinant plasmids and expression of mutated proteins. Plasmid-containing strains were maintained on LB agar plates with antibiotics as required (ampicillin, 50 µg/ml; kanamycin 25 µg/ml; tetracycline 10 µg/ml). A complete CT operon from *V. cholerae* 0395 was subcloned into the phagemid vector pSKII⁻, under the control of the lac promoter, to create the IPTG inducible plasmid designated pMGJ67 (42).

### Mutagenesis of ctxA gene

The method of Kunkel (43) was used to select for oligonucleotide-derived mutants created in plasmid pMGJ67. The oligonucleotides used to generate the five mutant CT-CRMs are described in Table 1.

**Table 1**

| Sequence of Oligonucleotides Introduced into *ctx*A | | |
|---|---|---|
| Substitution | Oligonucleotide Sequence^{a} | |
| R7K | AAGTTATATAAGGCAGATTC | (SEQ ID NO:1) |
| R11K | CAGATTCTAAACCTCCTG | (SEQ ID NO:2) |
| E29H | GACAGAGTNAGTACTTTGACCG | (SEQ ID NO:3) |
| E110D | CAGATGAKCAAGAKGTTTCTGC | (SEQ ID NO:4) |
| E112D | CAGATGAKCAAGAKGTTTCTGC | (SEQ ID NO:5) |

| | | |
|---|---|---|
| ^{a} Altered bases are underlined; N=any base; K=T or G. | | |

Briefly, each single-stranded oligonucleotide was phosphorylated and used to direct second strand synthesis on a uracil-containing single-stranded DNA template rescued from the *E. coli* dut ung strain CJ236(F'Tc, pMGJ67). Following ligation and transformation of ung⁺ strain TX1, single-stranded DNA was rescued from Amp^{R} transformants and sequenced by the dideoxy chain termination method (44).

### Construction of the Plasmid Encoding CT-CRM_{E29H}

The plasmid encoding CT-CRM_{E29H} is designated pIIB29H. The plasmid contains the polycistron of *V. cholerae* genes *ctx*A and *ctx*B which encode CT. The *ctx*A gene in this plasmid was mutagenized as described above to encode a histidine at amino acid position 29 of CT-A. The wild-type polycistron was also altered by removing the native ToxR inducible promoter and replacing it with a lactose inducible promoter. Furthermore, the regions encoding the *ctx*A and *ctx*B signal sequences were replaced with the signal sequence-encoding region of *E. coli* LT (LTIIb-B leader) in order to promote secretion of CT-CRM_{E29H}. The plasmid pIIB29H was then modified in an attempt to increase the expression of CT-CRM_{E29H}. The resulting plasmid, designated pPX2492, contained synthetic Shine Dalgarno sequences upstream of each of *ctx*A and *ctx*B. The two genes are genetically separated in pPX2492, unlike in *V. cholerae*, where the genes overlap. The two genes also have the LTIIb-B leader sequence upstream of each.

### Expression of mutant ctxA alleles

Production of each variant holotoxin was tested in 5 ml cultures of TB medium (45) in 125 ml Erlenmeyer flasks at 37°C with shaking (200 rpm). Logarithmic phase cells (A₆₀₀ = 0.8-1.0) were induced by the addition of IPTG to 0.4 mM, followed by growth overnight. Polymyxin B was added to 1 mg/ml, followed by incubation for 10 minutes at 37°C. Cells were removed by centrifugation, and the supernatants were assayed to determine the concentrations of holotoxin and B pentamer as described below.

Specifically, the production of CT-CRM_{E29H} in *E. coli* involves the co-expression of the genes rpoH from *E. coli* and *dsb*A from *V. cholerae.* These gene products participate in the conformational maturation of both the A and B subunits of CT.

### Example 2

### The GM1 Binding Assay for Intact Holotoxin

The CT-CRMs were examined in a ganglioside GM₁-dependent solid phase radioimmunoassay (42) to determine whether intact holotoxin was present after purification. An enzyme-linked immunosorbent assay (ELISA) was used where ELISA plate microwells were coated overnight at 4°C with ganglioside GM₁ (10 µg/ml). Thereafter, the following reagents were added in sequence with an interval of one hour incubation at room temperature: CT-CRMs (titrated from 3 µg/ml to 0.00137 µg/ml), 100 µl of rabbit anti-CT-A sera (1:1,000), and alkaline phosphatase conjugated goat anti-rabbit antibody (1:2,000). To visualize the reaction, 100 µl of p-nitrophenyl phosphate at 1 µg/ml in diethanolamine was added and incubated for 30 minutes. The reaction was stopped by adding 100 µl of 2 N NaOH and immediately read by a Microelisa autoreader. When compared to wild-type CT, the data indicated that the CT-CRMs with amino acid substitutions at positions 7, 29, 110, or 112 were intact holotoxins (Figure 1). The results implied, however, that a portion of purified CT-CRM_{R11K} did not appear to be a holotoxin.

### Example 3

### Y-1 Adrenal Cell Assay for Residual Toxicity of CT-CRMs

The mutant CT-CRMs were compared several times with wild-type holotoxin for toxicity in the mouse Y-1 adrenal tumor cell assay. Y-1 adrenal cells (ATCC CCL-79) were seeded in 96-well flat-bottom plates at a concentration of 10⁴ cells per well. Thereafter, three-fold serial dilutions of CT-CRMs were added to the tumor cells and incubated at 37°C (5% CO₂) for 18 hours. The cells were then examined by light microscopy for evidence of toxicity (cell rounding). The endpoint titer was defined as the minimum concentration of toxin required to give greater than 50% cell rounding. The percent of residual toxicity is calculated using the endpoint titer of wild-type CT divided by the titer elicited by CT-CRM multiplied by 100. Table 2 depicts the residual toxicity of several purified mutant holotoxins tested in the Y-1 adrenal cell assay.

**Table 2**

| The toxicity for Y-1 adrenal cells | |
|---|---|
| Y-1 Adrenal CT-CRM | Cell Assay % Residual Toxicity |
| E112D | 0.13 |
| E112D | 0.13 |
| R11K | 0.04 |
| R7K | 0.04 |
| E110D | 0.13 |
| E110D | 0.40 |
| E29H | 1.20 |
| CT-B | 1.20 |
| CT | 100.00 |

### Example 4

### Patent Mouse Gut Weight Assay

In this assay, 10 µg of wild-type CT or CT-CRM_{E29H} was administered intragastrically to each group of BALB/c mice (three mice per group). Intestines were removed carefully three hours later and weighed. The results are presented in Table 3. Data are presented as the mean gut/carcass weight ratio per group.

**Table 3**

| Toxicity of CT-CRM_{E29H} | | | |
|---|---|---|---|
| Assay | CT | CT- CRM_{E29H} | PBS |
| Mouse Gut Weight (gut/carcass ratio) | 0.13 ± 0.01 | 0.09 ± 0.01^{a} | 0.08 ± 0.007 |

| | | | |
|---|---|---|---|
| ^{a} p < 0.05 compared to wild-type CT control, p > 0.05 compared to PBS. | | | |

### Example 5

### The ADP-ribosyltransferase Assay

NAD*:agmatine ADP-ribosyltransferase activity was measured as the release of [carbonyl-¹⁴C] nicotinamide from radiolabeled NAD⁺. Briefly, CT and CT-CRMs were trypsin activated and incubated for 30 minutes at 30°C with 50 mM glycine/20 mM dithiothreitol in TEAN buffer (Tris^{™}/EDTA/sodium azide/sodium chloride) (pH 8.0). Thereafter, the following materials were added to the reaction: 0.1 mg of soybean trypsin inhibitor, 50 mM potassium phosphate, 10 mM agmatine, 20 mM dithiothreitol, 10 mM magnesium chloride, 100 µM GTP, 3 mM dimyristoylphosphatidylcholine, 0.2% cholate, 0.03 mg of ovalbumin, 100 µM [adenine-U-¹⁴C] NAD (DuPont NEN^{™}, Boston, MA) and water to a final volume of 300 µl. After incubation for 90 minutes at 30° C, 100 µl samples were applied to columns (0.64 x 5 cm) of AG1-X2 (Bio-Rad) which were washed five times with 1.0 ml of distilled/deionized H₂O. Eluates containing [¹⁴C]ADP-ribosylagmatine were collected for radioassay. Mean recovery of ¹⁴C in the eluate is expressed as percentage of that applied to column. The results are presented in Table 4.

**Table 4**

| NAD:Agmatine ADP-Ribosyltransferase Activity | | |
|---|---|---|
| Adjuvant | ADP-ribosylagmatine formed (nmol/hr/µg protein) | % ADP-ribosylation activity |
| CT, 10 µg | 57.1 | 100 |
| | | |
| E29H, 10 µg | 6.7 | 11.7 |
| E110D, 10 µg | 0.4 | 0.7 |
| E112D, 10 µg | 0.9 | 1.6 |
| R7K, 10 µg | 0.4 | 0.7 |
| R11K, 10 µg | 0.4 | 0.7 |

### Example 6

### The Immune Responses of BALB/c Mice Immunized with Recombinant (r) P4 and P6 Outer Membrane Proteins of Nontypable Haemophilus influenzae (NTHi)

In a first experiment, five BALB/c mice per group were immunized intranasally on days 0, 21 and 35 with a 10 µl dose containing 5 µg rP4 or 10 µg rP6, plus 1 µg of the adjuvant as indicated in Tables 5 and 6 (one group did not receive adjuvant). The anti-rP4 IgG antibody titers were determined by ELISA on pooled samples collected at days 0, 21, 35 and 48 and the results shown in Table 5. The anti-rP6 IgG antibody titers were separately determined by ELISA on pooled samples collected at days 0, 21, 35 and 48 and the results shown in Table 6. The mucosal antibody responses to rP4 were also measured two weeks after the last immunization (day 49). Table 7 sets forth the IgA and IgG titers from nasal, bronchoalveolar and vaginal washes, respectively.

In a second experiment, five BALB/c mice per group were immunized intranasally on days 0, 21 and 35 with a 30 µl dose containing 5 µg rP4 or 10 µg rP6, plus ascending doses of CT-CRM_{E29H} as indicated in Table 8 (other groups each received CT or CT-B; one group received no adjuvant). The serum anti-rP4 IgA and IgG antibody titers were determined by ELISA on pooled samples collected at days 21, 35 and 48 and the results shown in Table 8. The IgA and IgG titers from bronchoalveolar and vaginal washes on day 49 were also determined and are shown in Table 8.

**Table 5**

| The systemic humoral immune responses of BALB/c mice immunized^{a} with recombinant P4 and P6 proteins^{b} formulated with mutant cholera holotoxins | | | | |
|---|---|---|---|---|
| | Serum Anti-Recombinant P4 IgG Antibody Titers^{c} | | | |
| Adjuvant ^{d} | Day 0 | Day 21 | Day 35 | Day 48 |
| None | 1,157 | 1,277 | 1,893 | 1,968 |
| CT | 751 | 1,657 | 17,589 | 45,885 |
| CT-B | 1,111 | 1,118 | 6,917 | 70,578 |
| E29H | 1,052 | 1,539 | 11,917 | 95,922 |
| E110D | 1,243 | 1,313 | 6,886 | 83,058 |
| E112D | 1,400 | 1,520 | 9,280 | 41,485 |
| R7K | 2,546 | 1,771 | 3,311 | 40,936 |
| R11K | 1,289 | 1,391 | 3,428 | 23,631 |

| | | | | |
|---|---|---|---|---|
| ^{a} The mice were immunized intranasally (IN, 10 µl volume) on days 0, 21 and 35. ^{b} Recombinant P4 and P6 proteins were administered at 5 and 10 µg per dose respectively. ^{c} Anti-recombinant P4 IgG antibody titers were determined by ELISA on pooled samples collected at the denoted times. There were 5 mice per group. ^{d} CT and CT mutants were administered at 1 *µ*g per dose. | | | | |

**Table 6**

| The systemic humoral immune responses of BALB/c mice immunized^{a} with recombinant P4 and P6 proteins^{b} formulated with mutant cholera holotoxins | | | | |
|---|---|---|---|---|
| | Serum Anti-Native P6 IgG Antibody Titers^{c} | | | |
| Adjuvant^{d} | Day 0 | Day 21 | Day 35 | Day 48 |
| None | < 100 | < 100 | < 100 | < 100 |
| CT | < 100 | < 100 | 9,644 | 54,821 |
| CT-B | < 100 | < 100 | 875 | 7,399 |
| E29H | < 100 | < 100 | 3,472 | 19,638 |
| E110D | < 100 | < 100 | 3,666 | 22,415 |
| E112D | < 100 | < 100 | 426 | 9,538 |
| R7K | < 100 | < 100 | 529 | 3,904 |
| R11K | < 100 | < 100 | 248 | 3,763 |

| | | | | |
|---|---|---|---|---|
| ^{a} The mice were immunized intranasally (IN, 10 *µ*l volume) on days 0, 21 and 35. ^{b} Recombinant P4 and P6 proteins were administered at 5 and 10 *µ*g per dose respectively. ^{c} Anti-recombinant P6 IgG antibody titers were determined by ELISA on pooled samples collected at the denoted times. There were 5 mice per group. ^{d} CT and CT mutants were administered at 1 *µ*g per dose. | | | | |

**Table 7**

| The mucosal antibody responses of BALB/c mice immunized^{a} with recombinant P4 and P6 proteins^{b} formulated with mutant cholera holotoxins | | | | | | |
|---|---|---|---|---|---|---|
| | Anti-Recombinant P4 Antibody Titers^{c} | | | | | |
| | NW^{d} | | BAW^{d} | | VW^{d} | |
| Adjuvant^{e} | IgA | IgG | IgA | IgG | IgA | IgG |
| None | < 5 | < 5 | < 5 | < 5 | < 50 | < 50 |
| CT | < 5 | < 5 | < 5 | 56 | 54 | < 50 |
| CT-B | < 5 | < 5 | < 5 | 99 | < 50 | < 50 |
| E29H | < 5 | < 5 | < 5 | 176 | 63 | < 50 |
| E110D | < 5 | < 5 | < 5 | 144 | < 50 | 98 |
| E112D | 11 | < 5 | < 5 | 48 | 564 | 58 |
| R7K | < 5 | < 5 | < 5 | 56 | < 50 | < 50 |
| R11K | 6 | < 5 | < 5 | 34 | 223 | < 50 |

| | | | | | | |
|---|---|---|---|---|---|---|
| ^{a} The mice were immunized intranasally (IN, 10 µl volume) on days 0, 21 and 35. ^{b} Recombinant P4 and P6 proteins were administered at 5 and 10 *µ*g per dose respectively. ^{c} Anti-recombinant P4 IgG and IgA antibody titers were determined by ELISA on pooled samples collected 2 weeks after the last immunization (day 49). There were 5 mice per group. ^{d} NW, BAW, and VW denote nasal wash, bronchoalveolar wash, and vaginal wash respectively. ^{e} CT and CT mutants were administered at 1 *µ*g per dose. | | | | | | |

**Table 8**

| The effect of ascending doses of CT-CRM_{E29H} on the generation of local and systemic immune responses against the recombinant P4 and P6 proteins of *Haemophilus influenzae* | | | | | | | | | | |
|---|---|---|---|---|---|---|---|---|---|---|
| | Anti-Recombinant P4 Antibody Titers^{a} | | | | | | | | | |
| | Sera | | | | | | Mucosal Wash Fluids^{b} | | | |
| | Day 21 | | Day 35 | | Day 48 | | BAW | | VW | |
| Adjuvant | IgA | IgG | IgA | IgG | IgA | IgG | IgA | IgG | IgA | IgG |
| none | 189 | 219 | 204 | 3,833 | 334 | 17,269 | 27 | 26 | 493 | 146 |
| 1 *µ*g CT | 145 | 741 | 1,803 | 67,470 | 5,855 | 495,735 | 864 | 2,279 | 1,446 | 1,934 |
| 10 *µ*g CT-B | 206 | 2,531 | 650 | 34,047 | 5,838 | 989,806 | 622 | 3,674 | 1,397 | 884 |
| 1 *µ*g CT-CRM_{E29H} | 171 | 2,640 | 1,021 | 59,031 | 8,643 | 588,586 | 845 | 3,180 | 1,898 | 1,479 |
| 10 *µ*g CT-CRM_{E29H} | <100 | 498 | <100 | 6,427 | 397 | 84,176 | 25 | 175 | 122 | 31 |
| 30 *µ*g CT-CRM_{E29H} | 119 | 622 | 801 | 14,151 | 2,194 | 161,187 | 67 | 568 | 215 | 116 |

| | | | | | | | | | | |
|---|---|---|---|---|---|---|---|---|---|---|
| ^{a} The mice were immunized intranasally (IN, 30 *µ*l volume) on days 0, 21 and 35 with recombinant P4 (5 *µ*g) and P6 (10 *µ*g) proteins. Anti-recombinant P4 IgG and IgA antibody titers were determined by ELISA on pooled samples collected at the indicated times. There were 5 mice per group. ^{b} BAW and VW denote bronchoalveolar wash, and vaginal wash respectively. | | | | | | | | | | |

### Example 7

### The Immune Responses of BALB/c Mice Immunized with the Native Hapₛ Protein of NTHi

NTHi strain P860295 (46) was obtained from Dr. Charles Brinton, University of Pittsburgh. It was obtained from the nasopharynx of a child with NTHi induced otitis media. NTHi strain TN106 (47) was obtained from Dr. Eric Hansen, University of Texas Southwestern Medical Center at Dallas. A streptomycin resistant mutant of TN106 was derived by selection on BHI-XV plates containing 100 µg/ml of streptomycin (Sigma, St. Louis, MO). This mutant was passaged twice in the nasopharynx of Balb/c mice and frozen as strain TN106.P2.

The Hapₛ protein from NTHi strain P860295 was purified as follows. NTHi strain P860295 was grown in BHI-XV media for 18 hours at 35°C with aeration. The bacterial cells were pelleted by centrifugation, 10K x g at 4°C, and discarded. The supernatant was brought to 60% saturation with solid (NH₄)₂SO₄, held at room temperature for 2-3 hours, and the precipitate was collected by centrifugation. The precipitate was dissolved in 50 mM sodium phosphate buffer, pH 5.8, 1 mM EDTA, 50 mM NaCl (Buffer 1), and was dialyzed at 4°C against the above buffer. A 10 ml bed volume CM Sepharose^{™} column (Pharmacia, Piscataway, NJ) was equilibrated with Buffer 1, and 30 ml of the above soluble material was loaded onto the column at a flow rate of 1 ml/min. The column was washed with Buffer 1 until the OD₂₈₀ reached baseline. The fall-through material was discarded. Bound proteins were eluted from the resin using a three step gradient: (1) sodium phosphate buffer, pH 7.0, 1mM EDTA, 50 mM NaCl; (2) sodium phosphate buffer, pH 8.0, 1 mM EDTA, 50 mM NaCl; and (3) sodium phosphate buffer, pH 8.0, 0.5 M NaCl, 1 mM EDTA. Proteins eluted in each step were pooled and saved for analysis. SDS-PAGE (48) analysis of pools indicated that the Hapₛ protein eluted in gradient steps 2 and 3. These pools contained highly purified Hapₛ and were combined.

Six week old, female Balb/c mice (ten per group) were then immunized IN with Hapₛ purified from NTHi strain P860295. The Hapₛ protein was diluted in D-PBS to 5 or 15 µg/40 µl with or without CT-CRM_{E29H}. Where used, the CT-CRM_{E29H} was used at a dosage of 0.1 µg/mouse. Control formulations containing CT-CRM_{E29H} in D-PBS, D-PBS alone and formalin fixed TN106.P2 (the NTHi challenge strain) were also administered to the mice in 40 µl volumes.

Prior to IN immunization, mice were anesthetized and then immunized by intranasal inoculation of 20 µl/nostril from a pipette. The pipette was held so the tip touched the opening of the nostril and the formulation was automatically drawn into the nostril during breathing. The mice were placed in a supine position so noses were not touching anything after administration of the formulation or the challenge. The mice were immunized at weeks 0, 1, 3, and 5. Sera were drawn at week 7. The results are shown in Table 9.

**Table 9**

| Systemic humoral immune response in Balb/c mice after intranasal immunization with Hapₛ admixed with or without CT-CRM_{E29H} | | | |
|---|---|---|---|
| Immunogen | Dose (*µ*g) | Adjuvant | Anti-Hapₛ IgG ELISA |
| Hap | 5 | - | 1,604 |
| Hap | 15 | - | 5,204 |
| Hap | 5 | CT-E29H | 4,653 |
| Hap | 15 | CT-E29H | 15,111 |
| - | - | CT-E29H | <500 |
| 1xPBS | - | - | <500 |
| Formalin Fixed TN106.P2 | | - | <500 |

### Example 8

### The Immune Responses of BALB/c and C57B1/6 Mice Immunized with the Recombinant (r) Urease Protein of Helicobacter pylori

In a first experiment, five BALB/c mice per group were immunized as follows: Seven groups were immunized intragastrically on days 0, 2, 14 and 16 with 100 µg rUrease plus 10 µg of the adjuvant as indicated in Tables 10-12. One group was immunized with 10 µg rUrease subcutaneously in the rump; another group was immunized with 10 µg rUrease subcutaneously in the neck; both groups also received 20 µg of Stimulon^{™} QS-21 as an adjuvant on days 0 and 16. The anti-rUrease antibody titers were determined by ELISA on pooled samples collected on day 28. The IgG results are shown in Table 10 and the IgA results are shown in Table 11. The mucosal antibody responses to rUrease were also measured on day 29. Table 12 sets forth the IgA and IgG titers from bronchoalveolar and vaginal washes, respectively.

In a second experiment, the ability of rUrease plus adjuvant to protect mice against a challenge with *H. felis* was assessed. Ten BALB/c mice per group were immunized as follows: Two groups were immunized intragastrically on days 0, 2, 14 and 16 with 100 µg rUrease plus 10 µg of the adjuvant as indicated in Table 13; a control group received PBS instead of rUrease plus 10 µg of adjuvant. One group was immunized subcutaneously on days 0 and 16 with 10 µg rUrease plus 20 µg of Stimulon^{™} QS-21. The anti-rUrease antibody titers were determined by ELISA on pooled samples collected on day 28. The mice were also challenged with three doses of 10⁸ *H. felis* on days 29, 31 and 34 and were assayed for protection on day 44. Protection was assessed by the rapid urease test. In the rapid urease test, one-half stomach was incubated at 37°C for five hours in 0.5 ml of the urease test medium containing 2% of urea and phenol red, a pH indicator, at 7 µg/ml. Urease activity generates ammonium and bicarbonate from urea, thus raising the pH and inducing a colorimetric change of the solution with a higher absorbance at 550 nm. The level of urease activity was measured by spectrophotometic analysis. The test was considered positive for *H. felis* when the mean of the absorbance values were two standard deviations above that of those obtained for the gastric tissues of non-infected mice. The results are shown in Table 13.

In a third experiment, two groups of C57BL/6 mice (five per group) were immunized intragastrically on days 0, 2, 14 and 16 with 100 µg rUrease plus 10 µg of the adjuvant as indicated in Table 14. A third group was immunized subcutaneously on days 0 and 16 with 10 µg rUrease plus 100 µg alum. A fourth group was immunized intragastrically on days 0, 2, 14 and 16 with 100 µg rUrease, but without adjuvant. The anti-rUrease antibody titers were determined by ELISA on pooled samples collected on day 28. Table 14 sets forth the IgA and IgG titers from sera, bronchoalveolar wash, fecal pellet extract and vaginal wash, respectively.

In a fourth experiment, five C57BL/6 mice per group were immunized as follows: Three groups of mice were immunized IG on days 0, 2, 14 and 16 with 100 µg rUrease plus 10 µg of the adjuvant as indicated in Table 15; a fourth group received no adjuvant. The anti-rUrease antibody titers were determined by ELISA on pooled samples collected on day 29. The IgA and IgG results are shown in Table 15. Table 15 also presents the IgE (PCA) and total IgE titers. PCA denotes that the IgE antibody titers were determined by the passive cutaneous anaphylaxis reaction. The PCA was performed on female Sprague-Dawley rats. The rats were sedated with ketamine/xylazine, shaved, and injected intradermally with 0.1 ml sera (serially diluted four-fold) from C57B1/6 mice immunized with rUrease formulated with either CT, LT, or CT-CRM_{E29H}. The rats were sedated 48 to 60 hours later and then injected (0.1 ml) intravenously via the tail vein with 2 µg rUrease in PBS containing 1% Evan's blue dye.

**Table 10**

| The effect of CT-CRMs on the generation of systemic anti-Urease IgG antibody titers in BALB/c mice | | | |
|---|---|---|---|
| Anti-Recombinant Urease IgG Antibody Titers^{a} | | | |
| Adjuvant ^{b} | Route ^{b} | Mean | SE |
| CT | IG | 234,010 | 43,316 |
| E29H | IG | 131,032 | 64,183 |
| R7K | IG | 17,692 | 9,271 |
| R11K | IG | 25,502 | 11,413 |
| E110D | IG | 22,299 | 8,571 |
| E112D | IG | 8,784 | 5,208 |
| CT-B | IG | 47,060 | 38,991 |
| QS-21 | SC-R | 4,038,430 | 1,702,556 |
| QS-21 | SC-N | 5,609,764 | 353,824 |

| | | | |
|---|---|---|---|
| ^{a} The geometric mean anti-recombinant Urease antibody titers were determined by ELISA on serum samples collected on day 28. There were 5 mice per group. ^{b} The mice were immunized with 10 µg rUrease subcutaneously (SC) in the rump (R), or neck (N) on days 0 and 16. Mice immunized intragastrically (IG) received 100 µg rUrease on days 0, 2, 14 and 16. The adjuvants were either Stimulon^{™} QS-21 (20 µg), CT (10 µg), or CT mutants (10 µg). | | | |

**Table 11**

| The effect of CT-CRMs on the generation of systemic anti-Urease IgA antibody titers in BALB/c mice | | | |
|---|---|---|---|
| Anti-Recombinant Urease IgA Antibody Titers^{a} | | | |
| Adjuvant^{b} | Route^{b} | Mean | SE |
| CT | IG | 2,529 | 584 |
| E29H | IG | 1,013 | 426 |
| R7K | IG | 82 | 15 |
| R11K | IG | 153 | 39 |
| E110D | IG | 351 | 137 |
| E112D | IG | 232 | 93 |
| CT-B | IG | 455 | 280 |
| QS-21 | SC-R | 5,675 | 562 |
| QS-21 | SC-N | 4,793 | 528 |

| | | | |
|---|---|---|---|
| ^{a} The geometric mean anti-recombinant Urease IgA antibody titers were determined by ELISA on serum samples collected on day 28. There were 5 mice per group. ^{b} The mice were immunized with 10 *µ*g rUrease subcutaneously (SC) in the rump (R), or in the neck (N) on days 0 and 16. Mice immunized intragastrically (IG) received 100 µg rUrease on days 0, 2, 14, and 16. The adjuvants were either Stimulon^{™} QS-21 (20 µg), CT (10 µg), or CT derivatives (10 µg). | | | |

**Table 12**

| The effect of CT-CRMs on the generation of anti-Urease IgA antibody titers in the mucosal secretions of BALB/c mice | | | | | |
|---|---|---|---|---|---|
| | Anti-Recombinant Urease Antibody Titers^{a} | | | | |
| | | BAW^{b} | | VW^{b} | |
| Adjuvant^{c} | Route^{c} | IgA | IgG | IgA | IgG |
| CT | IG | 387 | 1005 | 3,471 | 464 |
| E29H | IG | 63 | 317 | 2,095 | 265 |
| R7K | IG | < 5 | 27 | 79 | 42 |
| R11K | IG | 7 | 62 | 29 | 21 |
| E110D | IG | 13 | 98 | 217 | 84 |
| E112D | IG | < 5 | 17 | 991 | 108 |
| CT-B | IG | 65 | 312 | 140 | 60 |
| QS-21 | SC-R | 6 | 9816 | 809 | 10,272 |
| QS-21 | SC-N | 11 | 10,545 | 235 | 6,237 |

| | | | | | |
|---|---|---|---|---|---|
| ^{a} The anti-rUrease IgG and IgA antibody titers were determined by ELISA on pooled samples collected on day 29. There were 5 mice per group. ^{b} BAW and VW denote bronchoalveolar and vaginal wash respectively. ^{c} The mice were immunized with 10 µg rUrease subcutaneously (SC) in the rump (R), or in the neck (N) on days 0 and 16. Mice immunized intragastrically (IG) received 100 µg rUrease on days 0, 2, 14, and 16. The adjuvants were either Stimulon^{™} QS-21 (20 µg), CT (10 µg), or CT derivatives (10 µg). | | | | | |

**Table 13**

| The generation of protective immune responses in BALB/c mice immunized with recombinant urease formulated with CT or CT-CRM_{E29H} | | | | | |
|---|---|---|---|---|---|
| | | | IgA TITERS^{a} | | |
| Antigen^{b} | Adjuvant^{c} | Route | Sera | BAW^{d} | No. Protected/ Total (%)^{e} |
| PBS | CT | IG | <100 | ND | 2/10 (20) |
| r Urease | CT | IG | 2,730 | 11 | 8/10 (80) |
| r Urease | E29H | IG | 1,225 | 124 | 8/10 (80) |
| r Urease | QS-21 | SC | 14,917 | 7 | 3/10 (30) |
| NONE | NONE | ND | <100 | ND | 1/10 (10) |

| | | | | | |
|---|---|---|---|---|---|
| ^{a} The anti-recombinant (r) urease IgA antibody titers were determined by ELISA on pooled samples collected on day 28. There were 10 mice per group. ^{b} Mice were immunized intragastrically (IG) on days 0, 2, 14 and 16 with 100 µg r urease per dose. Control mice were injected subcutaneously (SC) on days 0 and 16 with 10 µg rUrease per dose. ^{c} The rUrease was formulated with either 10 µg CT or CT-CRM per dose, or mixed with 20 µg Stimulon^{™} QS-21 per dose. ^{d} BAW denotes bronchoalveolar wash. ^{e} The mice were challenged with 3 doses of 10⁸ *H. felis* on days 29, 31 and 34 and assayed for protection on day 44. Protection was assessed by the rapid urease test. | | | | | |

**Table 14**

| The effects of CT-CRM_{E29H} on the immune response to recombinant Urease in C57BL/6 mice | | | | | | | | | |
|---|---|---|---|---|---|---|---|---|---|
| | | Anti-Recombinant Urease Antibody Titers^{a} | | | | | | | |
| | | Sera | | BAW^{b} | | FP^{b} | | VW^{b} | |
| Adjuvant^{c} | Route^{c} | IgA | IgG | IgA | IgG | IgA | IgG | IgA | IgG |
| E29H | IG | 1,358 | 252,289 | 14 | 426 | 265 | 9 | 40 | 140 |
| CT | IG | 1,117 | 92,182 | 12 | 331 | 97 | 7 | 45 | 309 |
| Alum | SC | 2,658 | 1,012,790 | 3 | 795 | 7 | < 5 | 49 | 173 |
| None | IG | 110 | 10,938 | < 5 | 14 | < 5 | < 5 | < 50 | 29 |

| | | | | | | | | | |
|---|---|---|---|---|---|---|---|---|---|
| ^{a} The endpoint anti-rUrease antibody titers were determined by ELISA on pooled serum samples collected on day 28. There were 5 mice per group. ^{b} BAW, FP, and VW denote bronchoalveolar wash, fecal pellet extract, and vaginal wash respectively. ^{c} The mice were immunized with 10 µg rUrease subcutaneously (SC) on days 0 and 16. The mice immunized intragastrically (IG) received 100 µg rUrease on days 0, 2, 14, and 16. The adjuvants were either alum (100 µg), CT (10 µg), or CT-CRM_{E29H} (10 µg). | | | | | | | | | |

**Table 15**

| The generation of urease-specific IgE antibodies in the circulation of C57Bl/6 mice immunized with recombinant urease prepared with either CT, LT, or CT-CRM_{E29H} | | | | |
|---|---|---|---|---|
| | Anti-rUrease Antibody Titers^{a} | | | |
| Adjuvant^{b} | IgA | IgG | IgE (PCA)^{c} | Total IgE^{d} |
| NONE | 732 | 30,591 | <4 | ND |
| CT | 2,504 | 496,373 | 32 | 1591 |
| CT-CRM_{E29H} | 4,039 | 477,098 | 16 | 888 |
| LT | 5,251 | 670,807 | 64 | 3589 |

| | | | | |
|---|---|---|---|---|
| ^{a} The endpoint IgA and IgG antibody titers were determined by ELISA on pooled serum samples collected on day 29. There were 5 mice per group. ^{b} The mice were immunized intragastrically (IG) with 100 µg rUrease on days 0, 2, 14, and 16. The adjuvants were 10 µg CT, CT-CRM_{E29H} or LT. ^{c} PCA denotes that the IgE antibody titers were determined by the passive cutaneous anaphylaxis reaction. The PCA was performed on female Sprague Dawley rats. The rats were sedated with ketamine/xylazine, shaved, and injected intradermally with 0.1 ml sera (serially diluted 4-fold) from C57B1/6 mice immunized with rUrease formulated with either CT, LT, or CT-CRM_{E29H}. The rats were sedated 48 to 60 hours later and then injected (0.1 ml) intravenously via the tail vein with 2 µg rUrease in PBS containing 1% Evan's blue dye. ^{d} The numbers are in ng/ml. ND denotes not detected. | | | | |

### Example 9

### The Immune Responses of Swiss-Webster Mice Immunized with Recombinant Class 1 Pilin and Class 1 Outer Membrane Protein of Neisseria meningitidis

In a first experiment, 6-8 week-old Swiss-Webster mice (15 per group) were immunized IN (10 µl) at weeks 0, 2 and 3 with 5 µg of purified recombinant class 1 pilin (rpilin) formulated with CT-CRM_{E29H} (0.1 or 1 µg/mouse). Serum samples, bronchoalveolar washes (BAW), nasal washes (NW) and vaginal washes (VW) were collected from five mice in each group for determination of serum and mucosal IgA and IgG antibodies specific to *N. meningitidis* pilin by ELISA at week 4. The results are presented in Table 16. The remaining ten mice in each group immunized in parallel were challenged IN with 2 x 10⁷ CFUs of the homologous *N. meningitidis* strain H355P⁺.p2IR (passed through infant rats twice) at week 4. Recovery of Group B *N. meningitidis* from nasal tissue 24 hours post-challenge was determined by quantitative culture, as shown in Figure 2.

In a second experiment, the protection of rpilin formulated with CT-CRM_{E29H} against a homologous meningococcal strain was compared to that of CT-CRM_{E29H} admixed with an unrelated protein, KLH. Groups of five Swiss-Webster mice (six-week old) were immunized IN (10 µl volume) with 5 µg of rpilin with or without CT-CRM_{E29H} (0.1 µg), or with PorA H355 with CT-CRM_{E29H} (0.1 µg) at weeks 0, 2, and 3. Control groups were either non-immunized (naive group) or IN immunized with KLH (5 µg) plus CT-CRM_{E29H} (0.1 µg). The endpoint antibody titers were determined by whole cell and antigen-specific ELISA on pooled serum samples collected at week 4 before challenge with 1 x 10⁷ CFU's of meningococcal strain H355P⁺. The results are presented in Tables 17 and 18.

In a third experiment, the immunogenicity of meningococcal PorA formulated with CT-CRM_{E29H} for IN immunization was assessed. Five Swiss-Webster mice per group were immunized IN on weeks 0, 2 and 3 with 20 µg/dose PorA from meningococcal strain H44/76 with or without CT-CRM_{E29H} (1 µg/dose) as indicated in Table 19. The anti-PorA H44/76 antibody titers and whole cell ELISA for IgG were assayed on pooled serum and mucosal samples collected at week 4 of the experiment. The results are presented in Table 19.

In a fourth experiment, the ability of rpilin and PorA adjuvanted with CT-CRM_{E29H} to protect mice against the challenge of a heterologous strain of meningococci was assessed. Ten Swiss-Webster mice per group were immunized IN (10 µl volume) with 5 µg of either rpilin, PorA from strain H355, or PorA from strain 870227, formulated with CT-CRM_{E29H} (0.1 µg) at weeks 0, 2, and 3. Control groups were either heat-inactivated meningococcal strain 870227 whole cells or KLH (5 µg) plus CT-CRM_{E29H} (0.1 µg). The endpoint IgG and IgA titers were determined by whole cell and antigen-specific ELISA on pooled serum samples collected at week 4 before challenge with the 870227 strain. The results are presented in Tables 20 and 21. The bacterial recovery from nasal tissue was determined by quantitative culture 24 hours after challenge with strain 870227 and was expressed as Log₁₀ CFU ± standard deviation. The results are shown in Figure 4.

A fifth experiment was conducted to examine the potential of CT-CRM_{E29H} as an adjuvant for parenteral immunization. Groups of 10 female Swiss-Webster mice, 5-6 weeks old, were immunized subcutaneously with 5 µg of PorA H355 formulated with either CT-CRM_{E29H} (10 µg) or MPL™ (100 µg) at weeks 0 and 4. Control groups were immunized subcutaneously with heat-inactivated meningococcal 870227 whole cells or KLH (5 µg) plus MPL^{™} (100 µg). Mice were challenged IN with 1.2 x 10⁷ CFUs of meningococcal strain 870227 at week 6. Twenty-four hours post-challenge, mice were sacrificed and nasal tissues were homogenized and plated on selective medium. Colonies were counted after incubation at 37°C overnight and expressed as Log₁₀ CFU ± standard deviation. The results of this experiment are presented in Table 22 and Figure 5.

**Table 16**

| Adjuvant effects of CT-CRM_{E29H} on the systemic and mucosal immune responses to *N. meningitidis* rPilin (Class I H44/76) in Swiss-Webster mice | | | | | | | | | | |
|---|---|---|---|---|---|---|---|---|---|---|
| | Anti-rPilin antibody ELISA titers on pooled samples | | | | | | | | | |
| | Sera (wk 0) | | Sera (wk 4) | | BW | | NW | | VW | |
| Immunogen | IgA | IgG | IgA | IgG | IgA | IgG | IgA | IgG | IgA | IgG |
| rPilin | <50 | <50 | 878 | 69,013 | 4 | 41 | 23 | 2 | 37 | 17 |
| rPilin + CT-CRM_{E29H} (0.1 µg) | <50 | <50 | 2,209 | 209,228 | <2 | 19 | 34 | 40 | 61 | 51 |
| rPilin + CT-CRM_{E29H} (1 µg) | <50 | <50 | 4,089 | 1,344,776 | 41 | 540 | 75 | 45 | 135 | 216 |

**Table 17**

| The effect of CT-CRM_{E29H} on the immune response to meningococcal antigens in Swiss-Webster mice | | | | | | |
|---|---|---|---|---|---|---|
| | Total Sera IgG of Meningococcal B Whole Cell ELISA | | | | | |
| Group | H355P⁺ | strain | FAM 18 | strain | M982 | strain |
| | Wk 0 | Wk 4 | Wk 0 | Wk 4 | Wk 0 | Wk 4 |
| 5 µg rPilin | 175* | 2,371 | 294* | 639 | 137* | 2,375 |
| 5 µg rPilin + 0.1 µg CT-CRM_{E29H} | 175* | 24,965 | 294* | 2,702 | 137* | 21,862 |
| 5 µg PorA+ 0.1 µg CT-CRM_{E29H} | 175* | 10,156 | 294* | 9,136 | 137* | 5,733 |
| 5 µg KLH + 0.1 µg CT-CRM_{E29H} | 175* | 192 | 294* | 230 | 137* | 100 |

| | | | | | | |
|---|---|---|---|---|---|---|
| * represent week 0 pooled samples from all groups | | | | | | |

**Table 18**

| The effect of CT-CRM_{E29H} on the immune response to meningococcal antigens in Swiss-Webster mice | | | | |
|---|---|---|---|---|
| | Anti-rPilin and anti-PorA antibody ELISA titers on pooled samples | | | |
| Group | rPilin | | Class I OMP H355 | |
| | Sera IgG | Sera IgA | Sera IgG | Sera IgA |
| | Wk 4 | Wk 4 | Wk 4 | Wk 4 |
| 5 µg rPilin | 8,840 | <50 | <50 | <50 |
| 5 µg rPilin + 0.1 µg CT-CRM_{E29H} | 149,221 | 860 | 120 | <50 |
| 5 µg PorA H355 + 0.1 µg CT-CRM_{E29H} | <50 | <50 | 13,795 | 60 |
| 5 µg KLH + 0.1 µg CT-CRM_{E29H} | <50 | <50 | <50 | <50 |

**Table 19**

| Immune responses of Swiss-Webster mice immunized intranasally with meningococcal PorA and CT-CRM_{E29H} | | | | | | | | | |
|---|---|---|---|---|---|---|---|---|---|
| Anti-PorA H44/76 Antibody Titers | | | | | | | | | |
| Group | | Sera | | BW | | NW | | VW | |
| | | IgG | IgA | IgG | IgA | IgG | IgA | IgG | IgA |
| 20 µg PorA | WCE | 3,530 | ND | ND | ND | ND | ND | ND | ND |
| | PorA | 1,220 | <50 | <2 | <2 | <2 | <2 | <7 | <7 |
| 20 µg PorA + 1 µg CT-CRM_{E29H} | WCE | 26,660 | ND | ND | ND | ND | ND | ND | ND |
| | PorA | 17,673 | <50 | 5 | <2 | <2 | <2 | <7 | <7 |

| | | | | | | | | | |
|---|---|---|---|---|---|---|---|---|---|
| ND = No data WCE = whole cell ELISA to H44/76 strain PorA = PorA H44/76 specific ELISA. | | | | | | | | | |

**Table 20**

| Immune responses of Swiss-Webster mice immunized intranasally with heterologous and homologous meningococcal PorA and rPilin with CT-CRM_{E29H} | | | | | | |
|---|---|---|---|---|---|---|
| Assay Antigen | Serum at week | IgG titer^{#} by ELISA in mice immunized with | | | | |
| | | 5µg KLH + 0.1µg CT-CRM_{E29H} | 5µg rPilin + 0.1µg CT-CRM_{E29H} | 5µg PorA H355 + 0.1µg CT-CRM_{E29H} | 5µg PorA 870227 + 0.1µg CT-CRM_{E29H} | 25 µg HI WC 870227 + 0.1µg CT-CRM_{E29H} |
| WC | 0 | <100 | <100 | <100 | <100 | <100 |
| 870227 | 4 | 350 | 13,376 | 7,088 | 24,815 | 74,930 |
| WC | 0 | 257 | 257 | 257 | 257 | 257 |
| H355P | 4 | 310 | 3,687 | 5,140 | 3,930 | 5,933 |
| rPilin | 0 | 146 | 146 | 146 | 146 | 146 |
| | 4 | 585 | 1,999,530 | <100 | <100 | <100 |
| PorA | 0 | <100 | <100 | <100 | <100 | <100 |
| H355 | 4 | <100 | 673 | 29,770 | 19,009 | 463 |
| PorA | 0 | <100 | <100 | <100 | <100 | <100 |
| 870227 | 4 | <100 | <100 | 10,020 | 23,045 | 5,935 |

| | | | | | | |
|---|---|---|---|---|---|---|
| # Week 0 titers are pools from all groups. WC = Whole Cell HI = Heat-inactivated | | | | | | |

**Table 21**

| Immune responses of Swiss-Webster mice immunized intranasally with heterologous and homologous meningococcal PorA and rPilin with CT-CRM_{E29H} | | | | | | |
|---|---|---|---|---|---|---|
| Assay Antigen | Serum at week | IgA titer^{#} by ELISA in mice immunized with | | | | |
| | | 5µg KLH + 0.1µg CT-CRM_{E29H} | 5µg rPilin + 0.1µg CT-CRM_{E29H} | 5µg PorA H355 + 0.1µg CT-CRM_{E29H} | 5µg PorA 870227 + 01.µg CT-CRM_{E29H} | 25 µg HI WC 870227 + 0.1µg CT-CRM_{E29H} |
| WC | 0 | <25 | <25 | <25 | <25 | <25 |
| 870227 | 4 | <25 | <25 | <25 | <25 | ND |
| WC | 0 | <25 | <25 | <25 | <25 | <25 |
| H355P | 4 | <25 | <25 | <25 | <25 | <25 |
| rPilin | 0 | <25 | <25 | <25 | <25 | <25 |
| | 4 | <25 | 5,097 | <25 | <25 | <25 |
| PorA | 0 | <25 | <25 | <25 | <25 | <25 |
| H355 | 4 | <25 | <25 | 233 | 200 | <25 |
| PorA | 0 | <25 | <25 | <25 | <25 | <25 |
| 870227 | 4 | <25 | <25 | <25 | <25 | <25 |

| | | | | | | |
|---|---|---|---|---|---|---|
| # Week 0 titers are pools from all groups. WC = Whole Cell HI = Heat-inactivated ND = No data | | | | | | |

**Table 22**

| *N. meningitidis* Bactericidal Activity from mouse sera subcutaneously immunized with PorA with CT-CRM_{E29H} or MPL^{™} | | |
|---|---|---|
| Mouse sera | H355 p+ | 870227 |
| wk5,d6 | | |
| KLH (5µg), MPL^{™} (100µg) | <25 | <25 |
| wk5,d6 | | |
| H355 class 1 OMP (5µg), MPL^{™} (100µg) | 200 | <25 |
| wk5,d6 | | |
| 870227 class 1 OMP (5µg), MPL^{™} (100µg) | <25 | 100 |
| wk5,d6 | | |
| heat-inactivated 870227 WC (25µg), MPL^{™} (100µg) | 25 | 200 |
| wk5,d6 | | |
| H355 class 1 OMP (5µg), CT-CRM_{E29H} (10µg) | <25 | <25 |
| wk0 pool | | |
| negative control | <10 | <10 |
| wk6 | | |
| positive control 1 | 200 | nd |
| wk6 | | |
| positive control 2 | nd* | 400 |

| | | |
|---|---|---|
| * nd = not done Complement used: Human UR4-97 | | |

### Example 10

### The Immune Responses of BALB/c Mice Immunized with the Purified Native Fusion (F) Glycoprotein of Respiratory Syncytial Virus (RSV)

In a first experiment, 6-8 week old BALB/c mice (5 mice/group) were immunized intranasally (10 µl) at weeks 0 and 14 with 3 µg of purified native protein formulated with CT-CRM_{E29H} (1 µg or 10 µg/mouse), wild-type CT (1 µg/mouse), CT-B (1 µg or 10 µg/mouse) or no adjuvant. Endpoint IgG and IgA antibody titers were assayed, by ELISA, on day 24 of the experiment. Titers were obtained from sera, bronchoalveolar lavage and vaginal washes. The results are presented in Table 23.

In a second experiment, 6-8 week old BALB/c mice (5 mice/group) were pre-immunized intranasally (50 µl) with either wild-type CT (1 µg/mouse) or CT-CRM_{E29H} (1 µg/mouse) on days 0 and 14. Control groups were not pre-immunized. Thereafter, on days 28 and 42, all mice were immunized intranasally with 3 µg of F protein formulated with the same amounts of CT or CT-CRM_{E29H}. The endpoint antibody titers were determined by ELISA on pooled samples collected on days 56 (sera) and 57 (bronchoalveolar and vaginal wash fluids). The results are presented in Table 24.

In a third experiment, naive female BALB/c mice (6-8 weeks, 5 mice/group) were immunized intranasally (IN) at weeks 0, 1 and 2 with purified native fusion (F) protein from RSV A2. Immunizations were prepared by formulating F protein (3 µg/mouse) with CT-CRM_{E29H} (1 µg or 10 µg /mouse), CT-B (1 µg or 10 µg /mouse) or alum (100 µg /mouse). The vaccine was administered intranasally by allowing anaesthetized mice to breathe in the vaccine placed at the tip of the nostril. Total volume per dose was 10 µl per mouse (in Table 25), at weeks 0, 1 and 2. Control mice received intramuscular primary immunization containing F/AlOH, or received primary and secondary immunizations of live RSV A2, delivered intranasally. Systemic humoral immune responses were assayed, by ELISA, nine days (for Tables 25 and 26) post-tertiary immunization. Bronchoalveolar lavage, vaginal and nasal washes were also collected and utilized in the characterization of mucosal antibody responses. Spleens from immunized mice were used to assay antigen-dependent killer cell activity against MHC-compatable RSV-infected target cells. A second cohort of mice, that had received an identical immunization schedule, was challenged with live RSV. Protection of lung compartments within this cohort was subsequently analyzed at four days post-challenge by determination of virus plaques in collected homogenized lung tissue. Statistical analyses were performed using ANOVA. The results are presented in Tables 25 and 26.

In a fourth experiment, BALB/c mice were immunized intranasally at weeks 0, 1 and 2 with purified RSV F protein (3 µg/mouse) in combination with CT-CRM_{E29H} (1 µg or 10 µg/mouse), CTB (1 µg or 10 µg/mouse) or PBS. As a control, mice also received intranasal delivery of RSV or intramusular delivery of F/AlOH. Splenocytes were isolated nine days post-final immunization and stimulated in vitro with syngeneic RSV-infected stimulator cells. After six days in culture, antigen-dependent killer cell activity was determined by quantitation of ⁵¹Chromium release by RSV-infected target cells. The results are presented in Figure 6.

In a fifth experiment, a viral protection assay, the lung compartments of immunized mice were isolated four days after challenge with live RSV, homogenized, and quantitation of infectious virus was performed. BALB/c mice were immunized intranasally with vaccines containing purified F protein from RSV and either CT-CRM_{E29H}, CTB or PBS. Groups of mice were also immunized intramuscularly with F/AlOH as a control. Eight days after final immunization (three weeks post F/AlOH vaccine), mice were challenged with live RSV. Four days later, pulmonary tissues were harvested and utilized in the quantitation of infectious virus. The results are presented in Figure 7.

In a sixth experiment, naive female BALB/c mice (6-8 weeks, 5 mice/group) were immunized intranasally (IN) at weeks 0, 1 and 2 with purified native fusion (F) protein from RSV A2. Immunizations were prepared by formulating F protein (3 µg/mouse) with CT-CRM_{E29H} (1 µg/mouse) or alum (100 µg/mouse). The vaccine was administered intranasally by allowing anaesthetized mice to breathe in the vaccine placed at the tip of the nostril. Total volume per dose was 5 µl per mouse (in Table 27), at weeks 0, 1 and 2. Control mice received intramuscular primary immunization containing F/AlOH, or received primary and secondary immunizations of live RSV A2, delivered intranasally. Systemic humoral immune responses were assayed, by ELISA, two weeks (for Tables 27 and 28) post-tertiary immunization. Bronchoalveolar lavage, vaginal and nasal washes were also collected and utilized in the characterization of mucosal antibody responses. Spleens from immunized mice were used to assay antigen-dependent killer cell activity against MHC-compatable RSV-infected target cells. A second cohort of mice, that had received an identical immunization schedule, was challenged with live RSV. Protection of lung compartments within this cohort was subsequently analyzed at four days post-challenge by determination of virus plaques in collected homogenized lung tissue. Statistical analyses were performed using ANOVA. Results are presented in Tables 27 and 28.

In a seventh experiment, the protocol from the fourth experiment was again utilized to determine the antigen-dependent CTL activity to RSV-infected target cells. The results are presented in Figure 8.

In an eighth experiment, another viral protection assay, BALB/c mice (5 mice/group) were immunized intranasally with formulations containing purified RSV F protein, with or without CT-CRM_{E29H}. Groups of mice were also immunized intramuscularly with F/AlOH and left unimmunized (naive) as a control. Two weeks after final immunization (four weeks post-F/AlOH administration), all groups were challenged with live RSV. Four days later, pulmonary tissues were harvested and utilized in the quantitation of infectious virus. The results are presented in Figure 9.

In a ninth experiment, a three dose protocol was employed to investigate the adjuvant response of CT-CRM_{E29H} in more detail. Groups of five BALB/c mice were immunized IN (5 µl) at weeks 0, 1 and 2 with F protein (3 µg), admixed with either 0.01, 0.1 or 1.0 µg CT-CRM_{E29H}. Control mice were primed at day 0 with F/AlOH (intramuscular) or RSV A2 (IN). Serum antibody titers were determined two weeks post-tertiary immunization. The results are presented in Table 29. Data are presented as the log₁₀ geometric mean antibody titer (± 1 SD). Similar results were obtained in two separate studies.

After IN immunization with F/CT-CRM_{E29H}, the mice in the ninth experiment were also tested for their local antibody responses to F protein. Mucosal wash samples were taken from mice sacrificed two weeks post-tertiary immunization and analyzed for anti-F protein-specific IgG and IgA by ELISA. The results are presented in Table 30. Data are presented as the log₁₀ of the geometric mean endpoint titer that resulted in an OD₄₁₀ of 0.03. Similar results were obtained in two separate studies.

In a tenth experiment, in order to investigate the functional capacity of the humoral immune responses induced by F/CT-CRM_{E29H} immunization, sera were tested in a plaque reduction assay for neutralizing antibody titers to RSV A2. The results are presented in Table 31. Geometric mean neutralizing antibody titers (log₁₀) were determined on individual sera (five mice per group) in the presence (+C') and absence (-C') of 5% guinea pig serum as a complement source. Similar results were obtained in two separate studies.

In an eleventh experiment, mice (five mice per group) received IN immunizations of F protein formulated with 0.01, 0.1 or 1 µg of CT-CRM_{E29H} on days 0, 7 and 14. Control mice were immunized with F/AlOH (intramuscular) or RSV A2 (IN). Immunized mice were challenged two weeks after tertiary immunization, in order to determine the ability of F/CT-CRM_{E29H} to protect against subsequent infection. Four days post-infection, virus levels were determined in homogenized lung and nose tissues of individual mice. The results are presented in Table 32. Data are presented as the geometric mean virus titer per g of tissue (± 1 standard deviation).

**Table 23**

| The generation of anti-cholera toxin antibodies in BALB/c mice after intranasal immunization with either CT, CT-B, or CT-CRM_{E29H} | | | | | | |
|---|---|---|---|---|---|---|
| Anti-Cholera Toxin Antibody Titers^{a} | | | | | | |
| | Sera | | BAW | | VW | |
| Adjuvant^{b} | IgG | IgA | IgG | IgA | IgG | IgA |
| None | <100 | <100 | <25 | <25 | <25 | <25 |
| CT (1 µg) | 434,180 | 3,944 | 70 | <25 | 214 | 1,431 |
| CT-B (1 µg) | 781,628 | 4,665 | 280 | <25 | 127 | 709 |
| CT-B (10 µg) | 156,239 | 2,544 | 241 | <25 | 163 | 1,243 |
| E29H (1 µg) | 118,541 | 2,207 | 94 | <25 | 227 | 1,578 |
| E29H (10 µg) | 514,233 | 4,881 | 410 | ND^{c} | 214 | 980 |

| | | | | | | |
|---|---|---|---|---|---|---|
| ^{a} The endpoint antibody titers were determined on pooled samples collected on day 24 of the study. BAW and VW denote bronchoalveolar and vaginal wash respectively. There were 5 mice per group. ^{b} The mice were immunized intranasally (10 µl) on days 0 and 14 with 3 µg of native F protein admixed with the indicated amount of either wild-type CT, commercial CT-B, or CT-CRM_{E29H}. ^{c} ND = No data | | | | | | |

**Table 24**

| The effect of pre-existing anti-cholera toxin antibodies on the immunogenicity of the fusion protein of respiratory syncytial virus formulated with either CT or CT-CRM_{E29H} | | | | | | | |
|---|---|---|---|---|---|---|---|
| Anti-Fusion Protein Antibody Titers^{a} | | | | | | | |
| | | Sera | | BAW | | VW | |
| Pre-Vax^{b} | Adjuvant | IgG | IgA | IgG | IgA | IgG | IgA |
| None | None | <1,000 | <100 | <25 | <25 | <25 | 25 |
| None | CT | 887,136 | 11,337 | 16,219 | 8,709 | 274 | 1,447 |
| + | CT | >10,000,000 | 22,344 | 253,641 | 24,331 | 36,415 | 4,391 |
| None | E29H | 1,430,836 | 10,786 | 2,232 | 1,680 | 333 | 378 |
| + | E29H | 6,346,730 | 17,890 | 131,217 | 12,367 | 2,750 | 6,359 |

| | | | | | | | |
|---|---|---|---|---|---|---|---|
| ^{a} The endpoint antibody titers were determined by ELISA on pooled samples collected on days 56 (sera) and 57 (bronchoalveolar (BAW) and vaginal (VW) wash fluids). There were 5 mice per group. ^{b} The mice were pre-immunized (intranasally, 50 µl) with either wild-type CT (1 µg) or CT- CRM_{E29H} (1 µg) on days 0 and 14. Thereafter on days 28 and 42 all mice were immunized (intranasally, 50 µl) with 3 µg of F protein formulated with same amounts (1 µg) of either wild-type CT or CT-CRM_{E29H.} The endpoint anti-CT antibody titers of mice pre-immunized with either wild-type CT or CT-CRM_{E29H} and subsequently immunized with F protein were greater than 1,000,000 on day 42. | | | | | | | |

**Table 25**

| Systemic Immune Responses of BALB/c mice immunized intranasally with RSV F protein and CT-CRM_{E29H} | | | | | |
|---|---|---|---|---|---|
| Anti-F Antibody Titers | | | | | |
| Group | Immunogen | IgG | IgG1 | IgG2a | IgA |
| 776 | 10 µg E29H | <100 | <100 | <100 | <100 |
| 777 | 3 µg F | 126,463 +/- 32,646 | 62,344 +/- 27,002 | 4,899 +/- 1,027 | 16,202+/- 2,031 |
| | 1 µg E29H | | | | |
| 778 | 3 µg F | 209,123 +/- 75,688 | 75,711 +/- 29,659 | 19,425 +/- 13,508 | 15,706 +/- 10,909 |
| | 10 µg E29H | | | | |
| 779 | 3 µg F | 46,742 +/- 32,987 | 26,902 +/- 14,985 | 4,239 +/- 3,658 | 4,076 +/- 614 |
| | 1 µg CTB | | | | |
| 780 | 3 µg F | 285,116 +/- 110,154 | 116,245 +/- 34,596 | 10,512 +/- 11,016 | 11,679 +/- 7,246 |
| | 10 µg CTB | | | | |
| 784 | 3 µg F | 2,171 +/- 1,921 | 521 784 +/- 743 | <100 | <100 |
| | PBS | | | | |
| 785 | 3 µg F | 23,303 +/- 16,994 | 5,519 +/- 2,348 | <1000 | <100 |
| | 100 µg AlOH | | | | |
| 907 | RSV A2 | 52,749 +/- 23,557 | 6,252 +/- 4,286 | 8,718 +/- 2,826 | 4,284 +/- 2,350 |

| | | | | | |
|---|---|---|---|---|---|
| For total IgG: p<0.05: 777 to 780 versus 784; 780 vs 779; 777 to 780 vs 785; 777, 778, 780 vs 907 (779 vs 907 p=0.7) p>0.05: 778 vs 777 (p=0.125); For IgG1: p<0.05: 777 to 780 vs 784; 777 to 780 vs 907; 777 to 780 vs 785 p>0.05: 781 to 780 vs 907 For IgG2a p<0.05: 777 to 780 vs 784 | | | | | |

**Table 26**

| Mucosal Immune Responses of BALB/c mice immunized intranasally with RSV F protein and CT-CRM_{E29H} | | | | | | | |
|---|---|---|---|---|---|---|---|
| Anti-F Antibody Titers | | | | | | | |
| Group | Immunogen | BAL pools | | VW pools | | NW pools | |
| | | IgG | IgA | IgG | IgA | IgG | IgA |
| 776 | 10 µg E29H | <25 | <25 | <25 | <25 | <25 | <25 |
| 777 | 3 µg F + 1 µg E29H | 463 | <25 | 253 | 2855 | <25 | 237 |
| 778 | 3 µg F + 10 µg E29H | 370 | <25 | 239 | 848 | 491 | 242 |
| 779 | 3 µg F + 1 µg CTB | 137 | <25 | 298 | 426 | 77 | 272 |
| 780 | 3 µg F + 10 µg CTB | 1109 | 226 | 903 | 3574 | 512 | 372 |
| 784 | 3 µg F PBS | <25 | <25 | <25 | 78 | <25 | <25 |
| 785 | 3 µg F + 100 µg AlOH | <25 | <25 | <25 | <25 | <25 | <25 |
| 907 | RSV A2 | 2870 | 1126 | 167 | 738 | 172 | 170 |

**Table 27**

| Systemic Immune Responses of BALB/c mice immunized intranasally with RSV F protein and CT-CRM_{E29H} | | | | | |
|---|---|---|---|---|---|
| | | Anti-F Antibody Titers | | | |
| Group | Immunogen | IgG | IgG1 | IgG2a | IgA |
| 250 | 3 µg F PBS | <100 | <100 | <100 | <100 |
| 256 | 3 µg F | 315,878 +/- 131,746 | 78,380 +/- 40,870 | 10,718 +/- 16,475 | 444 +/- 1,458 |
| | 1 µg E29H | | | | |
| 257 | 1 µg E29H | <100 | <100 | <100 | <100 |
| 258 | 3 µg F | 121, 551 +/- 52,023 | 63, 595 +/- 27,491 | 428 +/- 4,205 | |
| | 100 µg AlOH | | | | <100 |
| 259 | RSV A2 | 112,451 +/- 50,247 | 8,871 +/- 5,206 | 9,953 +/- 4,924 | 224 +/- 344 |

| | | | | | |
|---|---|---|---|---|---|
| For total IgG: p<0.05: 256 vs 250 and 257 p>0.05: 256 vs 258 and 259 For IgG1: p<0.05: 256 vs 250 and 257 p>0.05: 256 vs 258 For IgG2a p<0.05: 256 vs 250, 257 and 258 p>0.05: 256 vs 259 For IgGA p>0.05: 256 vs 259 | | | | | |

**Table 28**

| Mucosal Immune Responses of BALB/c mice immunized intranasally with RSV F protein and CT-CRM_{E29H} | | | | | | | |
|---|---|---|---|---|---|---|---|
| | | Anti-F Antibody Titers | | | | | |
| Group | Immunogen | BAL pools | | VW pools | | NW pools | |
| | | IgG | IgA | IgG | IgA | IgG | IgA |
| 250 | 3 µg F | <25 | <25 | <25 | <25 | <25 | <25 |
| | PBS | | | | | | |
| 256 | 3 µg F | 826 | <25 | 1,195 | 3,730 | 554 | 875 |
| | 1 µg E29H | | | | | | |
| 257 | 1 µg E29H | <25 | <25 | <25 | <25 | <25 | <25 |
| 258 | 3 µg F | 706 | <25 | 577 | 108 | 148 | <25 |
| | 100 µg | | | | | | |
| | AlOH | | | | | | |
| 259 | RSV A2 | 347 | <25 | 172 | 1,449 | 305 | <25 |

**Table 29**

| Anti-F Protein Serum Antibody Response Induced After Immunization with F Protein and CT-CRM_{E29H} | | | | | | | |
|---|---|---|---|---|---|---|---|
| Geometric mean antibody titers (log₁₀) | | | | | | | |
| Immunogen | Anti-F protein | | | | | Anti-CT | |
| | IgG | IgG1 | IgG2a | IgG2b | IgA | IgG | IgA |
| F/PBS | 3.0 ± 1.0 | 2.9 ± 0.9 | 2.2 ± 0.4 | 2.4 ± 0.6 | <2.0 | <3.7 | <2.0 |
| CT-CRM_{E29H} (1.0 µg) | <2.0 | <2.0 | <2.0 | <2.0 | <2.0 | 6.4 ± 0.3 | 4.4 ± 0.1 |
| F/CT-CRM_{E29H} (1.0 µg) | 5.6 ± 0.3^{a,b} | 5.2 ± 0.3^{a,b} | 4.5 ± 0.3^{a,b} | 5.0 ± 0.3^{a,b} | 3.0 ± 0.6^{a,b} | 6.4 ± 0.2^{c} | 4.3 ± 0.2^{c} |
| F/CT-CRM_{E29H} (0.1 µg) | 5.4 ± 0.2^{a} | 5.1 ± 0.2^{a} | 3.9 ± 0.7^{a} | 4.6 ± 0.5^{a} | 3.1 ± 0.2^{a} | 5.5 ± 0.2^{d} | 3.8 ± 0.2^{d} |
| F/CT-CRM_{E29H} (0.01 µg) | 3.8 ± 0.8^{e} | 3.5 ± 0.9^{e} | 2.3 ± 0.7^{e} | 3.0 ± 0.7^{e} | 2.1 ± 0.2^{e} | <3.7 | <2.0 |
| F/AlOH | 4.9 ± 0.8 | 4.7 ± 0.7 | 3.5 ± 0.9 | 3.7 ± 1.0 | <2.0 | ND⁹ | ND |
| RSV A2 | 4.9 ± 0.1 | 4.0 ± 0.2 | 4.4 ± 0.2 | 4.1 ± 0.3 | 2.3 ± 0.3^{f} | ND | ND |

| | | | | | | | |
|---|---|---|---|---|---|---|---|
| ^{a}p < 0.05 compared to F/PBS or F/CT-E29H (0.01 µg), ^{b}p > 0.05 compared to F/CT-E29H (0.1 µg) or F/AlOH. ^{c}p < 0.05 compared to F/CT-E29H (0.1 and 0.01 µg) and F/PBS. p > 0.05 compared to CT-E29H (1.0 µg). ^{d}p < 0.05 compared to F/PBS and F/CT-E29H (0.01 µg). ^{e}p > 0.05 compared to F/PBS. ^{f}p < 0.05 compared to F/ CT-E29H (0.1 or 1.0 µg). ^{g}ND = not done. | | | | | | | |

**Table 30**

| Anti-F Protein Antibodies in the Mucosal Fluids of Mice Immunized with F Protein Formulated with CT-CRM_{E29H} | | | | | | |
|---|---|---|---|---|---|---|
| Immunogen | BAL | | VW | | NW | |
| | IgG | IgA | IgG | IgA | IgG | IgA |
| F/PBS | <1.4 | <1.4 | <1.4 | <1.4 | <1.4 | <1.4 |
| CT-CRM_{E29H} (1 µg) | <1.4 | <1.4 | <1.4 | <1.4 | <1.4 | <1.4 |
| F/CT-CRM_{E29H} (1 µg) | 3.1 | <1.4 | 2.8 | 3.5 | 2.0 | 2.2 |
| F/CT-CRM_{E29H} (0.1 µg) | 2.5 | <1.4 | 2.5 | 2.9 | <1.4 | 2.3 |
| F/CT-CRM_{E29H} (0.01 µg) | <1.4 | <1.4 | <1.4 | 2.2 | <1.4 | <1.4 |
| F/AlOH | 2.4 | <1.4 | <1.4 | <1.4 | <1.4 | <1.4 |

**Table 31**

| Generation of Systemic Anti-RSV Neutralizing Antibodies After IN Immunization with F Protein Formulated with CT-CRM_{E29H} | | |
|---|---|---|
| Immunogen | Neutralizing antibody titer(+C') | Neutralizing antibody titer(-C') |
| F/PBS | <1.3 | <1.3 |
| CT-CRM_{E29H} (1 µg) | <1.3 | <1.3 |
| F/CT-CRM_{E29H} (1 µg) | 2.3 ± 0.7^{a} | <1.3 |
| F/CT-CRM_{E29H} (0.1 µg) | 2.2 ± 0.4^{b} | <1.3 |
| F/CT-CRM_{E29H} (0.01 µg) | <1.3 | <1.3 |
| F/AlOH | 2.0 ± 0.3 | <1.3 |
| RSV A2 | 2.3 ± 0.3 | <1.3 |

| | | |
|---|---|---|
| ^{a}p < 0.05 compared to F/PBS or F/CT-CRM_{E29H} (0.01 µg), p > 0.05 compared to F/CT-CRM_{E29H} (0.1 µg), F/AlOH or RSV A2. ^{b}p < 0.05 compared to F/PBS or F/CT-CRM_{E29H} (0.01 µg), p > 0.05 compared to F/CT-CRM_{E29H} (1 µg), F/AlOH or RSV A2. | | |

**Table 32**

| Virus Infectivity of Lung and Nasal Tissue After IN Immunization with F Protein and CT-CRM_{E29H} | | |
|---|---|---|
| Immunogen | Lung virus titer (log₁₀ mean ± SD) | Nasal virus titer (log₁₀ mean ± SD) |
| F/PBS | 4.6 ± 0.5 | 2.7 ± 0.2 |
| CT-CRM_{E29H} (1 µg) | 4.6 ± 0.5 | 3.5 ± 0.2 |
| F/CT-CRM_{E29H} (1 µg) | <2.0 ± 0.1^{a,b} | <1.9 ± 0.1 ^{c} |
| F/CT-CRM_{E29H} (0.1 µg) | <1.9 ± 0.1 ^{a} | <1.8 ± 0.1 ^{d} |
| F/CT-CRM_{E29H} (0.01 µg) | 3.9 ± 0.7 | 2.9 ± 0.4 |
| F/AlOH | 2.6 ± 0.7 | 2.3 ± 0.4 |
| RSV | <2.0 ± 0.03 | <1.8 ± 0.1 |
| Naive | 4.6 ± 0.1 | 3.4 ± 0.5 |

| | | |
|---|---|---|
| ^{a}p < 0.05 compared to F/PBS, F/CT-CRM_{E29H} (0.01 µg), CT-CRM_{E29H} or naive, p > 0.05 compared to F/AlOH or RSV A2. ^{b}p > 0.05 compared to F/CT-CRM_{E29H} (0.1 µg). ^{c}p < 0.05 compared to F/CT-CRM_{E29H} (0.01 µg), F/PBS, CT-CRM_{E29H} or naive, p > 0.05 compared to F/CT-CRM_{E29H} (0.1 µg), F/AlOH or RSV A2. ^{d}p < 0.05 compared to F/PBS, F/CT-CRM_{E29}, (0.01 µg), CT-CRM_{E29H} or naive, p > 0.05 compared to F/CT-CRM_{E29H} (1 µg), F/AlOH or RSV A2. | | |

### Example 11

### The Immune Responses of Mice Immunized with Rotavirus Recombinant Virus-Like Particles

*Spodoptera frugiperda* (Sf-9) cells (American Type Culture Collection, Manassas, VA) were maintained in SF-900 II serum free medium (Gibco-BRL, Grand Island, NY). Sf9 cells were co-infected with recombinant baculovirus constructs expressing VP2 and VP6 genes from Simian rotavirus strain SA11 (32).

Released 2/6-VLPs were purified from the growth medium of these infected Sf9 cells as follows. The cells were clarified by centrifugation at 830 x g for 30 minutes at room temperature. The supernatants were then further clarified by centrifugation at 8000 x g for 30 minutes. VLPs were purified from the supernatants by centrifugation twice through 35% sucrose in TNC buffer (10 mM Tris, 140 mM NaCl, 10 mM CaCl₂, pH 8.0) at 96500 x g for two hours, then suspended in TNC buffer and stored at 4°C. Purified VLPs were analyzed by SDS-PAGE, followed by silver and Commassie brilliant blue staining to determine purity, Western blot analysis to analyze protein composition, electron microscopy to determine integrity of the particles, and BCA protein assay to measure total protein concentration.

Commassie brilliant blue, silver staining and Western blot analysis of purified 2/6-VLP confirmed the presence of VP2 and VP6 proteins, as well as their purity and immunoreactivity with specific monoclonal antibodies. Purity of VLPs was estimated to be about 95% from the band intensities on the gels. In addition, electron microscopic analysis of these purified 2/6-VLPs confirmed their morphological integrity (data not shown).

Mice were immunized as follows. BALB/c and CD-1 mice used in this study were purchased from Charles River Laboratories (Storeridge, NY), bred in a rotavirus-free environment. Four week old BALB/c mice were immunized twice on week 0 and 2, either orally (n=4) or IN (n=5, n=4), with 100 and 10 µg of 2/6-VLPs respectively; each dose was formulated with 10 µg of CT-CRM_{E29H}. A third group of BALB/c mice (n=4) received 2/6-VLPs with CT-CRM_{E29H} IN, followed by an oral booster immunization (i.e., mixed group). Control mice in this experiment were immunized with CT-CRM_{E29H} (n=10), 1X TNC buffer (n=5) or 2/6-VLPs plus 1X TNC buffer (n=5). Each mouse was immunized IN with 20 µl of inoculum, 2 µl at a time, into alternating nares at one minute intervals. Serum and fecal samples were collected from all animals on weeks 0, 1, 2, 4, 6, 8, 10, 13 and the levels of rotavirus-specific serum IgG, IgM and IgA antibodies produced, as well as fecal IgG and IgA, were determined by ELISA. The serum antibody results are presented in Figures 10 and 11; the fecal antibody results are presented in Figure 13. Week 13 serum samples were used to determine IgG1 and IgG2a subclasses. The antibody subclass results are presented in Figure 12.

Pre-immunization sera diluted 1:100 and 1:2 dilutions of pre-immunization stool samples showed no reactivity in ELISA. Sera and stools from controls receiving only TNC buffer or CT-CRM_{E29H} were analyzed in parallel. The control groups showed no rotavirus-specific serum or fecal antibodies throughout the study.

Four week old CD-1 mice were immunized three times orally (n=4) or IN (n=4) on weeks 0, 2 and 13 as above using CT-CRM_{E29H} as the adjuvant. A control group (n=2) received CT-CRM_{E29H} alone. Serum and stool samples were collected on weeks 0-9, 11-14, 26-28 and the levels of rotavirus-specific serum and fecal antibodies were determined.

For detection and quantification of IgG, IgM and IgA in stool and serum samples, 96-well polyvinyl chloride microtiter plates (Dynex Technologies, Chantilly, VA) were coated with a hyperimmune guinea pig anti-SA11 rotavirus serum diluted in phosphate buffered saline (PBS) and incubated at 37°C for four hours, or overnight at room temperature. The plates were then blocked with 5% BLOTTO (5% w/v nonfat powdered milk in PBS) at 37°C for two hours. Suspended stool samples were diluted 1:1 in 1% BLOTTO and added to the plates. The plates were then incubated overnight at 4°C, after which they were washed three times with TNC buffer plus 0.05% Tween^{™} 20 (TNC-T). Rabbit anti-rhesus rotavirus hyperimmune serum was diluted in 1% BLOTTO plus 2.5% normal guinea pig serum (NGPS) and added to the plates for one hour at 37°C. The plates were then washed three times with TNC-T. Horseradish peroxidase-conjugated goat anti-rabbit IgG, IgM and IgA (Kirkegaard and Perry Laboratories, Gaithersburg, MD) was diluted in 1% BLOTTO plus 2.5% NGPS and added to the plates, which were incubated for one hour at 37°C. The plates were then washed four times with TNC-T. TMB substrate (Kirkegaard and Perry Laboratories) was added, and the color reactions produced were allowed to develop for seven minutes at room temperature. The reaction was stopped by the addition of 1M phosphoric acid. The OD was determined at 450 nm using a micro-plate reader (BIO-TEK Instrument, Winooski, VT). Measurements of 0.1 above the blank were considered significant. SA11 stock virus was diluted in 1% BLOTTO and added to the plates, which were then incubated overnight at 4°C. The plates were washed three times with TNC-T; thereafter, stool samples diluted 1:1 in 1% BLOTTO or serum samples diluted serially in 1% BLOTTO were applied. As a negative control, duplicates of stool samples were added to a well with no anti-SAll antibody coating. The plates were incubated for two hours at 37°C and then washed three times with TNC-T. Peroxidase-conjugated goat anti-mouse IgG, IgM and IgA were diluted in 1% BLOTTO plus 2.5% NGPS and added to wells; peroxidase-conjugated goat anti-mouse IgA+IgG+IgM(H+L) antibodies were similarly diluted and added to wells for pre-immunization antibody detection. The plates were incubated for one hour at 37°C and then washed four times with TNC-T. The plates were developed as described above for the antigen detection ELISA. The ELISA protocol used to determine IgG subclasses was a modification of the protocol described that employed HRP-labeled rat (monoclonal) anti-mouse IgG1 and IgG2a as the secondary antibodies (Biosource International, Camarillo, CA).

An immunohistochemical assay, described by Ishida et al (49), was modified and used to detect anti-rotaviral VP2 and VP6 antibodies in the serum of mice immunized with 2/6-VLPs. Briefly, early log phase Sf9 cells in shaker flasks were seeded into 96 well tissue culture plates at a density of 2.5 x 10⁴ cell/well and then incubated one hour at room temperature (RT). Subsequently, cells were infected with recombinant baculoviruses encoding VP2 or VP6 genes at a multiplicity of infection of 10, and the infection was allowed to proceed at 28°C for three days. The culture medium was then discarded, plates were dried in a vacuum oven at RT for one hour and fixed with 10% formalin (37% formaldehyde solution containing 10-15% methanol; Sigma) in PBS at RT for 30 minutes. Cells were subsequently permeablized with 1% Triton X-100 (Sigma) in TNC buffer at RT for five minutes.

Each set of infected cells expressing the designated rotavirus protein was exposed to pre-challenge or post-immunization serum from each BALB/c or CD-1 mouse, followed by immunostaining. Mouse serum samples were serially diluted in PBS with 5% FCS. Samples were added to the wells and the plates were incubated at 37°C for two hours. Plates were then washed four times with PBS. Horseradish peroxidase labeled goat anti-mouse IgG, IgM or IgA antibody (Kirkegaard & Perry Laboratories) was added in PBS with 5% FCS, and incubated at 37°C for one hour. Stained cells were detected with 3-amino-9-ethyl-carbazole substrate (AEC)(Sigma) after washing the wells twice with PBS. Uninfected Sf9 cells, serum from unimmunized mice, and pre-immunization sera from immunized mice were used as negative controls. Monoclonal antibodies against VP6 (7D9, 5E6) and VP2 (BP2) were used as positive controls (50).

Unimmunized animals and those immunized with 2/6-VLPs were challenged by gavage with 10 SD₅₀ of wild-type murine EDIM rotavirus (51) on week 26 (CD-1 mice) or on week 13 (BALB/c mice). The titer of EDIM strain was determined as shedding dose 50 (SD₅₀), the dose required to induce fecal viral shedding in 50% of adult mice. The trypsin-activated challenge virus (100 *µ*l) was administered following oral administration of 100 *µ*l of 4% sodium bicarbonate solution to neutralize gastric acidity. Viruses were diluted in M199 media (Irvine Scientific, Santa Ana, CA) and activated with 10 µl of trypsin stock (1 mg/ml) (Sigma Chemical Co, St. Louis, MO) per ml of viral stock solution. Following challenge, stool samples were collected from all animals for 9 days.

Rotavirus antigen shedding in fecal samples was measured by ELISA and expressed as net optical density (OD) values, i.e., OD of the post-challenge fecal sample minus OD of the pre-challenge sample from the same mice. The area under the shedding curve for each animal was determined and the percent reduction in antigen shedding (PRAS) for each animal was calculated by comparing the area under the curve for each animal to the mean area of the control group. The mean PRAS was then calculated for each immunized group. Only PRAS levels above 50% were considered protective. The results are presented in Figure 14.

Statistical analyses were performed with SPSS for version 8.0 for Windows (SPSS, Inc., Chicago, IL.). Independent t tests were used to compare pre-challenge geometric mean titer values and to compare PRAS between groups. Up to three digits after the decimal point were considered in calculating P values (0.000=0).

### Example 12

### Increasing the Expression of CT-CRM_{E29H} Through Use of an Arabinose Inducible Promoter

The construction of the arabinose inducible system was as follows: PCR primers were synthesized to amplify the toxin encoding bicistron from pIIB29H.
Primer #1, CT29 Forward NheI: 5'-TTTTTTGGGCTAGCATGGAGGAAAAGATG AGC-3' (SEQ ID NO:6);
Primer #2, CT29 Reverse HindIII: 5'-GCAGGTCGAAGCTTGCATGTTTGGGC-3' (SEQ ID NO:7). The CT-CRM_{E29H} *ctxA* and *ctxB* encoding PCR fragment was cloned into pBAD18-Cm using the endonuclease sites NheI and HindIII, resulting in construct pCT18. The *ctxB* (*V. cholerae* 2125) gene from pCT18 was removed using ClaI and *Hind*III and replaced with a similar fragment from plasmid pMGJ142 which was shown to encode a *ctxB* gene from *V. cholerae* strain 569B. The resulting construct, pPX7490 encodes the CT-CRM_{E29H} *ctxA* and *ctxB* genes from strain 569B under control of the arabinose promoter, and has the LTIIb-B leader sequence.

Protocols for the large scale expression and purification of CT-CRM_{E29H} were developed and are as follows: 3L fluted Fernbach flasks were used, with 1L per flask of Hy-Soy growth media (per liter: Hy-Soy 10 g; yeast extract 12.5 g; sodium chloride 5 g; sodium phosphate, monobasic 3.3 g; sodium phosphate, dibasic 13.1 g). For starter-stock preparation, 20 µg/ml chloramphenicol and 20 ml of sterile 50% glucose were added to one flask (1% final glucose concentration). The flask was then inoculated with 300 µl DH5α transformed with pPX7490, -70°C frozen stock. The flask was incubated at 37°C with shaking at 200 rpm overnight. All growth media to be used the following day was prewarmed by shaking overnight at 37°C at 200 rpm. The following day, a 1:40 dilution of the overnight culture was made into Hy-Soy growth media, supplemented with 20µg/ml chloramphenicol and 10 ml sterile glycerol (1% final glycerol concentration) as a carbon source. This culture was outgrown at 37°C to an OD₆₀₀ of 4.5-5.5 in Fernbach flasks (or higher in a bioreactor). The culture was then induced with 20 ml L-Arabinose (0.5% final concentration) and allowed to incubate for an additional three hours. After a three hour induction period, the majority of toxin was cell-associated. Cells were harvested by centrifugation and the pellet resuspended into 10 mM NaPO₄, 1 mM EDTA (pH 7.0) buffer at 9% of original culture volume. The cell suspension was mechanically disrupted through a microfluidizer and centrifuged for ten minutes at 8500xg to remove cellular debris. The cellular lysate (supernatant) was further clarified at 42,000 rpm for one hour in a 45Ti rotor, 160,000xg _{AV}. The clarified cell lysate was loaded onto a carboxymethyl (CM) - Sepharose^{™} column (Pharmacia) equilibrated with 10mM NaPO₄ (pH 7.0). Approximately 300 ml CM-Sepharose^{™} was used per 10 liters of culture volume. The cell lysate was loaded onto the column at the rate of 0.102 cm/min (2 ml/min). The column was washed extensively with 10-15 column volumes of 10 mM NaPO₄ (pH 7.0) at 0.255 cm/min (5 ml/min) to remove contaminants. The CT-CRM_{E29H} holotoxin was eluted with four column volumes of 10mM NaPO₄ (pH 8.3) at 0.255 cm/min. The CT-CRM_{E29H}-containing eluate was buffer exchanged by filtration or dialyzed against PBS, then stored at 4°C.

### Example 13

### The Immune Responses of BALB/c Mice Immunized with Plasmid DNA Encoding the Full Length Glycoprotein D of Herpes Simplex Virus Type 2

Six to eight week old female BALB/c mice were immunized with plasmid DNA (pDNA) encoding the full length glycoprotein D of herpes simplex virus type 2 (gD2) (the plasmid containing the gD2 gene is described in Pachuk et al. (40), which is hereby incorporated by reference), formulated with 0.25% bupivacaine, and adjuvanted with wild-type CT, CT-CRM_{E29H} or no adjuvant. Animals were given a secondary immunization three weeks following the primary and euthanised two weeks after the last injection. The protocol for immunization was as follows.

**Table 33**

| Group | Route of Delivery | Conc. of Plasmid | Conc. of Adjuvant | Volume Injected |
|---|---|---|---|---|
| | | | | |
| Mouse 1 | ID | 50 µg 024 | 50 µg CT-CRM_{E29H} | 10 µl |
| Mouse 2 | ID | 50 µg 024 | 50 µg CT | 10 µl |
| Mouse 3 | ID | 50 µg 024 | - | 10 µl |
| Mouse 4 | IM | 50 µg 024 | - | 100 µl |
| Mouse 5 | ID | 50 µg 023 | - | 10 µl |

| | | | | |
|---|---|---|---|---|
| 024 - pDNA including gD2 gene; 023 = pDNA backbone without gD2 gene inserted ID = Intradermal; IM = Intramuscular Group 4 served as a positive control; Group 5 served as a negative control. | | | | |

A proliferation assay was conducted as follows: 1X10⁵ spleen cells were cultured in the presence or absence of 200ng/ml of gD2 protein in complete RPMI media with 10% FCS. After four days incubation at 37°C in the presence of 5% CO₂, cultures were pulsed overnight with ³[H]. ³[H] incorporation was measured on a beta counter. The counts were reported as SI (Stimulation Index = counts in presence of antigen stimulation divided by counts in absence of antigen stimulation). The results are presented in Table 34.

An ELISA was carried out to measure the antigen-specific humoral response in sera and vaginal washes. Briefly, 96 well flat bottom plates (Maxisorb, Nunc) were coated overnight at 4°C with purified gD2 protein at a concentration of 0.4 µgs/ml. The plates were washed three times with PBS and blocked with 4% BSA for one hour at room temperature. Fifty microliters (1:100 dilution) of serum samples or 50µl of vaginal wash sample was added to the plate. After incubation for one hour for sera and overnight at 4°C for vaginal wash, the plates were washed with PBS five times, and a 1:3000 dilution of peroxidase-conjugated anti-mouse Ig (Sigma, St. Louis, MO) was added and the plates incubated for one hour. The plates were washed with PBS before adding the substrate 3,3',5,5'-tetramethylbenzidine (TMB)-H₂O₂ (Biotecx, Houston, TX). Color was allowed to develop for 30 minutes before reading at 450 nm on a Eₘₐₓ microplate reader (Molecular Devices, Sunnyvale, CA). The results are presented in Table 35 (sera) and Table 36 (vaginal washes).

Cytokines were measured using a standard ELISA as described above. Plates were suitably coated to capture either IL-5 or gamma interferon from supernatants from 24 hour or 72 hour old gD2-stimulated cultures respectively. The results are presented in Table 37.

**Table 34**

| gD2-Specific Cellular Proliferation Response (SI) After Administration of pDNA for HSV gD2 with CT or CT-CRM_{E29H} | | | | |
|---|---|---|---|---|
| Group | ID CT | ID CT-CRM_{E29H} | ID | IM |
| | | | | |
| Mouse 1 | 6521 | 28826 | 24696 | 30949 |
| Mouse 2 | 9641 | 15760 | 20249 | 26159 |
| Mouse 3 | 32078 | 25558 | 12472 | 35366 |
| Mouse 4 | 28792 | 19023 | 7092 | 5151 |
| Mouse 5 | 12486 | 22510 | 14702 | 30790 |
| Average | 17904 | 22335 | 15842 | 25683 |

**Table 35**

| gD2-Specific Humoral Response (ng/ml) After Administration of pDNA HSV gD2 with CT or CT-CRM_{E29H} in Sera | | | | |
|---|---|---|---|---|
| Group | ID CT | ID CT-CRM_{E29H} | ID | IM |
| | | | | |
| Mouse 1 | -27 | 606 | 516 | 932 |
| Mouse 2 | -15 | 1387 | 1547 | 1315 |
| Mouse 3 | 333 | 33 | 113 | 430 |
| Mouse 4 | 582 | 755 | 688 | 108 |
| Mouse 5 | 3 | 208 | 13 | 234 |
| Average | 175 | 598 | 575 | 604 |

**Table 36**

| gD2-Specific Humoral Response (ng/ml) After Administration of pDNA HSV gD2 with CT or CT-CRM_{E29H} in Vaginal Washes | | | | |
|---|---|---|---|---|
| Group | ID CT | ID CT-CRM_{E29H} | ID | IM |
| | | | | |
| Mouse 1 | 0 | 0 | 69 | 374 |
| Mouse 2 | 0 | 224 | 0 | 198 |
| Mouse 3 | 0 | 145 | 0 | 83 |
| Mouse 4 | 0 | 0 | 347 | 0 |
| Mouse 5 | 0 | -49 | 0 | 0 |
| Average | 0 | 70 | 54 | 112 |

**Table 37**

| gD2-Specific Cytokine ELISA Profile (pg/ml) | | | | | |
|---|---|---|---|---|---|
| | 024+ CT ID | 024+ CT-CRM_{E29H} ID | 024 ID | 024 IM | 023 Control |
| gamma IFN | 1597 | 1751 | 136 | 716 | 505 |
| IL-5 | 63 | 209 | 9 | 388 | 16 |

### Bibliography

1. Mekalanos, J.J., et al., Nature, 306, 551-557 (1983).
2. Gill, D.M., Biochemistry, 15, 1242-1248 (1976).
3. Cuatrecasas, P., Biochemistry, 12, 3558-3566 (1973).
4. Kassis, S., et al., J. Biol. Chem., 257, 12148-12152 (1982).
5. Mekalanos, J.J., et al., J. Biol. Chem., 254, 5855-5861 (1979).
6. Gill, D.M., and Meren, R., Proc. Natl. Acad. Sci., USA, 75, 3050-3054 (1978).
7. Welsh, C.F., et al., "ADP-Ribosylation Factors: A Family of Guanine Nucleotide-Binding Proteins that Activate Cholera Toxin and Regulate Vesicular Transport", pages 257-280 in Handbook of Natural Toxins: Bacterial Toxins and Virulence Factors in Disease Vol. 8 (Moss, J., et al., Eds., Marcel Dekker, Inc., New York, NY (1995)).
8. Hanson, L.A., Intl. Arch. Allergy Appl. Immunol., 18, 241-267 (1961).
9. Tomasi, T.B., and Zigelbaum, S., J. Clin. Invest., 42, 1552-1560 (1963).
10. Tomasi, T.B., et al., J. Exptl. Med., 121, 101-124 (1965).
11. Brandtzaeg, P., and Baklein, K., Scand. J. Gastroenterol., 11 (Suppl. 36), 1-45 (1976).
12. Brandtzaeg, P., "Immune Functions of Human Nasal Mucosa and Tonsils in Health and Disease", page 28 et seq. in Immunology of the Lung and Upper Respiratory Tract (Bienenstock, J., Ed., McGraw-Hill, New York, NY (1984)).
13. Solari, R., and Kraehenbuhl, J-P, Immunol. Today, 6, 17-20 (1985).
14. Crabbe, P.A., et al., J. Exptl. Med., 130, 723-744 (1969).
15. Bazin, H., et al., J. Immunol., 105, 1049-1051 (1970).
16. Craig, S.W., and Cebra, J.J., J. Exptl. Med., 134, 188-200 (1971).
17. Mestecky, J., et al., J. Clin. Invest., 61, 731-737 (1978).
18. Elson, C.O., and Ealding, W., J. Immunol., 132, 2736-2741 (1984).
19. Maneval, D.R., et al., J. Tissue Cult. Methods, 6, 85-90 (1981).
20. Guidry, J.J., et al., Infect. Immun., 65, 4943-4950 (1997).
21. United States Patent number 5,601,831.
22. United States Patent number 5,108,744.
23. International Patent Application number WO 96/05858.
24. Hu, L.T., et al., Infect. Immun., 60, 2657-2666 (1992).
25. International Patent Application number PCT/US99/09486, filed April 29, 1999.
26. European Patent Application number 449,958.
27. Walsh, E.E., et al., Infect. Immun., 43, 756-758 (1984).
28. Crawford, S.E., et al., J. Virology, 68, 5945-5952 (1994).
29. Lee, A.J., et al., Gastroenterology, 112, 1386-1397 (1997).
30. Snider, D.P., et al., J. Immunol. 153, 647-657 (1994).
31. Tamura, S.Y., et al., Vaccine, 12, 1238-1240 (1994).
32. Van der Akker, F., et al., Structure, 4, 665-678 (1996).
33. Rudin A., et al., Infect. Immun., 66, 3390-3396 (1998).
34. O'Neal, C.M., et al., J. Virology, 71, 8707-8717 (1997).
35. O'Neal C.M., et al., J. Virology, 72, 3390-3393 (1998).
36. International Patent Application number WO 93/13202.
37. International Patent Application number WO 98/42375.
38. International Patent Application number WO 98/20734.
39. United States Patent number 5,593,972.
40. Pachuk, C., et al., Curr. Topics Microbiol. Immunol., 59, 1024-1031 (1997).
41. Jobling, M.G., and Holmes, R.K., Infect. Immun., 60, 4915-4924 (1992).
42. Jobling, M.G., and Holmes, R.K., Mol. Microbiol., 5, 1755-1767 (1991).
43. Kunkel, T.A., Proc. Natl. Acad. Sci., USA, 82, 488-492 (1985).
44. Sanger, F., et al., Proc. Natl. Acad. Sci., USA, 74, 5463-5467 (1977).
45. Tartof, K.D., and Hobbs, C.A., Focus, 9, 12 (1987).
46. Karasic, R., et al., Ped. Inf. Dis. J., 8 (Suppl.), S62-65 (1988).
47. Hansen, E.J., et al., Infect. Immun., 56, 182-190 (1988).
48. Laemmli, U.K., Nature (London), 227, 680-685 (1970).
49. Ishida, S.I., et al., J. Clin. Microbiol., 34, 1694-1700 (1996)..
50. Burns, J.W., et al., Science, 272, 104-107 (1996).
51. McNeal, M.M., and Ward, R.L., Virology, 211, 474-480 (1995).

## Claims

1. An antigenic composition comprising a selected antigen from a pathogenic bacterium, virus, fungus or parasite and an effective adjuvanting amount of a mutant cholera holotoxin, wherein the holotoxin has reduced toxicity compared to a wild-type cholera holotoxin and has a substitution at position 29 of the A subunit of the cholera holotoxin, wherein the glutamic acid residue is replaced by histidine; and wherein said holotoxin enhances the immune response in a vertebrate host to said antigen.

2. The antigenic composition of Claim 1 wherein the selected antigen is the recombinant P4 outer membrane protein of nontypable *Haemophilus influenzae*, the recombinant P6 outer membrane protein of nontypable *Haemophilus influenzae,* the *Helicobacter pylori* recombinant urease protein, or the respiratory syncytial virus fusion protein.

3. The antigenic composition of claim 1 or claim 2 which further comprises a diluent or carrier.

4. The antigenic composition of any one of the preceding claims wherein at least one additional mutation is made to the A subunit of the cholera holotoxin at a position other than amino acid 29, wherein said mutation includes replacing the arginine at position 7, aspartic acid at position 9, arginine at position 11, histidine at position 44, valine at position 61, serine at position 63, histidine at position 70, valine at position 97, tyrosine at position 104, proline at position 106, histidine at position 107, serine at position 109, glutamic acid at position 110, glutamic acid at position 112, serine at position 114, tryptophan at position 127, arginine at position 146, or arginine at position 192.

5. The use of an antigenic composition as claimed in any one of the preceding claims in the preparation of a medicament, wherein said composition further contains a selected antigen from a pathogenic bacterium, virus, fungus or parasite, and wherein said antigen elicits an immune response of a vertebrate host.

6. A use as claimed in Claim 5 wherein the selected antigen in the antigenic composition is the recombinant P4 outer membrane protein of nontypable . *Haemophilus influenzae,* the recombinant P6 outer membrane protein of nontypable *Haemophilus influenzae,* purified native *Haemophilus influenzae* adherence and penetration protein (Haps), the *Helicobacter pylori* recombinant urease protein, the *Neisseria meningitidis* Group B recombinant class 1 rpilin, the *Neisseria meningitidis* Group B class 1 PorA protein, the respiratory syncytial virus fusion protein, 2/6 virus-like particles of rotavirus or the herpes simplex virus (HSV) type 2 glycoprotein D (gD2).

7. A plasmid containing an isolated and purified DNA sequence comprising a DNA sequence which encodes an immunogenic mutant cholera holotoxin having a substitution at position 29 of the A subunit of the cholera holotoxin, wherein the glutamic acid residue is replaced by histidine; and wherein the DNA sequence is operatively linked to an arabinose inducible promoter.

8. A host cell transformed, transduced or transfected with the plasmid of Claim 7.

9. A method of producing an immunogenic mutant cholera holotoxin, wherein the cholera holotoxin has reduced toxicity compared to a wild type cholera holotoxin and has a substitution at position 29 of the A subunit of the cholera holotoxin, wherein the glutamic acid residue is replaced by histidine; and which comprises transforming, transducing or transfecting a host cell with the plasmid of Claim 7, and culturing the host cell under conditions which permit the expression of said recombinant immunogenic detoxified protein by the host cell.

10. Use of an effective adjuvanting amount of a mutant cholera holotoxin, wherein the holotoxin has a substitution at position 29 of the A subunit of the cholera holotoxin, wherein the glutamic acid residue is replaced by histidine, in combination with a selected antigen from a pathogenic bacterium, virus, fungus or parasite, to prepare an antigenic composition, wherein said holotoxin enhances the immune response in a vertebrate host to said antigen.

## Patentansprüche

1. Antigene Zusammensetzung mit einem aus einem pathogenen Bakterium, Virus, Pilz oder Parasiten ausgesuchten Antigen sowie einer wirksamen adjuvanten Menge eines mutanten Cholera-Holotoxins, wobei das Holotoxin im Vergleich mit einem Wildtyp-Cholera-Holotoxin eine verminderte Toxizität aufweist und an der Position 29 der A-Untereinheit des Cholera-Holotoxins substituiert ist, wobei der Glutaminsäurerest durch Histidin ersetzt ist; und wobei das Holotoxin in einem Vertebraten-Wirt die Immunantwort gegen dieses Antigen verstärkt.

2. Antigene Zusammensetzung nach Anspruch 1, in welcher das ausgesuchte Antigen das rekombinante P4-Protein auf der Außenmembran von nicht-typisierbarem *Hämophilus influenzae,* das rekombinante P6-Protein auf der Außenmembran von nicht-typisierbarem *Hämophilus influenzae,* das rekombinante Urease-Protein von *Heliobacter pylori* oder das Fusionsprotein des RS-Virus ist.

3. Antigene Zusammensetzung nach Anspruch 1 oder 2, welche ferner ein Verdünnungsmittel oder einen Träger umfasst.

4. Antigene Zusammensetzung nach jedem der vorhergehenden Ansprüche, in welcher an der A-Untereinheit des Cholera-Holotoxins an einer anderen Position als der Aminosäure 29 mindestens eine zusätzliche Mutation erfolgt ist, wobei diese Mutation das Ersetzen des Arginins an der Position 7, der Asparaginsäure an der Position 9, des Arginins an der Position 11, des Histidins an der Position 44, des Valins an der Position 61, des Serins an der Position 63, des Histidins an der Position 70, des Valins an der Position 97, des Tyrosins an der Position 104, des Prolins an der Position 106, des Histidins an der Position 107, des Serins an der Position 109, der Glutaminsäure an der Position 110, der Glutaminsäure an der Position 112, des Serins an der Position 114, des Tryptophans an der Position 127, des Arginins an der Position 146 oder des Arginins an der Position 192 umfasst.

5. Verwendung einer antigenen Zusammensetzung nach jedem der vorhergehenden Ansprüche bei der Herstellung eines Medikaments, wobei die Zusammensetzung ferner ein aus einem pathogenen Bakterium, Virus, Pilz oder Parasiten ausgesuchtes Antigen enthält und wobei das Antigen eine Immunantwort eines Vertebraten-Wirts auslöst.

6. Verwendung nach Anspruch 5, wobei in der antigenen Zusammensetzung das ausgesuchte Antigen das rekombinante P4-Protein auf der Außenmembran von nicht-typisierbarem *Hämophilus influenzae,* das rekombinante P6-Protein auf der Außenmembran von nicht-typisierbarem *Hämophilus influenzae,* das gereinigte native Adhäsions- und Penetrationsprotein von *Hämophilus influenzae* (Hapₛ), das rekombinante Urease-Protein von *Helicobacter pylori,* das rekombinante Klasse 1-rPilin von *Neisseria meningitidis* Gruppe B, das Klasse 1-PorA-Protein von *Neisseria meningitidis* Gruppe B, das Fusionsprotein des RS-Virus, die 2/6 virusähnlichen Teilchen vom Rotavirus oder das Glykoprotein D des Herpes simplex-Virus (HSV) Typ 2 (gD2) ist.

7. Plasmid, das eine isolierte und gereinigte DNA.Sequenz enthält, welche eine DNA-Sequenz umfasst, die für ein immunogenes mutantes Cholera-Holotoxin mit einer Substitution an der Position 29 der A-Untereinheit des Cholera-Holotoxins codiert, wobei der Glutaminsäurerest durch Histidin ersetzt ist; und wobei die DNA-Sequenz funktionsfähig an einen durch Arabinose induzierbaren Promotor gebunden ist.

8. Eine mit dem Plasmid des Anspruchs 7 transformierte, transduzierte oder transfizierte Wirtszelle.

9. Verfahren zur Herstellung eines immunogenen mutanten Cholera-Holotoxins, wobei das Holotoxin im Vergleich mit einem Wildtyp-Cholera-Holotoxin eine verminderte Toxizität aufweist und an der Position 29 der A-Untereinheit des Cholera-Holotoxins substituiert ist, wobei der Glutaminsäurerest durch Histidin ersetzt ist; und wobei das Verfahren die Transformation, Transduktion und Transfektion einer Wirtszelle mit dem Plasmid des Anspruchs 7 umfasst sowie die Kultivierung der Wirtszelle unter Bedingungen, welche die Expression des rekombinanten, immunogenen, entgifteten Proteins durch die Wirtszelle erlauben.

10. Verwendung einer effektiven adjuvanten Menge eines mutanten Cholera-Holotoxins, wobei das Holotoxin an der Position 29 der A-Untereinheit des Cholera-Holotoxins substituiert ist, wobei der Glutaminsäurerest durch Histidin ersetzt ist; zusammen mit einem aus einem pathogenen Bakterium, Virus, Pilz oder Parasiten ausgesuchten Antigen, um eine antigene Zusammensetzung herzustellen, wobei das Holotoxin in einem Vertebraten-Wirt die Immunantwort gegen das Antigen verstärkt.

## Revendications

1. Composition antigénique comprenant un antigène sélectionné parmi une bactérie pathogène, un virus, un champignon ou un parasite et une quantité adjuvante efficace d'une holotoxine mutante du choléra, où l'holotoxine a une toxicité réduite par rapport à une holotoxine du choléra de type sauvage et a une substitution en position 29 de la sous-unité A de l'holotoxine du choléra, où le résidu d'acide glutamique est remplacé par l'histidine ; et où ladite holotoxine accentue la réponse immunitaire au dit antigène chez un hôte vertébré.

2. Composition antigénique selon la revendication 1, dans laquelle l'antigène sélectionné est la protéine de membrane externe P4 recombinante de *Haemophilus influenzae* non typable, la protéine de membrane externe P6 recombinante de *Haemophilus influenzae* non typable, la protéine d'uréase recombinante de *Helicobacter pylori,* ou la protéine de fusion du virus respiratoire syncytial.

3. Composition antigénique selon la revendication 1 ou la revendication 2, qui comprend en outre un diluant ou un véhicule.

4. Composition antigénique selon l'une quelconque des revendications précédentes, dans laquelle au moins une mutation additionnelle est opérée à la sous-unité A de l'holotoxine du choléra en une position autre que l'acide aminé 29, où ladite mutation comprend le remplacement de l'arginine en position 7, de l'acide aspartique en position 9, de l'arginine en position 11, de l'histidine en position 44, de la valine en position 61, de la sérine en position 63, de l'histidine en position 70, de la valine en position 97, de la tyrosine en position 104, de la proline en position 106, de l'histidine en position 107, de la sérine en position 109, de l'acide glutamique en position 110, de l'acide glutamique en position 112, de la sérine en position 114, du tryptophane en position 127, de l'arginine en position 146, ou de l'arginine en position 192.

5. Utilisation d'une composition antigénique selon l'une quelconque des revendications précédentes, dans la préparation d'un médicament, dans laquelle ladite composition contient en outre un antigène sélectionné parmi une bactérie pathogène, un virus, un champignon ou un parasite, et où ledit antigène provoque une réponse immunitaire d'un hôte vertébré.

6. Utilisation selon la revendication 5, dans laquelle l'antigène sélectionné dans la composition antigénique est la protéine de membrane externe P4 recombinante de *Haemophilus influenzae* non typable, la protéine de membrane externe P6 recombinante de *Haemophilus influenzae* non typable, la protéine de pénétration et d'adhérence à *Haemophilus influenzae* native purifiée (Hapₐ), la protéine d'uréase recombinante de *Helicobacter pylori,* la rpiline de catégorie 1 recombinante de *Neisseria meningitidis* du groupe B, la protéine PorA de catégorie 1 de *Neisseria meningitidis* du groupe B, la protéine de fusion du virus respiratoire syncytial, les particules de rotavirus de type viroïdal 2/6 ou la glycoprotéine D du virus de l'herpès simplex (VHS) de type 2 (gD2).

7. Plasmide contenant une séquence d'ADN isolée et purifiée comprenant une séquence d'ADN qui code une holotoxine mutante du choléra immunogénique ayant une substitution en position 29 de la sous-unité A de l'holotoxine du choléra, dans lequel le résidu d'acide glutamique est remplacé par l'histidine ; et dans lequel la séquence d'ADN est liée de manière fonctionnelle à un promoteur inductible arabinose.

8. Cellule hôte transformée, traduite ou transfectée avec le plasmide selon la revendication 7.

9. Procédé de production d'une holotoxine mutante du choléra immunogénique, dans lequel l'holotoxine du choléra a une toxicité réduite par rapport à une holotoxine du choléra de type sauvage et a une substitution en position 29 de la sous-unité A de l'holotoxine du choléra, dans lequel le résidu d'acide glutamique est remplacé par l'histidine; et qui comprend la transformation, la transduction ou la transfection d'une cellule hôte avec le plasmide selon la revendication 7, et la culture de la cellule hôte dans des conditions permettant l'expression de ladite protéine détoxifiée immunogénique recombinante par la cellule hôte.

10. Utilisation d'une quantité adjuvante efficace d'une holotoxine mutante de choléra, dans laquelle l'holotoxine a une substitution en position 29 de la sous-unité A de l'holotoxine du choléra, où le résidu d'acide glutamique est remplacé par l'histidine, en combinaison avec un antigène sélectionné parmi une bactérie pathogène, un virus, un champignon ou un parasite, pour préparer une composition antigénique, dans laquelle ladite holotoxine accentue la réponse immunitaire au dit antigène chez un hôte vertébré.
